(19) Europäisches Patentamt
European Patent Office
Office européen des brevets

(11) EP 4 565 565 B1

(12) **EUROPEAN PATENT SPECIFICATION**

(45) Date of publication and mention of the grant of the patent:
**27.05.2026 Bulletin 2026/22**

(21) Application number: **23754383.0**

(22) Date of filing: **02.08.2023**

(51) International Patent Classification (IPC):
***C07D 231/06*** *(2006.01)* ***C07D 403/10*** *(2006.01)*
***A61K 31/4152*** *(2006.01)* ***A61K 31/4155*** *(2006.01)*
***A61P 1/00*** *(2006.01)* ***A61P 1/04*** *(2006.01)*
***A61P 1/06*** *(2006.01)* ***A61P 1/10*** *(2006.01)*
***A61P 1/12*** *(2006.01)* ***A61P 11/00*** *(2006.01)*
***A61P 11/06*** *(2006.01)* ***A61P 11/08*** *(2006.01)*
***A61P 11/14*** *(2006.01)*

(52) Cooperative Patent Classification (CPC):
**C07D 231/06; A61P 1/00; A61P 1/04; A61P 1/06; A61P 1/10; A61P 1/12; A61P 11/00; A61P 11/06; A61P 11/08; A61P 11/14; C07D 403/10**

(86) International application number:
**PCT/GB2023/052046**

(87) International publication number:
**WO 2024/028601 (08.02.2024 Gazette 2024/06)**

(54) **1,2-SUBSTITUTED 3-OXOPYRAZOLIDINE DERIVATIVES AS PROSTAGLANDIN E2 RECEPTOR 4 (EP4) AGONISTS FOR THE TREATMENT OF GASTROINTESTINAL AND PULMONARY DISEASES**

1,2-SUBSTITUIERTE 3-OXOPYRAZOLIDIN-DERIVATE ALS PROSTAGLANDIN E2 RECEPTOR 4 (EP4) AGONISTEN ZUR BEHANDLUNG VON MAGEN-DARM- UND LUNGEN-ERKRANKUNGEN

DÉRIVÉS DE 3-OXOPYRAZOLIDINE SUBSTITUTÉS 1,2- EN TANT QU'AGONISTS DU RÉCÉPTEUR 4 DE PROSTAGLANDIN E2 (EP4) POUR LE TRAITEMENT DE MALADIES GASTRO-INTESTINALES ET PULMONAIRES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **02.08.2022 GB 202211234**

(43) Date of publication of application:
**11.06.2025 Bulletin 2025/24**

(73) Proprietor: **Nxera Pharma UK Limited**
**Cambridge, Cambridgeshire CB21 6DG (GB)**

(72) Inventors:
- **SWAIN, Nigel Alan**
  **Cambridge CB21 6DG (GB)**
- **WHITEHURST, Benjamin**
  **Cambridge CB21 6DG (GB)**
- **CONGREVE, Miles Stuart**
  **Cambridge CB21 6DG (GB)**
- **BROWN, Giles Albert**
  **Cambridge CB21 6DG (GB)**

(74) Representative: **Gill Jennings & Every LLP**
**The Broadgate Tower**
**20 Primrose Street**
**London EC2A 2ES (GB)**

(56) References cited:
**WO-A1-03/035064**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 565 565 B1

## Description

This application relates to novel compounds and their use as prostaglandin $E_2$ receptor 4 ($EP_4$) agonists. Compounds described herein may be useful in the treatment or prevention of diseases in which $EP_4$ receptors are involved. The application is also directed to pharmaceutical compositions comprising these compounds and the compounds and compositions for use in the prevention or treatment of such diseases in which $EP_4$ receptors are involved.

BACKGROUND OF THE INVENTION

Prostanoids including prostaglandins and thromboxanes are metabolite derivatives of arachidonic acid that play key roles in cellular physiological function. Arachidonic acid is an integral component of membrane phospholipids which is released via the activity of phospholipase A2 ($PLA_2$). The biosynthesis of prostaglandins is mediated via cyclooxygenase (COX) which catalyses conversion of arachidonic acid to an unstable intermediate ($PGH_2$) and leads to the generation of prostaglandins including $PGE_2$. $PGE_2$ represents the most widely produced prostanoid whose activity is mediated through action at 4 functionally distinct subtypes of receptors, EP1-4.

EP receptors belong to a family of G protein coupled receptors (GPCR) which are integral membrane proteins with seven-transmembrane domains. This receptor class can broadly be classified according to their signaling pathways: i) Gs coupled receptors (adenylate cyclase activation and cyclic adenosine monophosphate (cAMP) production), EP2 and EP4 ii) Gq coupled receptor (PLC activation), EP1 and iii) Gi coupled receptor (inhibition of adenylate cyclase), EP3.

EP4 receptor signals through Gs and couples positively to adenylate cyclase to increase cAMP levels. The receptor was originally described in 1993 with the identification of an EP2 like receptor which couples positively to adenylate cyclase but does not bind butaprost (A Honda et al. *J. Biol. Chem.* **1993,** 268, 7759-7762).

The EP4 receptor plays key roles in diverse physiological functions including gastrointestinal homeostasis, regulation of vascular tone, renal function, inflammation, fever and carcinogenesis. The potent biological action of PGE2 has fuelled interest in developing subtype selective EP4 agonists and antagonists for the treatment of a broad range of indications.

Functional gastrointestinal diseases (FGIDs), including chronic constipation, are common gastrointestinal conditions encountered by primary care physicians and gastroenterologists. The prevalence of chronic constipation ranges from 1% to 8% and can negatively impact the quality of life (QoL), resulting in major social and economic burden. Chronic constipation can bring discomfort to patients and affect their daily lives. Symptoms include hard and lumpy stools, straining during defecation and a sensation of incomplete evacuation. The use of laxatives and stool softners remains high, despite many patients not obtaining substantial benefit. There remains a need for treatments that can provide complete and sustainable symptomatic relief.

The intestinal mucosa plays an important role in the homeostasis of anion and body fluid maintenance. These functions are mediated through coordinated ion transport via membrane bound transporters and channels localized on the apical and basolateral membrane of intestinal epithelial cells. PGE2 is a well established secretagogue that may directly promote chloride secretion from intestinal epithelial cells. The secretory effects of PGE2 are in part mediated via the EP4 receptor which can stimulate anion chloride secretion across the gut mucosa. Lubiprostone, a bicyclic fatty acid derivative of PGE1 clinically approved for the treatment of chronic constipation and IBS-C has been shown to promote fluid secretion and gastrointestinal tract motility through the activation of prostaglandin (EP4) receptors. EP4 selective agonists may have therapeutic value in promoting intestinal fluid homeostasis in chronic constipation conditions.

Inflammatory bowel disease (IBD) is a chronic debilitating gastrointestinal disorder that includes ulcerative colitis and Crohn's disease. Patients with IBD commonly present with symptoms that include diarrhoea, abdominal pain, weight loss, rectal bleeding and fever. Clinical management involves strategies to control the abnormal dysregulated immune response in the intestinal mucosa. A wide range of agents are employed to induce and maintain remission, depending on the severity of the disease. These include aminosalicylates, corticosteroids, immunosuppressive agents, antibiotics and biologic agents. This may be given in a stepwise approach, with intensification of therapy according to the severity and progression of the disease. It is clear, however, that some patients are refractory to medical therapy or fail to tolerate it due to systemic side effects. Only a subset of patients achieve long term remission with current therapeutic strategies, suggesting the need for improved therapies.

Intestinal barrier dysfunction plays a key pathogenic role in IBD and there is emerging interest in the development of agents that restore barrier function (mucosal healin g). EP4 is expressed in a number of cell types including gastrointestinal (GI) epithelial cells, lamina propria mononuclear cells and colon innervating sensory neurones and may offer benefits in dampening the inappropriate mucosal immune response as well as protecting the GI mucosal barrier. PGE2 signalling through EP4 promotes cellular differentiation to a wound-associated epithelial cell phenotype which is important for wound repair (Miyoshi, H. et al. EMBO J. 2017, 36, 5-24). Administration of EP4 agonists provide benefit in chemically induced colitis models (Kabashima, K. et al. J. Clin. Invest. 2002, 109, 883-893; Watanabe, Y. et al. Eur. J. Pharmacol. 2015, 754, 179-189; Nitta, M. et al. Scand. J. Immunol. 2002, 56, 66-75) while mutant mice lacking EP4

develop severe dextran sodium sulfate induced colitis characterised by impaired mucosal barrier function, increased epithelial cell loss, crypt damage and increased immune cell infiltration (Kabashima, K. et al. J. Clin. Invest. 2002, 109, 883-893). In a small PhII study, the EP4 agonist, ONO-4819CD was evaluated in patients with mild to moderate ulcerative colitis refractory to 5-ASA (Nakase, H. et al. Inflamm. Bowel Dis. 2010, 16, 731-733). Although the study was not powered for efficacy, patients treated with ONO-4819CD showed signs of improved disease activity index (DAI) score and histological scores. These data support a potential clinical benefit of EP4 agonist therapy in IBD.

Asthma and chronic obstructive pulmonary disease are inflammatory diseases of the airway characterised by limitations in airflow. It is estimated that approximately 300 million people have asthma and represents the most common chronic disease in children. Despite the advances in the management of asthma, a significant proportion of patients have uncontrolled disease which can lead to mortality and morbidity. Therapies that can achieve effective control of asthma symptoms and reduce risk of future exacerbations for long term management are still needed. PGE2 is known to have bronchodilator and anti-inflammatory effects in rodent and human isolated airway smooth muscle. Inhaled PGE2 has been shown to be beneficial on inflammation and airway calibre in patients with chronic bronchitis and asthma. PGE2 however, also induces a reflex cough, potentially via EP3 receptor mediated irritancy of the upper airway. This has led to efforts to discover EP receptor selective agents for the treatment of airway disorders. Interestingly, key species differences have been reported in the receptor subtype responsible for mediating airway smooth muscle relaxation. In guinea pig, monkey and mouse, EP2 agonists could induce relaxation of airway smooth muscle, whilst in human, this is mediated via the EP4 receptor (Buckley, J. et al. Thorax 2011, 66, 1029-1035). Selective EP4 receptor agonists may have potential therapeutic value in airway diseases.

WO 03/035064 discloses a group of substituted pyrazolidinone compounds having EP2 and EP4 receptor activity.

EP4 receptor has been shown to exert roles in the regulation of blood pressure. EP4 is expressed on smooth muscle and endothelial cells and may induce vasodilatory effects via endothelial nitric oxide synthase (eNOS) mediated nitric oxide (NO) production. PGE2 has been shown to dose dependently relax smooth muscle in aortic rings, an effect that is abolished in EP4 knockout mouse. In halothane anesthetised dogs, the EP4 selective agonist, ONO-AE1-329 induces a vasodepressor response (Honda, A. et al. Eur. J. Pharmacol. 2016, 775, 130-137) and similarly, hypotension was reported as one of the adverse drug reactions in IBD patients receiving ONO-4819CD (Nakase, H. et al. Inflamm. Bowel Dis. 2010, 16, 731-733). EP4 agonists that can be used for the treatment of a range of gastrointestinal and airway disorders without cardiovascular systemic side effects may have potential therapeutic value. In particular, EP4 agonists that can be used for the treatment of a range of gastrointestinal disorders without cardiovascular systemic side effects may have potential therapeutic value. The discovery of safe and effective EP4 selective agents are needed.

## SUMMARY OF THE INVENTION

The present invention provides compounds having activity as prostaglandin $E_2$ receptor 4 ($EP_4$) agonists.

In one aspect, provided herein is a compound of the Formula I:

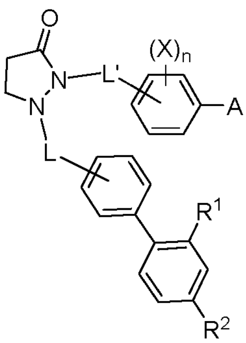

I

or a pharmaceutically acceptable salt, solvate, hydrate, tautomer, or optical isomer thereof, wherein;

- A is OR', C(O)R', $CO_2R'$, $C(O)N(R')_2$, $C(O)N(R')S(O)_2R^3$, $S(O)_2R'$, $S(O)_2OR'$, $SO_2N(R')_2$, $C_{1-8}$ alkyl, cycloalkyl, heterocycloalkyl, aryl, or heteroaryl;
- X is halo, OR', COOR', or $C_{1-6}$ alkyl;
- L and L' are each independently a $C_{2-4}$ alkylene;
- $R^1$ is H, halo, CN, $NO_2$, OR', SR', COOR', $C_{1-6}$ alkoxy, or $C_{1-6}$ alkyl;
- $R^2$ is OR', $OC(O)R^3$, $OC(O)OR^3$, $CO_2R'$, $CON(R')_2$, $SO_2N(R')_2$, $SO_2R^3$, $OSO_2R^3$, or $OSO_2N(R')_2$;
- $R^3$ is $C_{1-6}$ alkyl, $C_{3-6}$ cycloalkyl, or a phenyl;

R' is H, $C_{1-6}$ alkyl, or $C_{3-6}$ cycloalkyl; and
n is 0, 1, 2, or 3;
wherein at each occurrence, alkyl, alkylene, alkoxy and cycloalkyl are each optionally and independently substituted with up to 3 instances of OH, SH, CN, $NO_2$, COOH, halo, or $COOC_{1-4}$ alkyl;
wherein at each occurrence, heterocycloalkyl, aryl, and heteroaryl are each optionally and independently substituted with up to 3 instances of OR', SR', CN, $NO_2$, $CO_2R'$, halo, $C_{1-4}$ alkyl, or oxo.

In another aspect, the invention includes a pharmaceutical composition comprising a compound described herein, and a pharmaceutically acceptable excipient.

In another aspect, the invention includes a kit comprising a compound described herein and at least one additional therapeutic agent selected from the group consisting of aminosalicylates, corticosteroids, immunomodulators and combinations thereof.

In another aspect, the invention includes a compound described herein, a composition described herein, or a kit described herein for use as a medicament.

In another aspect, the invention includes a compound described herein, a composition described herein, or a kit described herein for use in the treatment of an EP4 receptor mediated disease.

In another aspect, the invention includes a compound or composition described herein for use in a method of modulating EP4 receptor agonist activity in a biological sample, the method comprising contacting said EP4 receptor with a compound or composition described herein.

In another aspect, the invention includes a compound or composition described herein for use in a method of treating an EP4 receptor mediated disease, the method comprising administering to a patient in need thereof a compound or composition described herein.

The compounds herein may be used as $EP_4$ receptor agonists. The compounds herein may selectively act at $EP_4$ receptor. The compounds may be used in the manufacture of compositions or medicaments. The compounds, compositions or medicaments may be used in treating, preventing, ameliorating, controlling or reducing the risk of diseases or disorders in which $EP_4$ receptors are involved. The compounds, compositions or medicaments may be used in treating, preventing, ameliorating, controlling or reducing the risk of gastrointestinal disorders and conditions, including but not limited to constipation disorders, constipation-predominant irritable bowel syndrome, mixed type irritable bowel syndrome, chronic idiopathic constipation, gastrointestinal symptoms associated with Parkinson's disease, gastrointestinal symptoms associated with cystic fibrosis, intestinal dysmotility, postoperative ileus, food allergy or food intolerance, celiac disease, gastrointestinal motility disorders, functional gastrointestinal disorders, drug induced enteropathy, NSAID induced gastric and intestinal injury, chemotherapy induced mucositis, gastroesophageal reflux disease (GERD), duodenogastric reflux, diarrhoeal diseases, immune mediated gastrointestinal diseases, Crohn's disease, ulcerative colitis, inflammatory bowel disease and ischemic colitis.

The compounds, compositions or medicaments may also be used in treating, preventing, ameliorating, controlling or reducing the risk of pulmonary diseases and conditions such as chronic obstructive pulmonary diseases, asthma, chronic bronchitis, cystic fibrosis, emphysema, chronic idiopathic cough, hyperactive airway disorder and idiopathic pulmonary fibrosis.

## DETAILED DESCRIPTION OF THE INVENTION

The invention relates to novel compounds. The invention also relates to the use of novel compounds as agonists of the $EP_4$ receptor. The invention further relates to the use of novel compounds in the manufacture of medicaments for use as $EP_4$ receptor agonists, and the compounds of the invention for use in methods of treatment comprising administering a compound of the invention as an $EP_4$ receptor agonist.

The compounds of Formula (I) may be used in treating, preventing, ameliorating, controlling or reducing the risk of diseases or disorders in which $EP_4$ receptors are involved. The compounds of Formula (I) may be used in treating, preventing, ameliorating, controlling or reducing the risk of gastrointestinal disorders and conditions, including but not limited to constipation disorders, constipation-predominant irritable bowel syndrome, mixed type irritable bowel syndrome, chronic idiopathic constipation, gastrointestinal symptoms associated with Parkinson's disease, gastrointestinal symptoms associated with cystic fibrosis, intestinal dysmotility, postoperative ileus, food allergy or food intolerance, celiac disease, gastrointestinal motility disorders, functional gastrointestinal disorders, drug induced enteropathy, NSAID induced gastric and intestinal injury, chemotherapy induced mucositis, gastroesophageal reflux disease (GERD), duodenogastric reflux, diarrhoeal diseases, immune mediated gastrointestinal diseases, Crohn's disease, ulcerative colitis, inflammatory bowel disease and ischemic colitis.

The compounds of Formula (I) may also be used in treating, preventing, ameliorating, controlling or reducing the risk of pulmonary diseases and conditions such as chronic obstructive pulmonary diseases, asthma, chronic bronchitis, cystic fibrosis, emphysema, chronic idiopathic cough, hyperactive airway disorder and idiopathic pulmonary fibrosis.

Certain novel compounds of the invention show particularly high activities as $EP_4$ receptor agonists.

The compounds of the invention have been demonstrated to have activity as $EP_4$ receptor agonists. Compounds of the invention also possess low gastrointestinal permeability, as demonstrated by Caco-2 studies. It is therefore believed that the compounds of the invention exhibit low systemic bioavailability when administered orally. Functional agonism of $EP_4$ receptors expressed in the gastrointestinal tract has the potential to treat a range of gastrointestinal disorders. The combination of $EP_4$ receptor agonist activity and low gastrointestinal permeability suggests that compounds of the invention are useful for the treatment of a range of gastrointestinal disorders without cardiovascular side effects arising from systemic distribution.

In one aspect, the invention includes a compound of the Formula I:

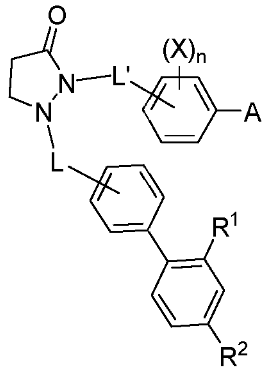
I

or a pharmaceutically acceptable salt, solvate, hydrate, tautomer or optical isomer thereof, wherein;

A is OR', C(O)R', $CO_2R'$, C(O)N(R')$_2$, C(O)N(R')S(O)$_2R^3$, S(O)$_2$R', S(O)$_2$OR', $SO_2N(R')_2$, $C_{1-8}$ alkyl, cycloalkyl, heterocycloalkyl, aryl, or heteroaryl;
X is halo, OR', COOR', or $C_{1-6}$ alkyl;
L and L' are each independently a $C_{2-4}$ alkylene;
$R^1$ is H, halo, CN, $NO_2$, OR', SR', COOR', $C_{1-6}$ alkoxy, or $C_{1-6}$ alkyl;
$R^2$ is OR', OC(O)$R^3$, OC(O)O$R^3$, $CO_2R'$, CON(R')$_2$, $SO_2N(R')_2$, $SO_2R^3$, $OSO_2R^3$, or $OSO_2N(R')_2$;
$R^3$ is $C_{1-6}$ alkyl, $C_{3-6}$ cycloalkyl, or a phenyl;
R' is H, $C_{1-6}$ alkyl, or $C_{3-6}$ cycloalkyl; and
n is 0, 1, 2, or 3;
wherein at each occurrence, alkyl, alkylene, alkoxy and cycloalkyl are each optionally and independently substituted with up to 3 instances of OH, SH, CN, $NO_2$, COOH, halo, or $COOC_{1-4}$ alkyl;
wherein at each occurrence, heterocycloalkyl, aryl, and heteroaryl are each optionally and independently substituted with up to 3 instances of OR', SR', CN, $NO_2$, $CO_2R'$, halo, $C_{1-4}$ alkyl, or oxo.

In some embodiments, the compound is a compound of Formula (I), Formula (Ia), Formula (Ib), Formula (Ic), Formula (1), Formula (1a), Formula (1b), Formula (1c), Formula (2a), Formula (2b), Formula (2c), Formula (2d), Formula (3a), Formula (3b), Formula (3c), Formula (3d), Formula (3e), Formula (3f), Formula (5), Formula (5a), Formula (5b), Formula (6), Formula (6a), Formula (6b) or a pharmaceutically acceptable salt or tautomer thereof.

In some embodiments, the compound is a compound of Formula (I), Formula (Ia), Formula (Ib), Formula (Ic), Formula (1), Formula (1a), Formula (1b), Formula (1c), Formula (2a), Formula (2b), Formula (2c), Formula (2d), Formula (3a), Formula (3b), Formula (3c), Formula (3d), Formula (3e), Formula (3f), Formula (5), Formula (5a), Formula (5b), Formula (6), Formula (6a), Formula (6b) or a pharmaceutically acceptable salt thereof.

In some embodiments, L' is the group

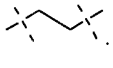

In some embodiments, L is the group

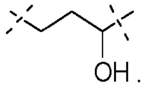

In one embodiment of this aspect, the compound is a compound of Formula (1):

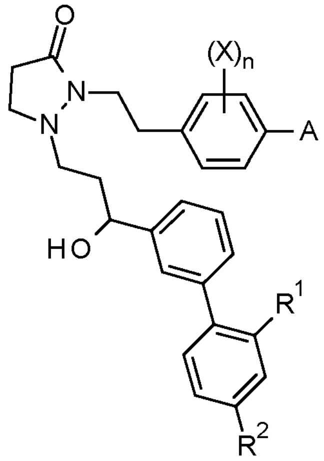

(1)

or a pharmaceutically acceptable salt, solvate, hydrate, tautomer or optical isomer thereof.

In some embodiments, A is selected from C(O)OR', C(O)N(R')S(O)$_2$R$^3$, S(O)$_2$OR', C$_{1-8}$ alkyl, heterocycloalkyl, or heteroaryl, wherein the heterocycloalkyl, and heteroaryl are each optionally and independently substituted with up to 3 instances of OR', SR', halo, C$_{1-4}$ alkyl, or oxo.

In some embodiments, A is selected from C(O)OR' and heteroaryl.

In some embodiments, A is selected from the group consisting of:

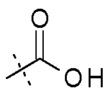 , 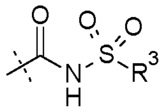 , 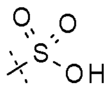 , 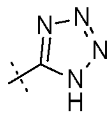 , 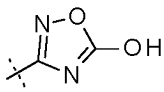 , 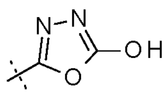 ,

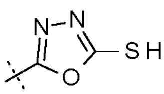 , 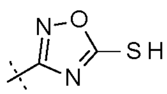 , 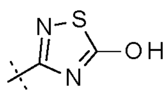 , 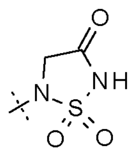 , 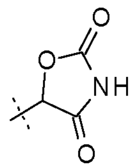 .

In some embodiments, A is:

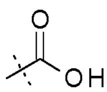 or 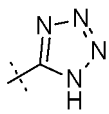 .

In some embodiments, A is

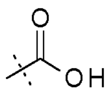 .

In some embodiments, the compound of Formula (I) is a compound of Formula (Ia), (Ib) or (Ic):

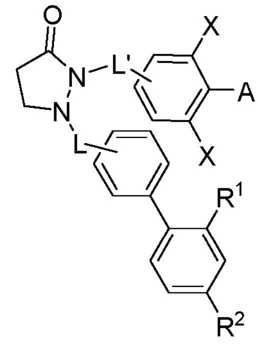 (Ia) 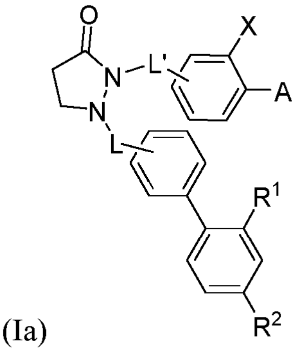 (Ib) 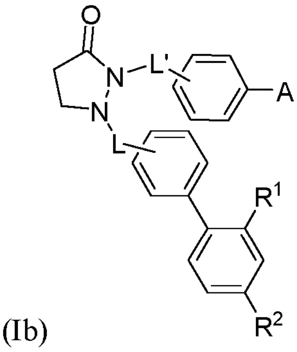 (Ic)

or a pharmaceutically acceptable salt, solvate, hydrate, tautomer or optical isomer thereof.

In some embodiments, the compound of Formula (I) is a compound of Formula (1a), (1b) or (1c):

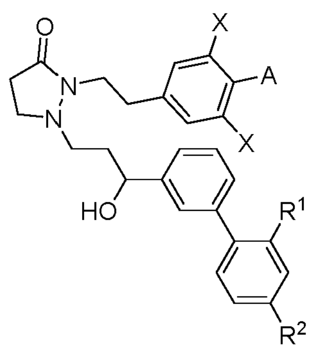 (1a) 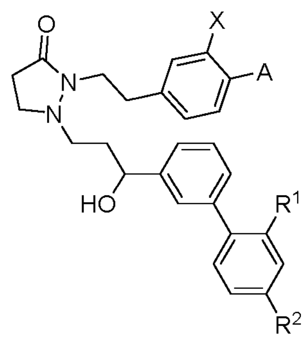 (1b) 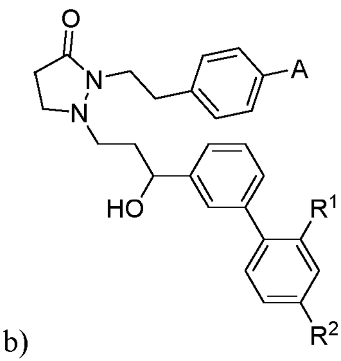 (1c)

or a pharmaceutically acceptable salt, solvate, hydrate, tautomer or optical isomer thereof.

In some embodiments, the compound of Formula (I) is a compound of Formula (3a), (3b), (3c), (3d), (3e) or (3f):

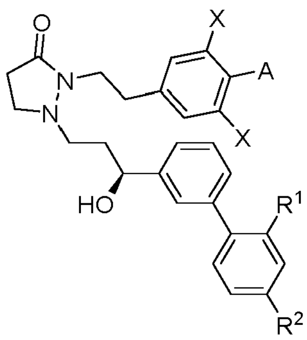 (3a) 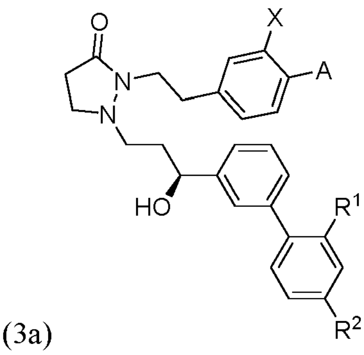 (3b) 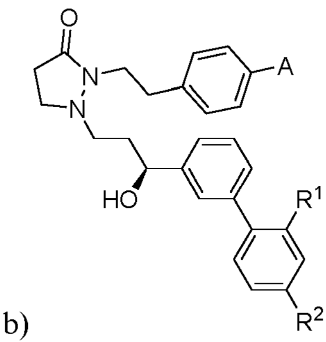 (3c)

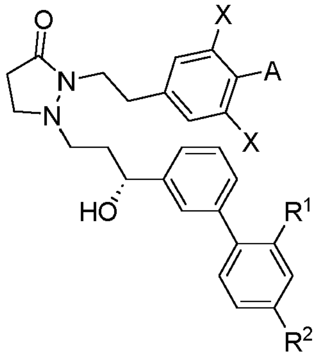 (3d) 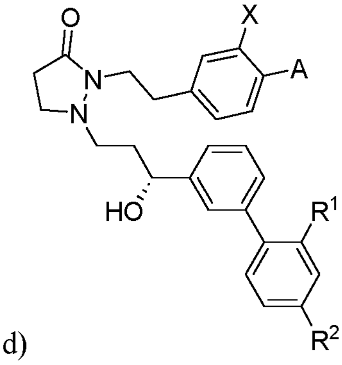 (3e) 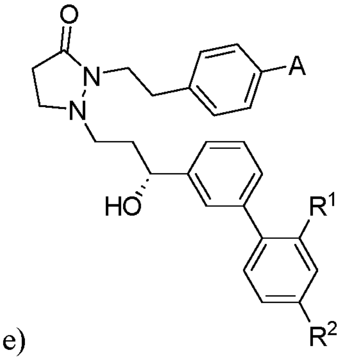 (3f)

or a pharmaceutically acceptable salt, solvate, hydrate, tautomer or optical isomer thereof.

In some embodiments, X is halo or OR'.

In some embodiments, X is F, Cl, or OH.
In some embodiments, X is F or OH, and n is 0, 1 or 2.
In some embodiments, X is F, Cl or OH, and n is 1, or 2.
In some embodiments, X is F or OH, and n is 1, or 2.
In some embodiments n is 0.
In some embodiments, $R^1$ is H, OH, halo, CN, $C_{1-6}$ alkoxy optionally substituted with 1-3 fluorine atoms or $C_{1-6}$ alkyl optionally substituted with 1-3 fluorine atoms.
In some embodiments, $R^1$ is H, OH, halo, CN, $C_{1-6}$ alkyl optionally substituted with 1-3 fluorine atoms or $C_{1-6}$ alkyl optionally substituted with 1-3 fluorine atoms.
In some embodiments, $R^1$ is methyl.
In some embodiments $R^2$ is OR', $CON(R')_2$, $SO_2N(R')_2$, or $OSO_2N(R')_2$.
In some embodiments $R^2$ is OH, $CONH_2$, $SO_2NH_2$, or $OSO_2NH_2$.
In some embodiments, $R^2$ is $CON(R')_2$, $SO_2N(R')_2$, $OSO_2R^3$, or $OSO_2N(R')_2$.
In some embodiments, $R^2$ is $CONH_2$, $SO_2NH_2$, or $OSO_2NH_2$.
In some embodiments, $R^2$ is $CONH_2$.
In some embodiments, provided herein is a compound of Formula (2a), (2b), (2c), or (2d):

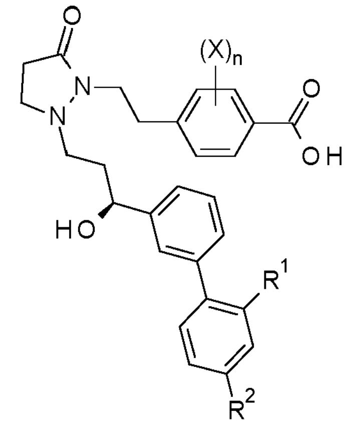
(2a)

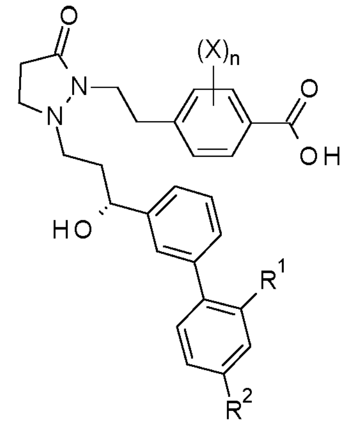
(2b)

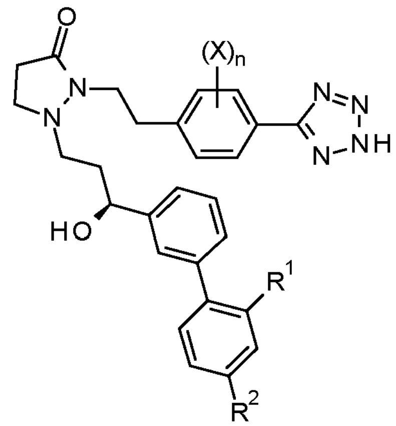
(2c)

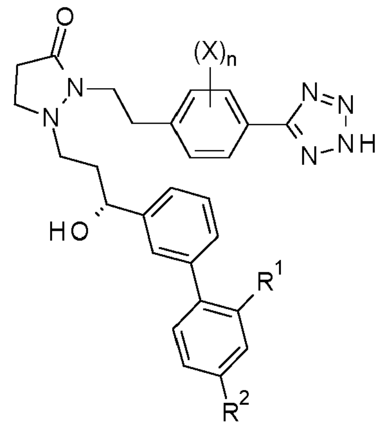
(2d)

or pharmaceutically acceptable salt, solvate, hydrate or tautomer thereof.
In some embodiments, $R^1$ is H or $C_{1-6}$ alkyl. In a further embodiment, $R^1$ is methyl. In another embodiment, n is 0.
In some embodiments, provided herein is compound of Formula (5), (5a), (5b), (6), (6a), (6b):

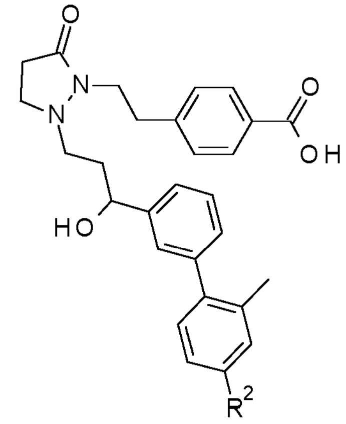

(5)

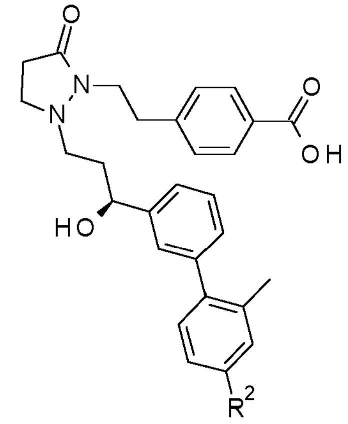

(5a)

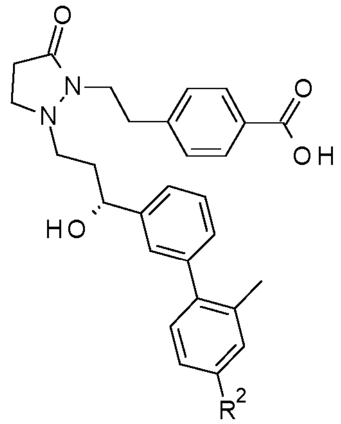

(5b)

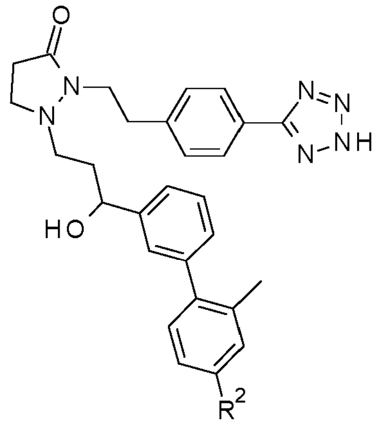

(6)

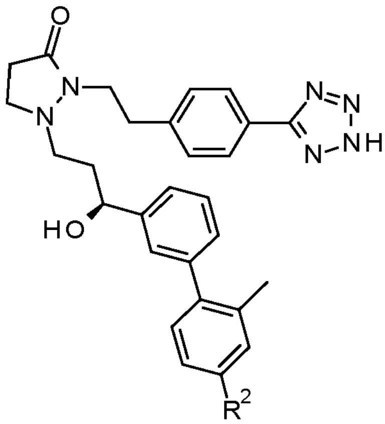

(6a)

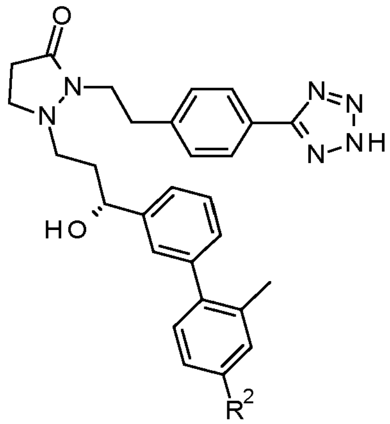

(6b)

or pharmaceutically acceptable salt, solvate, hydrate or tautomer thereof.

In some embodiments, $R^2$ is $CO_2R'$, $CON(R')_2$, $SO_2N(R')_2$ or $OSO_2N(R')_2$. In a further embodiment, $R^2$ is $CONH_2$, $SO_2NH_2$ or $OSO_2NH_2$.

In some embodiments, provided herein is compounds selected from the group consisting of:

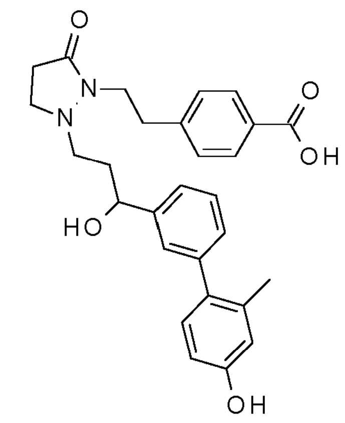
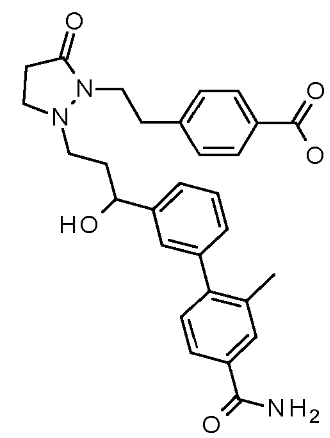
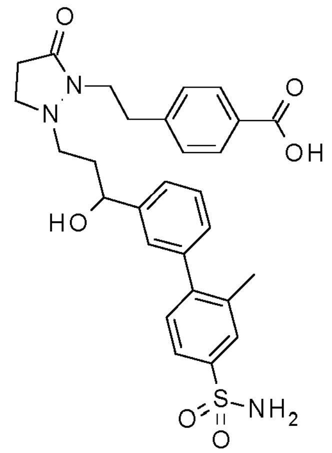
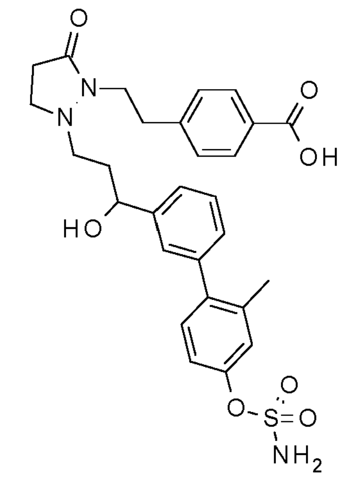
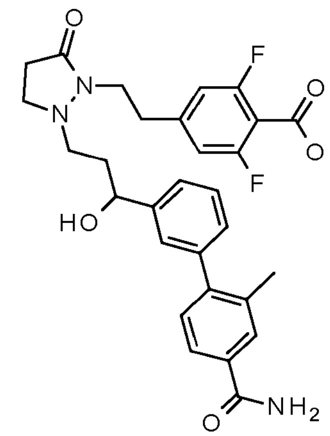
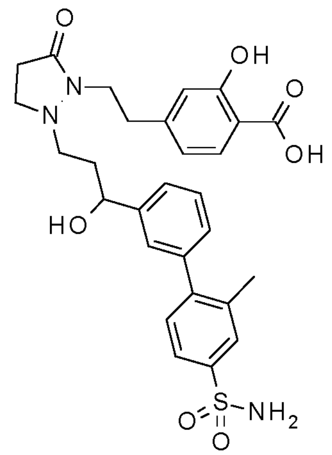
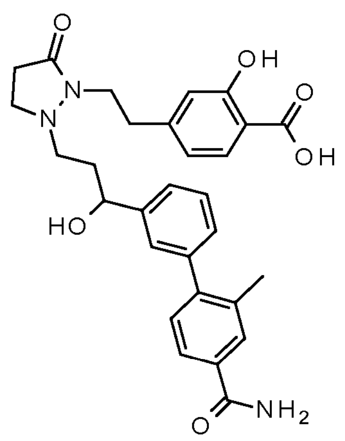
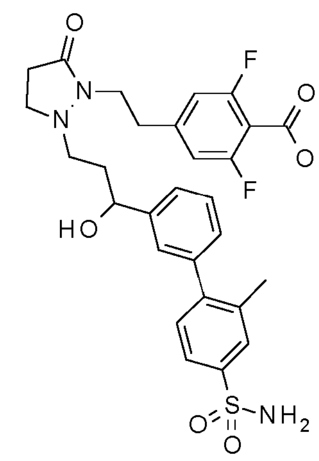
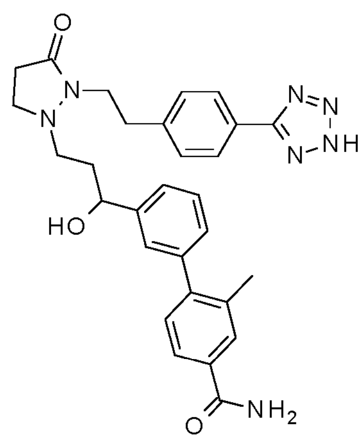

or a pharmaceutically acceptable salt, solvate, hydrate, tautomer or optical isomer thereof; or a pharmaceutically acceptable salt thereof.

In some embodiments, the invention includes a pharmaceutical composition comprising a compound described herein, and a pharmaceutically acceptable excipient.

In some embodiments, the pharmaceutical composition further comprises at least one additional therapeutic agent selected from the group consisting of aminosalicylates, corticosteroids, immunomodulators and combinations thereof.

In some embodiments, the invention includes a compound or composition as described herein, for use in a method of modulating EP4 receptor agonist activity in a biological sample, the method comprising contacting said EP4 receptor with a compound or composition described herein.

In some embodiments, the invention includes a compound or composition as described herein, for use in a method of treating an EP4 receptor mediated disease, the method comprising administering to a patient in need thereof a compound or composition described herein.

In some embodiments, the EP4 receptor mediated disease is a gastrointestinal disorder. In a further embodiment, the gastrointestinal disorder is selected from the group consisting of constipation disorders, constipation-predominant irritable bowel syndrome, mixed type irritable bowel syndrome, chronic idiopathic constipation, gastrointestinal symptoms associated with Parkinson's disease, gastrointestinal symptoms associated with cystic fibrosis, intestinal dysmotility, postoperative ileus, food allergy or food intolerance, celiac disease, gastrointestinal motility disorders, functional gastrointestinal disorders, drug induced enteropathy, NSAID induced gastric and intestinal injury, chemotherapy induced mucositis, gastroesophageal reflux disease (GERD), duodenogastric reflux, diarrhoeal diseases, immune mediated gastrointestinal diseases, Crohn's disease, ulcerative colitis, inflammatory bowel disease and ischemic colitis, or pulmonary diseases and conditions such as chronic obstructive pulmonary diseases, asthma, chronic bronchitis, cystic fibrosis, emphysema, chronic idiopathic cough, hyperactive airway disorder and idiopathic pulmonary fibrosis.

In some embodiments, the compound of Formula I is a compound listed in Table 1 or a pharmaceutically acceptable salt, solvate, hydrate or tautomer thereof.

In some embodiments, provided herein is compounds selected from the compounds listed in Table 1 or a pharmaceutically acceptable salt thereof:

Table 1: Exemplary compounds of Formula (I)

| Racemic Compound | *S* isomer | *R* isomer |
|---|---|---|
| 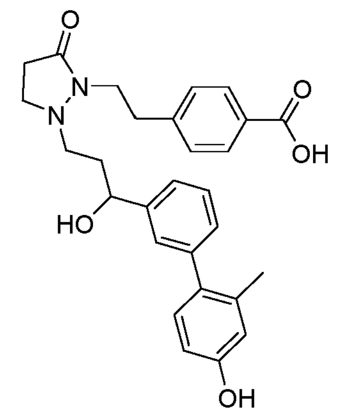 **1** | 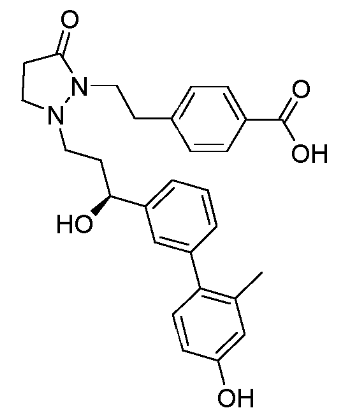 **1S** | 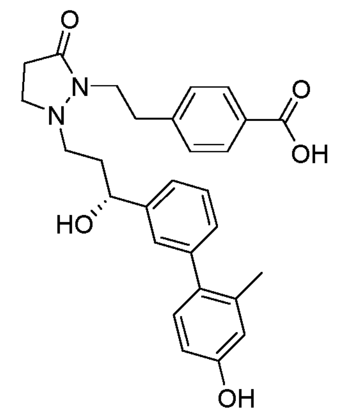 **1R** |
| 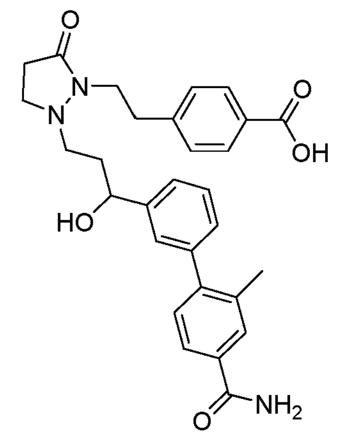 **2** | 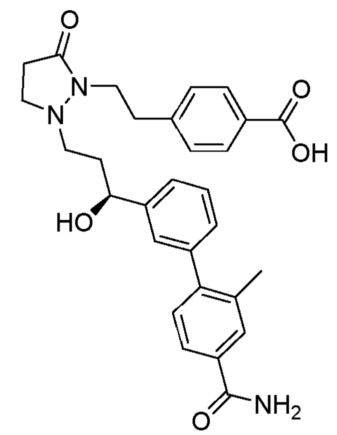 **2S** | 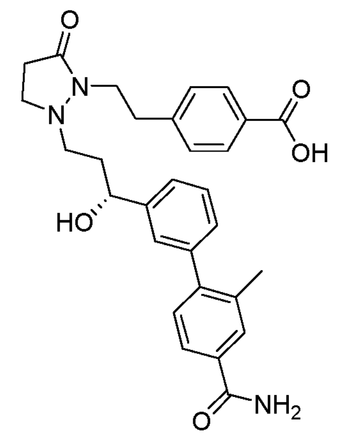 **2R** |

(continued)

| Racemic Compound | *S* isomer | *R* isomer |
|---|---|---|
| 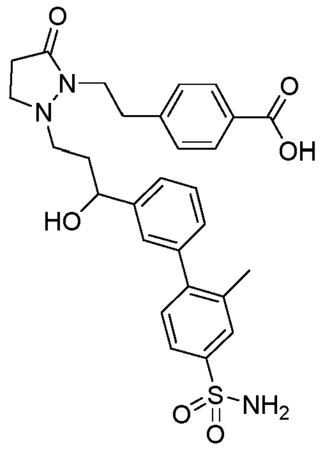<br>**3** | 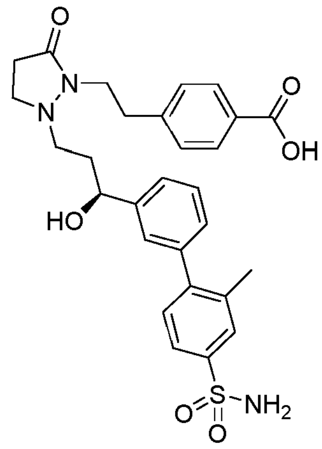<br>**3S** | 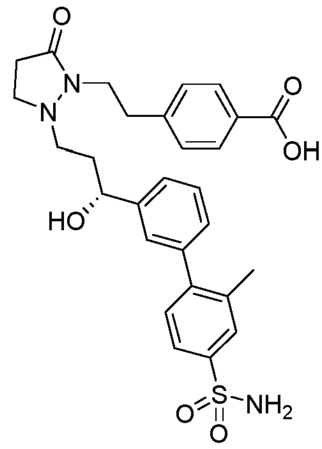<br>**3R** |
| 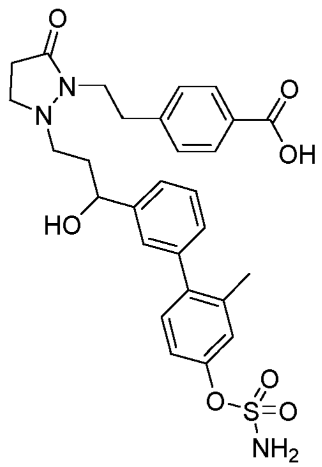<br>**4** | 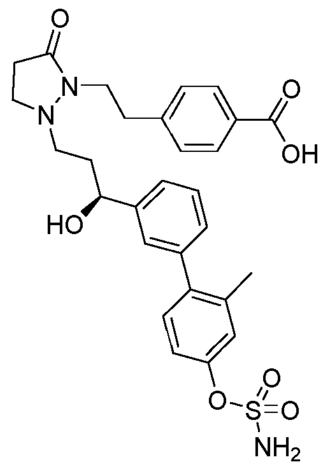<br>**4S** | 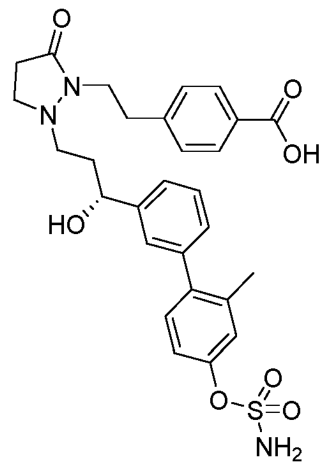<br>**4R** |
| 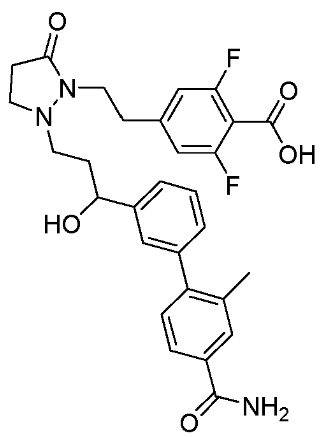<br>**5** | 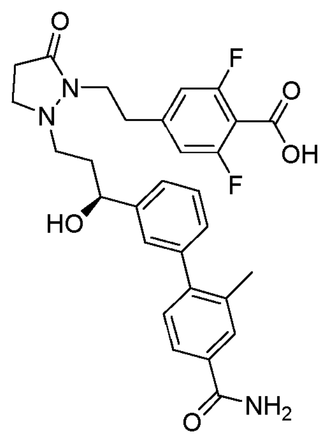<br>**5S** | 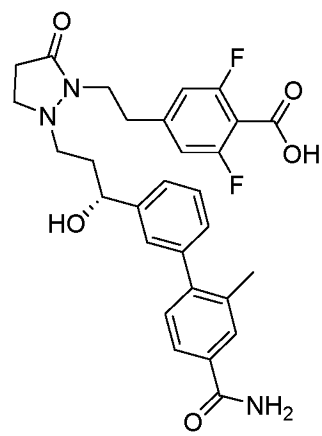<br>**5R** |

(continued)

| Racemic Compound | *S* isomer | *R* isomer |
|---|---|---|
| 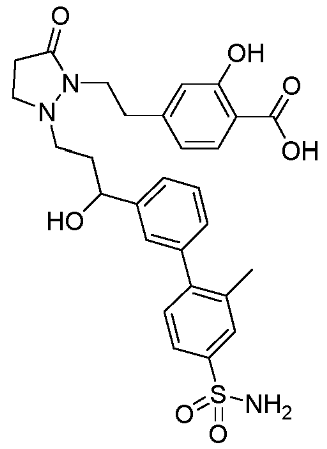 **6** | 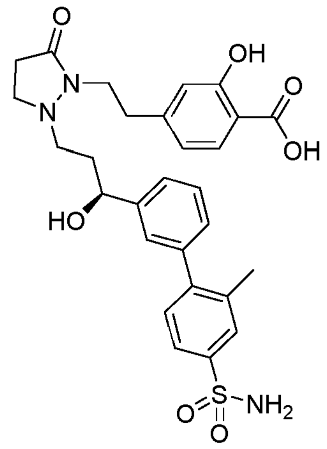 **6S** | 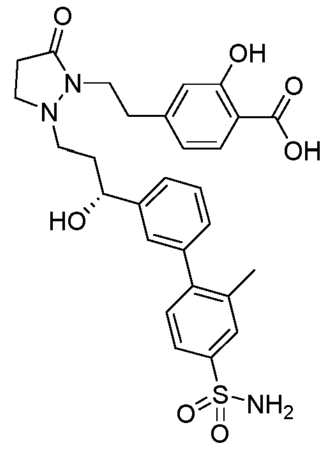 **6R** |
| 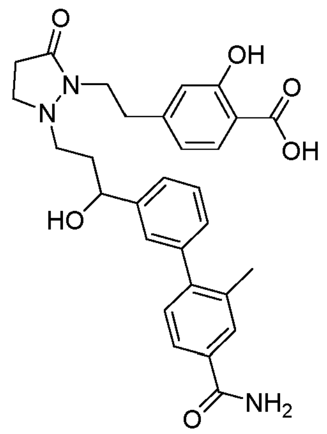 **7** | 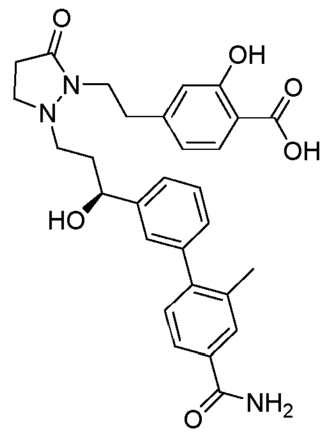 **7S** | 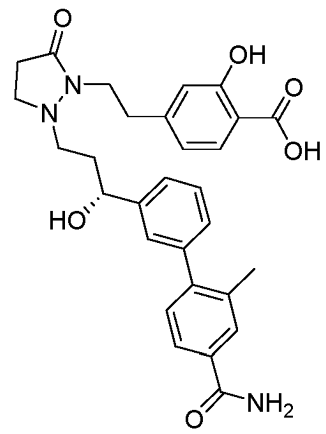 **7R** |
| 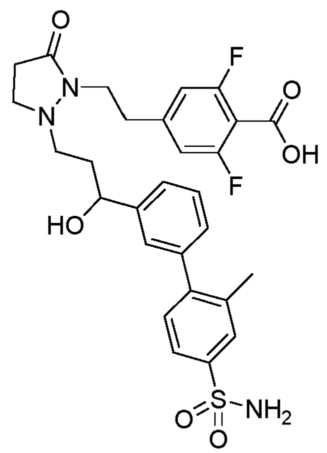 **8** | 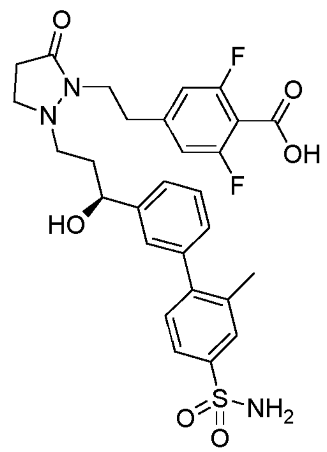 **8S** | 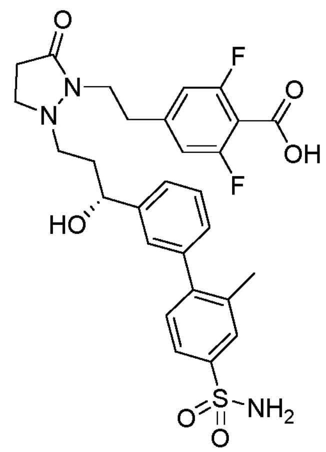 **8R** |

(continued)

| Racemic Compound | *S* isomer | *R* isomer |
|---|---|---|
| 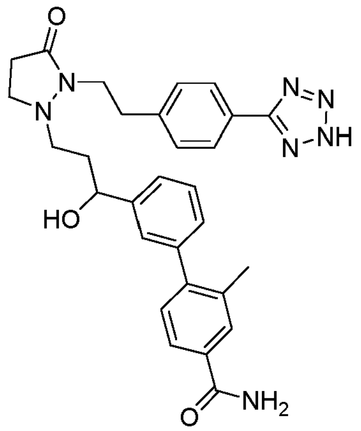 **9** | 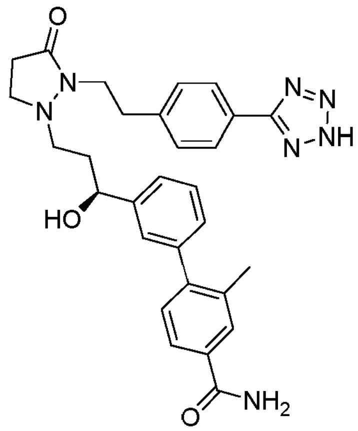 **9S** | 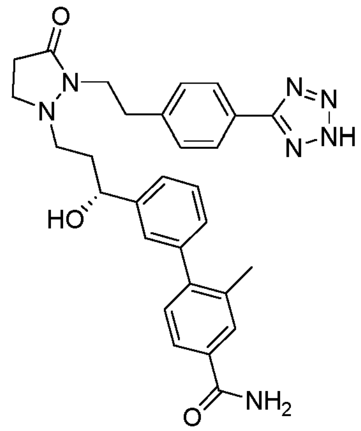 **9R** |

or a pharmaceutically acceptable salt thereof.

ABBREVIATIONS

| | |
|---|---|
| aq | aqueous |
| Bn | benzyl |
| DCM | dichloromethane |
| DMA | dimethylacetamide |
| DMF | dimethylformamide |
| dppf | 1,1'-bis(diphenylphosphino)ferrocene |
| EDCI | 1-ethyl-3-(3-dimethylaminopropyl)carbodiimide |
| EtOAc | ethyl acetate |
| FA | formic acid |
| HATU | 1-[Bis(dimethylamino)methylene]-1H-1,2,3-triazolo[4,5-b]pyridinium 3-oxide hexafluorophosphate, |
| HOBt | hydroxybenzotriazole |
| HPLC | high performance liquid chromatography |
| hr | hour |
| hrs | hours |
| LCMS | liquid chromatography mass spectrometry |
| M | molar |
| MeCN | acetonitrile |
| MeOH | methanol |
| N | normal |
| NBS | N-bromosuccinimide |
| prep HPLC | preparative high-performance liquid chromatography |
| RT | room temperature |
| sat | saturated |
| THF | tetrahydrofuran |
| UPLC | ultra performance liquid chromatography |

DEFINITIONS

In this application, the following definitions apply, unless indicated otherwise.

The term "treatment", in relation to the uses of any of the compounds described herein, including those of Formula (I), Formula (Ia), Formula (Ib), Formula (Ic), Formula (1), Formula (1a), Formula (1b), Formula (1c), Formula (2a), Formula (2b), Formula (2c), Formula (2d), Formula (3a), Formula (3b), Formula (3c), Formula (3d), Formula (3e), Formula

(3f), Formula (5), Formula (5a), Formula (5b), Formula (6), Formula (6a) and Formula (6b) is used to describe any form of intervention where a compound is administered to a subject suffering from, or at risk of suffering from, or potentially at risk of suffering from the disease or disorder in question. Thus, the term "treatment" covers both preventative (prophylactic) treatment and treatment where measurable or detectable symptoms of the disease or disorder are being displayed.

The term "effective therapeutic amount" (for example in relation to methods of treatment of a disease or condition) refers to an amount of the compound which is effective to produce a desired therapeutic effect. For example, if the condition is pain, then the effective therapeutic amount is an amount sufficient to provide a desired level of pain relief. The desired level of pain relief may be, for example, complete removal of the pain or a reduction in the severity of the pain.

As used herein, the term "hydroxyl" or "hydroxy" refers to an -OH moiety.

As used herein, an "alkyl" group refers to a saturated aliphatic hydrocarbon group containing 1-12 (e.g., 1-8, 1-6, or 1-4) carbon atoms. An alkyl group can be straight or branched. Examples of alkyl groups include, but are not limited to, methyl, ethyl, propyl, isopropyl, butyl, isobutyl, sec-butyl, tert-butyl, n-pentyl, n-heptyl, or 2-ethylhexyl. An alkyl group can be substituted (i.e., optionally substituted) with one or more substituents such as halo, phospho, cycloaliphatic [e.g., cycloalkyl or cycloalkenyl], heterocycloaliphatic [e.g., heterocycloalkyl or heterocycloalkenyl], aryl, heteroaryl, alkoxy, aroyl, heteroaroyl, acyl [e.g., (aliphatic)carbonyl, (cycloaliphatic)carbonyl, or (heterocycloaliphatic)carbonyl], nitro, cyano, amido [e.g., (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino alkylaminocarbonyl, cycloalkylaminocarbonyl, heterocycloalkylaminocarbonyl, arylaminocarbonyl, or heteroarylaminocarbonyl], amino [e.g., aliphaticamino, cycloaliphaticamino, or heterocycloaliphaticamino], sulfonyl [e.g., aliphatic-$SO_2$-], sulfinyl, sulfanyl, sulfoxy, urea, thiourea, sulfamoyl, sulfamide, oxo, carboxy, carbamoyl, cycloaliphaticoxy, heterocycloaliphaticoxy, aryloxy, heteroaryloxy, aralkyloxy, heteroarylalkoxy, alkoxycarbonyl, alkylcarbonyloxy, or hydroxy. Without limitation, some examples of substituted alkyls include carboxyalkyl (such as HOOC-alkyl, alkoxycarbonylalkyl, and alkylcarbonyloxyalkyl), cyanoalkyl, hydroxyalkyl, alkoxyalkyl, acylalkyl, aralkyl, (alkoxyaryl)alkyl, (sulfonylamino)alkyl (such as (alkyl-$SO_2$-amino)alkyl), aminoalkyl, amidoalkyl, (cycloaliphatic)alkyl, or haloalkyl.

As used herein, an "alkylene" group refers to a bivalent branched or straight alkyl group that contains 2-12 (e.g. 2-8, 2-6, or 2-4) carbon atoms, and serves to connect two chemical moieties. Examples of alkylene groups include, but are not limited to methylene, ethylene, propylene, butylene, isopropylene (methylethylene), and isobutylene (2-methylpropylene). An alkylene group can be substituted (i.e., optionally substituted) with one or more substituents as defined in the alkyl group.

As used herein, an "amido" encompasses both "aminocarbonyl" and "carbonylamino." These terms when used alone or in connection with another group refer to an amido group such as -N($R^X$)-C(O)-$R^Y$ or -C(O)-N($R^X$)$_2$, when used terminally, and -C(O)-N($R^X$)- or -N($R^X$)-C(O)-when used internally, wherein $R^X$ and $R^Y$ can be aliphatic, cycloaliphatic, aryl, araliphatic, heterocycloaliphatic, heteroaryl or heteroaraliphatic. Examples of amido groups include alkylamido (such as alkylcarbonylamino or alkylaminocarbonyl), (heterocycloaliphatic)amido, (heteroaralkyl)amido, (heteroaryl)amido, (heterocycloalkyl)alkylamido, arylamido, aralkylamido, (cycloalkyl)alkylamido, or cycloalkylamido.

As used herein, an "amino" group refers to -$NR^XR^Y$ wherein each of $R^X$ and $R^Y$ is independently hydrogen, aliphatic, cycloaliphatic, (cycloaliphatic)aliphatic, aryl, araliphatic, heterocycloaliphatic, (heterocycloaliphatic)aliphatic, heteroaryl, carboxy, sulfanyl, sulfinyl, sulfonyl, (aliphatic)carbonyl, (cycloaliphatic)carbonyl, ((cycloaliphatic)aliphatic) carbonyl, arylcarbonyl, (araliphatic)carbonyl, (heterocycloaliphatic)carbonyl, ((heterocycloaliphatic)aliphatic)carbonyl, (heteroaryl)carbonyl, or (heteroaraliphatic)carbonyl, each of which being defined herein and being optionally substituted. Examples of amino groups include alkylamino, dialkylamino, or arylamino. When the term "amino" is not the terminal group (e.g., alkylcarbonylamino), it is represented by -$NR^X$-, where $R^X$ has the same meaning as defined above.

As used herein, an "aryl" group used alone or as part of a larger moiety as in "aralkyl," "aralkoxy," or "aryloxyalkyl" refers to monocyclic (e.g., phenyl); bicyclic (e.g., indenyl, naphthalenyl, tetrahydronaphthyl, tetrahydroindenyl); and tricyclic (e.g., fluorenyl tetrahydrofluorenyl, or tetrahydroanthracenyl, anthracenyl) ring systems in which the monocyclic ring system is aromatic or at least one of the rings in a bicyclic or tricyclic ring system is aromatic. The bicyclic and tricyclic groups include benzofused 2-3 membered carbocyclic rings. For example, a benzofused group includes phenyl fused with two or more $C_{4-8}$ carbocyclic moieties. An aryl is optionally substituted with one or more substituents including aliphatic [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic ring of a benzofused bicyclic or tricyclic aryl); nitro; carboxy; amido; acyl [e.g., (aliphatic)carbonyl; (cycloaliphatic)carbonyl; ((cycloaliphatic)aliphatic) carbonyl; (araliphatic)carbonyl; (heterocycloaliphatic)carbonyl; ((heterocycloaliphatic)aliphatic)carbonyl; or (heteroaraliphatic)carbonyl]; sulfonyl [e.g., aliphatic-$SO_2$- or amino-$SO_2$-]; sulfinyl [e.g., aliphatic-S(O)- or cycloaliphatic-S(O)-]; sulfanyl [e.g., aliphatic-S-]; cyano; halo; hydroxy; mercapto; sulfoxy; urea; thiourea; sulfamoyl; sulfamide; or carbamoyl. Alternatively, an aryl can be unsubstituted.

Non-limiting examples of substituted aryls include haloaryl [e.g., mono-, di (such as *p,m*-dihaloaryl), and (trihalo) aryl]; (carboxy)aryl [e.g., (alkoxycarbonyl)aryl, ((aralkyl)carbonyloxy)aryl, and (alkoxycarbonyl)aryl]; (amido)aryl [e.g.,

(aminocarbonyl)aryl, (((alkylamino)alkyl)aminocarbonyl)aryl, (alkylcarbonyl)aminoaryl, (arylaminocarbonyl)aryl, and (((heteroaryl)amino)carbonyl)aryl]; aminoaryl [e.g., ((alkylsulfonyl)amino)aryl or ((dialkyl)amino)aryl]; (cyanoalkyl)aryl; (alkoxy)aryl; (sulfamoyl)aryl [e.g., (aminosulfonyl)aryl]; (alkylsulfonyl)aryl; (cyano)aryl; (hydroxyalkyl)aryl; ((alkoxy)alkyl)aryl; (hydroxy)aryl, ((carboxy)alkyl)aryl; (((dialkyl)amino)alkyl)aryl; (nitroalkyl)aryl; (((alkylsulfonyl)amino)alkyl)aryl; ((heterocycloaliphatic)carbonyl)aryl; ((alkylsulfonyl)alkyl)aryl; (cyanoalkyl)aryl; (hydroxyalkyl)aryl; (alkylcarbonyl)aryl; alkylaryl; (trihaloalkyl)aryl; *p*-amino-*m*-alkoxycarbonylaryl; *p*-amino-*m*-cyanoaryl; *p*-halo-*m*-aminoaryl; or (m-(heterocycloaliphatic)-o-(alkyl))aryl.

As used herein, a "cycloalkyl" group refers to a saturated carbocyclic mono- or bicyclic (fused or bridged) ring of 3-10 (e.g., 5-10) carbon atoms. Examples of cycloalkyl groups include cyclopropyl, cyclobutyl, cyclopentyl, cyclohexyl, cycloheptyl, adamantyl, norbornyl, cubyl, octahydro-indenyl, decahydro-naphthyl, bicyclo[3.2.1]octyl, bicyclo[2.2.2]octyl, bicyclo[3.3.1]nonyl, bicyclo[3.3.2.]decyl, bicyclo[2.2.2]octyl, adamantyl, or ((aminocarbonyl)cycloalkyl)cycloalkyl.

A cycloalkyl group can be optionally substituted with one or more substituents such as phospho, aliphatic [e.g., alkyl, alkenyl, or alkynyl], cycloaliphatic, (cycloaliphatic) aliphatic, heterocycloaliphatic, (heterocycloaliphatic) aliphatic, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido [e.g., (aliphatic)carbonylamino, (cycloaliphatic)carbonylamino, ((cycloaliphatic)aliphatic)carbonylamino, (aryl)carbonylamino, (araliphatic)carbonylamino, (heterocycloaliphatic)carbonylamino, ((heterocycloaliphatic)aliphatic)carbonylamino, (heteroaryl)carbonylamino, or (heteroaraliphatic)carbonylamino], nitro, carboxy [e.g., HOOC-, alkoxycarbonyl, or alkylcarbonyloxy], acyl [e.g., (cycloaliphatic)carbonyl, ((cycloaliphatic) aliphatic)carbonyl, (araliphatic)carbonyl, (heterocycloaliphatic)carbonyl, ((heterocycloaliphatic)aliphatic)carbonyl, or (heteroaraliphatic)carbonyl], cyano, halo, hydroxy, mercapto, sulfonyl [e.g., alkyl-$SO_2$- and aryl-$SO_2$-], sulfinyl [e.g., alkyl-S(O)-], sulfanyl [e.g., alkyl-S-], sulfoxy, urea, thiourea, sulfamoyl, sulfamide, oxo, or carbamoyl.

As used herein, a "heterocycloalkyl" group refers to a 3-10 membered mono- or bicylic (fused or bridged) (e.g., 5- to 10-membered mono- or bicyclic) saturated ring structure, in which one or more of the ring atoms is a heteroatom (e.g., N, O, S, or combinations thereof). Examples of a heterocycloalkyl group include piperidyl, piperazyl, tetrahydropyranyl, tetrahydrofuryl, 1,4-dioxolanyl, 1,4-dithianyl, 1,3-dioxolanyl, oxazolidyl, isoxazolidyl, morpholinyl, thiomorpholyl, octahydrobenzofuryl, octahydrochromenyl, octahydrothiochromenyl, octahydroindolyl, octahydropyrindinyl, decahydroquinolinyl, octahydrobenzo[b]thiopheneyl, 2-oxa-bicyclo[2.2.2]octyl, 1-aza-bicyclo[2.2.2]octyl, 3-aza-bicyclo[3.2.1]octyl, and 2,6-dioxa-tricyclo[3.3.1.$0^{3,7}$]nonyl. A monocyclic heterocycloalkyl group can be fused with a phenyl moiety to form structures, such as tetrahydroisoquinoline, that would be categorized as heteroaryls.

A heterocycloalkyl group can be optionally substituted with one or more substituents such as phospho, aliphatic [e.g., alkyl, alkenyl, or alkynyl], cycloaliphatic, (cycloaliphatic)aliphatic, heterocycloaliphatic, (heterocycloaliphatic)aliphatic, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido [e.g., (aliphatic)carbonylamino, (cycloaliphatic)carbonylamino, ((cycloaliphatic) aliphatic)carbonylamino, (aryl)carbonylamino, (araliphatic)carbonylamino, (heterocycloaliphatic)carbonylamino, ((heterocycloaliphatic) aliphatic)carbonylamino, (heteroaryl)carbonylamino, or (heteroaraliphatic)carbonylamino], nitro, carboxy [e.g., HOOC-, alkoxycarbonyl, or alkylcarbonyloxy], acyl [e.g., (cycloaliphatic)carbonyl, ((cycloaliphatic) aliphatic)carbonyl, (araliphatic)carbonyl, (heterocycloaliphatic)carbonyl, ((heterocycloaliphatic)aliphatic)carbonyl, or (heteroaraliphatic)carbonyl], nitro, cyano, halo, hydroxy, mercapto, sulfonyl [e.g., alkylsulfonyl or arylsulfonyl], sulfinyl [e.g., alkylsulfinyl], sulfanyl [e.g., alkylsulfanyl], sulfoxy, urea, thiourea, sulfamoyl, sulfamide, oxo, or carbamoyl.

A "heteroaryl" group, as used herein, refers to a monocyclic, bicyclic, or tricyclic ring system having 4 to 15 ring atoms wherein one or more of the ring atoms is a heteroatom (e.g., N, O, S, or combinations thereof) and in which the monocyclic ring system is aromatic or at least one of the rings in the bicyclic or tricyclic ring systems is aromatic. A heteroaryl group includes a benzofused ring system having 2 to 3 rings. For example, a benzofused group includes benzo fused with one or two 4 to 8 membered heterocycloaliphatic moieties (e.g., indolizyl, indolyl, isoindolyl, 3H-indolyl, indolinyl, benzo[b]furyl, benzo[b]thiophene-yl, quinolinyl, or isoquinolinyl). Some examples of heteroaryl are azetidinyl, pyridyl, 1H-indazolyl, furyl, pyrrolyl, thienyl, thiazolyl, oxazolyl, imidazolyl, tetrazolyl, benzofuryl, isoquinolinyl, benzthiazolyl, xanthene, thioxanthene, phenothiazine, dihydroindole, benzo[1,3]dioxole, benzo[b]furyl, benzo[b]thiophenyl, indazolyl, benzimidazolyl, benzthiazolyl, puryl, cinnolyl, quinolyl, quinazolyl, cinnolyl, phthalazyl, quinazolyl, quinoxalyl, isoquinolyl, 4H-quinolizyl, benzo-1,2,5-thiadiazolyl, or 1,8-naphthyridyl.

Without limitation, monocyclic heteroaryls include furyl, thiophene-yl, 2H-pyrrolyl, pyrrolyl, oxazolyl, thazolyl, imidazolyl, pyrazolyl, isoxazolyl, isothiazolyl, 1,3,4-thiadiazolyl, 2H-pyranyl, 4-H-pranyl, pyridyl, pyridazyl, pyrimidyl, pyrazolyl, pyrazyl, or 1,3,5-triazyl. Monocyclic heteroaryls are numbered according to standard chemical nomenclature.

Without limitation, bicyclic heteroaryls include indolizyl, indolyl, isoindolyl, 3H-indolyl, indolinyl, benzo[b]furyl, benzo[b]thiophenyl, quinolinyl, isoquinolinyl, indolizyl, isoindolyl, indolyl, benzo[b]furyl, bexo[b]thiophenyl, indazolyl, benzimidazyl, benzthiazolyl, purinyl, 4H-quinolizyl, quinolyl, isoquinolyl, cinnolyl, phthalazyl, quinazolyl, quinoxalyl, 1,8-naphthyridyl, or pteridyl. Bicyclic heteroaryls are numbered according to standard chemical nomenclature.

A heteroaryl is optionally substituted with one or more substituents such as aliphatic [e.g., alkyl, alkenyl, or alkynyl]; cycloaliphatic; (cycloaliphatic)aliphatic; heterocycloaliphatic; (heterocycloaliphatic)aliphatic; aryl; heteroaryl;

alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic or heterocyclic ring of a bicyclic or tricyclic heteroaryl); carboxy; amido; acyl [ e.g., aliphaticcarbonyl; (cycloaliphatic)carbonyl; ((cycloaliphatic)aliphatic)carbonyl; (araliphatic) carbonyl; (heterocycloaliphatic)carbonyl; ((heterocycloaliphatic)aliphatic)carbonyl; or (heteroaraliphatic)carbonyl]; sulfonyl [e.g., aliphaticsulfonyl or aminosulfonyl]; sulfinyl [e.g., aliphaticsulfinyl]; sulfanyl [e.g., aliphaticsulfanyl]; nitro; cyano; halo; hydroxy; mercapto; sulfoxy; urea; thiourea; sulfamoyl; sulfamide; or carbamoyl. Alternatively, a heteroaryl can be unsubstituted.

Non-limiting examples of substituted heteroaryls include (halo)heteroaryl [e.g., mono-and di-(halo)heteroaryl]; (carboxy)heteroaryl [e.g., (alkoxycarbonyl)heteroaryl]; cyanoheteroaryl; aminoheteroaryl [e.g., ((alkylsulfonyl)amino) heteroaryl and ((dialkyl)amino)heteroaryl]; (amido)heteroaryl [e.g., aminocarbonylheteroaryl, ((alkylcarbonyl)amino) heteroaryl, ((((alkyl)amino)alkyl)aminocarbonyl)heteroaryl, (((heteroaryl)amino)carbonyl)heteroaryl, ((heterocycloaliphatic)carbonyl)heteroaryl, and ((alkylcarbonyl)amino)heteroaryl]; (cyanoalkyl)heteroaryl; (alkoxy)heteroaryl; (sulfamoyl)heteroaryl [e.g., (aminosulfonyl)heteroaryl]; (sulfonyl)heteroaryl [e.g., (alkylsulfonyl)heteroaryl]; (hydroxyalkyl)heteroaryl; (alkoxyalkyl)heteroaryl; (hydroxy)heteroaryl; ((carboxy)alkyl)heteroaryl; (((dialkyl)amino)alkyl]heteroaryl; (heterocycloaliphatic)heteroaryl; (cycloaliphatic)heteroaryl; (nitroalkyl)heteroaryl; (((alkylsulfonyl)amino)alkyl)heteroaryl; ((alkylsulfonyl)alkyl)heteroaryl; (cyanoalkyl)heteroaryl; (acyl)heteroaryl [e.g., (alkylcarbonyl)heteroaryl]; (alkyl)heteroaryl; or (haloalkyl)heteroaryl [e.g., trihaloalkylheteroaryl].

As used herein, an "alkoxy" group refers to an alkyl-O- group where "alkyl" has been defined previously.

As used herein, a "carboxy" group refers to -COOH, -COOR$^X$, -OC(O)H, -OC(O)R$^X$, when used as a terminal group; or -OC(O)- or -C(O)O- when used as an internal group.

As used herein, a "mercapto" group refers to -SH.

As used herein, a "sulfo" group refers to -$SO_3$H or -$SO_3$R$^X$ when used terminally or -S(O)$_3$- when used internally.

As used herein, a "sulfamide" group refers to the structure -NR$^X$-S(O)$_2$-NR$^Y$R$^Z$ when used terminally and -NR$^X$-S(O)$_2$-NR$^Y$- when used internally, wherein R$^X$, R$^Y$, and R$^Z$ have been defined above.

As used herein, a "sulfamoyl" group refers to the structure -O-S(O)$_2$-NR$^Y$R$^z$ wherein R$^Y$ and R$^Z$ have been defined above.

As used herein, a "sulfonamide" group refers to the structure -S(O)$_2$-NRxR$^Y$ or -NR$^X$-S(O)$_2$-R$^Z$ when used terminally; or -S(O)$_2$-NR$^x$- or -NR$^x$ -S(O)$_2$- when used internally, wherein R$^x$, R$^y$, and R$^z$ are defined above.

As used herein a "sulfanyl" group refers to -S-R$^X$ when used terminally and -S- when used internally, wherein R$^X$ has been defined above. Examples of sulfanyls include aliphatic-S-, cycloaliphatic-S-, aryl-S-, or the like.

As used herein, a "halogen" or "halo" group refers to fluorine, chlorine, bromine or iodine.

As used herein, an "oxo" refers to =O.

As used herein, the term "vicinal" generally refers to the placement of substituents on a group that includes two or more carbon atoms, wherein the substituents are attached to adjacent carbon atoms.

As used herein, the term "geminal" generally refers to the placement of substituents on a group that includes two or more carbon atoms, wherein the substituents are attached to the same carbon atom.

The terms "terminally" and "internally" refer to the location of a group within a substituent. A group is terminal when the group is present at the end of the substituent not further bonded to the rest of the chemical structure. Carboxyalkyl, i.e., R$^X$O(O)C-alkyl, is an example of a carboxy group used terminally. A group is internal when the group is present in the middle of a substituent of the chemical structure. Alkylcarboxy (e.g., alkyl-C(O)O- or alkyl-OC(O)-) and alkylcarboxyaryl (e.g., alkyl-C(O)O-aryl- or alkyl-O(CO)-aryl-) are examples of carboxy groups used internally.

As used herein, an "aliphatic chain" refers to a branched or straight aliphatic group (e.g., alkyl groups, alkenyl groups, or alkynyl groups). A straight aliphatic chain has the structure - $[CH_2]_v$-, where v is 1-12. A branched aliphatic chain is a straight aliphatic chain that is substituted with one or more aliphatic groups. A branched aliphatic chain has the structure - $[CQQ]_v$- where Q is independently a hydrogen or an aliphatic group; however, Q shall be an aliphatic group in at least one instance. The term aliphatic chain includes alkyl chains, alkenyl chains, and alkynyl chains, where alkyl, alkenyl, and alkynyl are defined above.

The phrase "optionally substituted" is used herein interchangeably with the phrase "substituted or unsubstituted." As described herein, compounds of the invention can optionally be substituted with one or more substituents, such as are illustrated generally above, or as exemplified by particular classes, subclasses, and species of the invention. Unless otherwise noted, each of the specific groups for the variables recited herein can be optionally substituted with one or more substituents described herein. Each substituent of a specific group is further optionally substituted with one to three of halo, cyano, oxo, alkoxy, hydroxy, amino, nitro, aryl, cycloaliphatic, heterocycloaliphatic, heteroaryl, haloalkyl, and alkyl. For instance, an alkyl group can be substituted with alkylsulfanyl and the alkylsulfanyl can be optionally substituted with one to three of halo, cyano, oxo, alkoxy, hydroxy, amino, nitro, aryl, haloalkyl, and alkyl. As an additional example, the cycloalkyl portion of a (cycloalkyl)carbonylamino can be optionally substituted with one to three of halo, cyano, alkoxy, hydroxy, nitro, haloalkyl, and alkyl. When two alkoxy groups are bound to the same atom or adjacent atoms, the two alkxoy groups can form a ring together with the atom(s) to which they are bound.

As used herein, the term "substituted," whether preceded by the term "optionally" or not, refers generally to the replacement of hydrogen atoms in a given structure with the radical of a specified substituent. Specific substituents are described above in the definitions and below in the description of compounds and examples thereof. Unless otherwise indicated, an optionally substituted group can have a substituent at each substitutable position of the group, and when more than one position in any given structure can be substituted with more than one substituent selected from a specified group, the substituent can be either the same or different at every position. A ring substituent, such as a heterocycloalkyl, can be bound to another ring, such as a cycloalkyl, to form a spiro-bicyclic ring system, e.g., both rings share one common atom. As one of ordinary skill in the art will recognize, combinations of substituents envisioned by this invention are those combinations that result in the formation of stable or chemically feasible compounds.

As used herein, the phrase "stable or chemically feasible" refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40°C or less, in the absence of moisture or other chemically reactive conditions, for at least a week.

To the extent that any of the compounds described have chiral centres, the present invention extends to all optical isomers of such compounds, whether in the form of racemates or resolved enantiomers. The invention described herein relates to all crystal forms, solvates and hydrates of any of the disclosed compounds however so prepared. To the extent that any of the compounds disclosed herein have acid or basic centres such as carboxylates or amino groups, then all salt forms of said compounds are included herein. The invention described herein further relates to all tautomers of the compounds presented herein. In the case of pharmaceutical uses, the salt should be seen as being a pharmaceutically acceptable salt.

Salts or pharmaceutically acceptable salts that may be mentioned include acid addition salts and base addition salts. Such salts may be formed by conventional means, for example by reaction of a free acid or a free base form of a compound with one or more equivalents of an appropriate acid or base, optionally in a solvent, or in a medium in which the salt is insoluble, followed by removal of said solvent, or said medium, using standard techniques (e.g. in vacuo, by freeze-drying or by filtration). Salts may also be prepared by exchanging a counter-ion of a compound in the form of a salt with another counter-ion, for example using a suitable ion exchange resin.

Examples of pharmaceutically acceptable salts include acid addition salts derived from mineral acids and organic acids, and salts derived from metals such as sodium, magnesium, potassium and calcium.

Examples of acid addition salts include acid addition salts formed with acetic, 2,2-dichloroacetic, adipic, alginic, aryl sulfonic acids (e.g. benzenesulfonic, naphthalene-2-sulfonic, naphthalene-1,5-disulfonic and p-toluenesulfonic), ascorbic (e.g. L-ascorbic), L-aspartic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, gluconic (e.g. D-gluconic), glucuronic (e.g. D-glucuronic), glutamic (e.g. L-glutamic), $\alpha$-oxoglutaric, glycolic, hippuric, hydrobromic, hydrochloric, hydriodic, isethionic, lactic (e.g. (+)-L-lactic and ($\pm$)-DL-lactic), lactobionic, maleic, malic (e.g. (-)-L-malic), malonic, ($\pm$)-DL-mandelic, metaphosphoric, methanesulfonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulfuric, tannic, tartaric (e.g.(+)-L-tartaric), thiocyanic, undecylenic and valeric acids.

Also encompassed are any solvates of the compounds and their salts. Preferred solvates are solvates formed by the incorporation into the solid state structure (e.g. crystal structure) of the compounds of the invention of molecules of a non-toxic pharmaceutically acceptable solvent (referred to below as the solvating solvent). Examples of such solvents include water, alcohols (such as ethanol, isopropanol and butanol) and dimethylsulfoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGA), differential scanning calorimetry (DSC) and X-ray crystallography.

The solvates can be stoichiometric or non-stoichiometric solvates. Particular solvates may be hydrates, and examples of hydrates include hemihydrates, monohydrates and dihydrates. For a more detailed discussion of solvates and the methods used to make and characterise them, see Bryn et al, Solid-State Chemistry of Drugs, Second Edition, published by SSCI, Inc of West Lafayette, IN, USA, 1999, ISBN 0-967-06710-3.

The term "pharmaceutical composition" in the context of this invention means a composition comprising an active agent and comprising additionally one or more pharmaceutically acceptable carriers or pharmaceutically acceptable excipients. The composition may further contain ingredients selected from, for example, diluents, adjuvants, excipients, vehicles, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms. The compositions may take the form, for example, of tablets, dragees, powders, elixirs, syrups, liquid preparations including suspensions, sprays,

inhalants, tablets, lozenges, emulsions, solutions, cachets, granules, capsules and suppositories, as well as liquid preparations for injections, including liposome preparations.

The compounds of the invention may contain one or more isotopic substitutions, and a reference to a particular element includes within its scope all isotopes of the element. For example, a reference to hydrogen includes within its scope $^{1}H$, $^{2}H$ (D), and $^{3}H$ (T). Similarly, references to carbon and oxygen include within their scope respectively $^{12}C$, $^{13}C$ and $^{14}C$ and $^{16}O$ and $^{18}O$. In an analogous manner, a reference to a particular functional group also includes within its scope isotopic variations, unless the context indicates otherwise. For example, a reference to an alkyl group such as an ethyl group or an alkoxy group such as a methoxy group also covers variations in which one or more of the hydrogen atoms in the group is in the form of a deuterium or tritium isotope, e.g. as in an ethyl group in which all five hydrogen atoms are in the deuterium isotopic form (a perdeuteroethyl group) or a methoxy group in which all three hydrogen atoms are in the deuterium isotopic form (a trideuteromethoxy group). The isotopes may be radioactive or non-radioactive.

Therapeutic dosages may be varied depending upon the requirements of the patient, the severity of the condition being treated, and the compound being employed. Determination of the proper dosage for a particular situation is within the skill of the art. Generally, treatment is initiated with the smaller dosages which are less than the optimum dose of the compound. Thereafter the dosage is increased by small increments until the optimum effect under the circumstances is reached. For convenience, the total daily dosage may be divided and administered in portions during the day if desired.

The magnitude of an effective dose of a compound will, of course, vary with the nature of the severity of the condition to be treated and with the particular compound and its route of administration. The selection of appropriate dosages is within the ability of one of ordinary skill in this art, without undue burden. In general, the daily dose range may be from about 10 μg to about 30 mg per kg body weight of a human and non-human animal, preferably from about 50 μg to about 30 mg per kg of body weight of a human and non-human animal, for example from about 50 μg to about 10 mg per kg of body weight of a human and non-human animal, for example from about 100 μg to about 30 mg per kg of body weight of a human and non-human animal, for example from about 100 μg to about 10 mg per kg of body weight of a human and non-human animal and most preferably from about 100 μg to about 1 mg per kg of body weight of a human and non-human animal.

## Combination Therapy

An effective amount can be achieved in the pharmaceutical composition of the invention employing a compound of the invention (including a pharmaceutically acceptable salt or solvate (e.g., hydrate)) alone or in combination with an additional suitable therapeutic agent, for example, an antiviral agent or a vaccine. When "combination therapy" is employed, an effective amount can be achieved using a first amount of a compound of the invention and a second amount of an additional suitable therapeutic agent.

In another embodiment of this invention, a compound of the invention and the additional therapeutic agent, are each administered in an effective amount (i.e., each in an amount which would be therapeutically effective if administered alone). In another embodiment, a compound of the invention and the additional therapeutic agent, are each administered in an amount which alone does not provide a therapeutic effect (a sub-therapeutic dose). In yet another embodiment, a compound of the invention can be administered in an effective amount, while the additional therapeutic agent is administered in a sub-therapeutic dose. In still another embodiment, a compound of the invention can be administered in a sub-therapeutic dose, while the additional therapeutic agent, for example, a suitable cancer-therapeutic agent is administered in an effective amount.

As used herein, the terms "in combination" or "co-administration" can be used interchangeably to refer to the use of more than one therapy (e.g., one or more prophylactic and/or therapeutic agents). The use of the terms does not restrict the order in which therapies (e.g., prophylactic and/or therapeutic agents) are administered to a subject.

Co-administration encompasses administration of the first and second amounts of the compounds of the co-administration in an essentially simultaneous manner, such as in a single pharmaceutical composition, for example, capsule or tablet having a fixed ratio of first and second amounts, or in multiple, separate capsules or tablets for each. In addition, such co-administration also encompasses use of each compound in a sequential manner in either order.

In one embodiment, a compound of the invention and an additional therapeutic agent are administered separately, sequentially or simultaneously to the subject.

When co-administration involves the separate administration of the first amount of a compound of the invention and a second amount of an additional therapeutic agent, the compounds are administered sufficiently close in time to have the desired therapeutic effect. For example, the period of time between each administration which can result in the desired therapeutic effect, can range from minutes to hours and can be determined taking into account the properties of each compound such as potency, solubility, bioavailability, plasma half-life and kinetic profile. For example, a compound of the invention and the second therapeutic agent can be administered in any order within 24 hours of each other, within 16 hours of each other, within 8 hours of each other, within 4 hours of each other, within 1 hour of each other or within 30 minutes of each other.

More, specifically, a first therapy (e.g., a prophylactic or therapeutic agent such as a compound of the invention) can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (e.g., a prophylactic or therapeutic agent such as an anti-cancer agent) to a subject.

It is understood that the method of co-administration of a first amount of a compound of the invention and a second amount of an additional therapeutic agent can result in an enhanced or synergistic therapeutic effect, wherein the combined effect is greater than the additive effect that would result from separate administration of the first amount of a compound of the invention and the second amount of an additional therapeutic agent.

As used herein, the term "synergistic" refers to a combination of a compound of the invention and another therapy (e.g., a prophylactic or therapeutic agent), which is more effective than the additive effects of the therapies. A synergistic effect of a combination of therapies (e.g., a combination of prophylactic or therapeutic agents) can permit the use of lower dosages of one or more of the therapies and/or less frequent administration of said therapies to a subject. The ability to utilize lower dosages of a therapy (e.g., a prophylactic or therapeutic agent) and/or to administer said therapy less frequently can reduce the toxicity associated with the administration of said therapy to a subject without reducing the efficacy of said therapy in the prevention, management or treatment of a disorder. In addition, a synergistic effect can result in improved efficacy of agents in the prevention, management or treatment of a disorder. Finally, a synergistic effect of a combination of therapies (e.g., a combination of prophylactic or therapeutic agents) may avoid or reduce adverse or unwanted side effects associated with the use of either therapy alone.

The presence of a synergistic effect can be determined using suitable methods for assessing drug interaction. Suitable methods include, for example, the Sigmoid-Emax equation (Holford, N.H.G. and Scheiner, L.B., Clin. Pharmacokinet. 6: 429-453 (1981)), the equation of Loewe additivity (Loewe, S. and Muischnek, H., Arc h. Exp. Pathol Pharmacol. 114: 313-326 (1926)) and the median-effect equation (Chou, T.C. and Talalay, P., Adv. Enzyme Regul. 22: 27-55 (1984)). Each equation referred to above can be applied with experimental data to generate a corresponding graph to aid in assessing the effects of the drug combination. The corresponding graphs associated with the equations referred to above are the concentration-effect curve, isobologram curve and combination index curve, respectively.

In one aspect the invention provides a pharmaceutical composition comprising a compound, pharmaceutically acceptable salt, solvate, hydrate, tautomer or optical isomer of the invention, a pharmaceutically acceptable excipient and at least one additional therapeutic agent. In some embodiments the compound, pharmaceutically acceptable salt, solvate, hydrate, tautomer or optical isomer of the invention and the at least one additional therapeutic agent are co-formulated. In some embodiments the compound, pharmaceutically acceptable salt, solvate, hydrate, tautomer or optical isomer of the invention and the at least one additional therapeutic agent are formulated separately.

In another aspect the invention provides a kit comprising a compound, pharmaceutically acceptable salt, solvate, hydrate, tautomer or optical isomer of the invention and at least one additional therapeutic agent. The kit may comprise instructions to administer the compound, pharmaceutically acceptable salt, solvate, hydrate, tautomer or optical isomer of the invention and the at least one additional therapeutic agent to a subject in need thereof.

In another aspect the invention provides a combination therapy for use as a medicament, wherein the combination therapy comprises administering a compound, pharmaceutically acceptable salt, solvate, hydrate, tautomer or optical isomer of the invention to a subject in need thereof and administering an additional therapeutic agent to the subject in need thereof.

The compound, pharmaceutically acceptable salt, solvate, hydrate, tautomer or optical isomer of the invention and the additional therapeutic agent may be administered to the subject separately, sequentially or simultaneously.

The pharmaceutical composition, kit and/or combination therapy may be for use in the treatment of a gastrointestinal disorder or a pulmonary disease or condition. The gastrointestinal disorder may be selected from the group consisting of constipation disorders, constipation-predominant irritable bowel syndrome, mixed type irritable bowel syndrome, chronic idiopathic constipation, gastrointestinal symptoms associated with Parkinson’s disease, gastrointestinal symptoms associated with cystic fibrosis, intestinal dysmotility, postoperative ileus, food allergy or food intolerance, celiac disease, gastrointestinal motility disorders, functional gastrointestinal disorders, drug induced enteropathy, NSAID induced gastric and intestinal injury, chemotherapy induced mucositis, gastroesophageal reflux disease (GERD), duodenogastric reflux, diarrhoeal diseases, immune mediated gastrointestinal diseases, Crohn’s disease, ulcerative colitis, inflammatory bowel disease, and ischemic colitis. The pulmonary disease or condition may be selected from the group consisting of chronic obstructive pulmonary diseases, asthma, chronic bronchitis, cystic fibrosis, emphysema, chronic idiopathic cough, hyperactive airway disorder, and idiopathic pulmonary fibrosis.

The at least one additional therapeutic agent may be selected from the group consisting of aminosalicylates, corticosteroids, immunomodulators and combinations thereof. Aminosalicylates are also known as 5-aminosalicylates (5-ASAs). The at least one additional therapeutic agent may be an aminosalicylate. The aminosalicylate may be mesalamine.

The aminosalicylate may be sulfasalazine. The at least one additional therapeutic agent may be a corticosteroid. The corticosteroid may be budesonide. The at least one additional therapeutic agent may be an immunomodulator. The immunomodulator may be thiopurine. The immunomodulator may be methotrexate.

## Pharmaceutical formulations

While it is possible for the active compound to be administered alone, it is preferable to present it as a pharmaceutical composition (e.g. formulation).

Accordingly, in another embodiment of the invention, there is provided a pharmaceutical composition comprising at least one compound of Formula (I) as defined above together with at least one pharmaceutically acceptable excipient.

When the pharmaceutical composition comprises at least one additional therapeutic agent, the compound, pharmaceutically acceptable salt, solvate, hydrate, tautomer or optical isomer of the invention and the at least one additional therapeutic agent may be co-formulated or the compound, pharmaceutically acceptable salt, solvate, hydrate, tautomer or optical isomer of the invention and the at least one additional therapeutic agent may be formulated separately.

The composition may be a tablet composition.

The composition may be a capsule composition.

The pharmaceutically acceptable excipient(s) can be selected from, for example, carriers (e.g. a solid, liquid or semi-solid carrier), adjuvants, diluents (e.g solid diluents such as fillers or bulking agents; and liquid diluents such as solvents and co-solvents), granulating agents, binders, flow aids, coating agents, release-controlling agents (e.g. release retarding or delaying polymers or waxes), binding agents, disintegrants, buffering agents, lubricants, preservatives, anti-fungal and antibacterial agents, antioxidants, buffering agents, tonicity-adjusting agents, thickening agents, flavouring agents, sweeteners, pigments, plasticizers, taste masking agents, stabilisers or any other excipients conventionally used in pharmaceutical compositions.

The term "pharmaceutically acceptable" as used herein means compounds, materials, compositions, and/or dosage forms which are, within the scope of sound medical judgment, suitable for use in contact with the tissues of a subject (e.g. a human subject) without excessive toxicity, irritation, allergic response, or other problem or complication, commensurate with a reasonable benefit/risk ratio. Each excipient must also be "acceptable" in the sense of being compatible with the other ingredients of the formulation.

Pharmaceutical compositions containing compounds of the Formula (I) can be formulated in accordance with known techniques, see for example, Remington's Pharmaceutical Sciences, Mack Publishing Company, Easton, PA, USA.

The pharmaceutical compositions can be in any form suitable for oral, parenteral, topical, intranasal, intrabronchial, sublingual, ophthalmic, otic, rectal, intra-vaginal, or transdermal administration.

Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches.

Tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starc h. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Tablets may be designed to release the drug either upon contact with stomach fluids (immediate release tablets) or to release in a controlled manner (controlled release tablets) over a prolonged period of time or with a specific region of the GI tract.

The pharmaceutical compositions typically comprise from approximately 1% (w/w) to approximately 95%, preferably % (w/w) active ingredient and from 99% (w/w) to 5% (w/w) of a pharmaceutically acceptable excipient (for example as defined above) or combination of such excipients. Preferably, the compositions comprise from approximately 20% (w/w) to approximately 90% (w/w) active ingredient and from 80% (w/w) to 10% of a pharmaceutically excipient or combination of excipients. The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, pre-filled syringes, dragées, powders, tablets or capsules.

Tablets and capsules may contain, for example, 0-20% disintegrants, 0-5% lubricants, 0-5% flow aids and/or 0-99% (w/w) fillers/ or bulking agents (depending on drug dose). They may also contain 0-10% (w/w) polymer binders,

0-5% (w/w) antioxidants, 0-5% (w/w) pigments. Slow release tablets would in addition typically contain 0-99% (w/w) release-controlling (e.g. delayin g) polymers (depending on dose). The film coats of the tablet or capsule typically contain 0-10% (w/w) polymers, 0-3% (w/w) pigments, and/or 0-2% (w/w) plasticizers.

Parenteral formulations typically contain 0-20% (w/w) buffers, 0-50% (w/w) cosolvents, and/or 0-99% (w/w) water for Injection (WFI) (depending on dose and if freeze dried). Formulations for intramuscular depots may also contain 0-99% (w/w) oils.

The pharmaceutical formulations may be presented to a patient in "patient packs" containing an entire course of treatment in a single package, usually a blister pack.

The compounds of the Formula (I) will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular sub-ranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

For oral compositions, a unit dosage form may contain from 1 milligram to 2 grams, more typically 10 milligrams to 1 gram, for example 50 milligrams to 1 gram, e.g. 100 milligrams to 1 gram, of active compound.

The active compound will be administered to a patient in need thereof (for example a human or animal patient) in an amount sufficient to achieve the desired therapeutic effect (effective amount). The precise amounts of compound administered may be determined by a supervising physician in accordance with standard procedures.

## Synthesis of Compounds of the Formula (1)

### LC/MS Method 1

Instruments: Acquity UPLC with Photodiode Array Detector and QDA mass detector; Column: Acquity C-18, 1.6 micron, 50 x 2.1 mm; Gradient [time (min)/solvent B in A (%)]: 0.00/10, 0.75/10, 2.80/90, 4.50/100, 4.60/100, 4.70/10; Solvents: solvent A = 0.1% formic acid in water; solvent B = 0.1% formic acid in water / acetonitrile (10: 90); column temperature 35 °C; Flow rate 0.8 mL/min.

### LC/MS Method 2

Instruments: Acquity UPLC with Photodiode Array Detector and QDA mass detector; Column: Acquity C-18, 1.6 micron, 50 x 1.6 mm; Gradient [time (min)/solvent B in A (%)]: 0.00/3, 0.20/3, 2.70/98. 3.00/100, 3.50/100, 3.51/3, 4.00/3; Solvents: solvent A = 0.1% formic acid in water; solvent B = 0.1% formic acid in water / acetonitrile (10:90); column temperature 35 °C; Flow rate 0.9 mL/min.

### LC/MS Method 3

Instruments: Water 2690 with Photodiode Array Detector and QDA mass detector; Column: X-Bridge C-18, 5 micron, 100 x 1.6 mm; Gradient [time (min)/solvent B in A (%)]: 0.01/10, 1.00/10, 5.00/100, 7.00/100, 7.50/10, 8.00/10; Solvents: solvent A = 0.1% formic acid and 10mM ammonium carbonate in water; solvent B = acetonitrile; column temperature 35 °C; Flow rate 0.9 mL/min.

### General Synthetic Strategies

Compounds of formula G-4 can be synthesized according to Scheme 1 by first 1) alkylating tert-butyl carbazate with a bromide compound of formula G-1a, or 2) contacting *tert*-butyl carbazate with an aldehyde of formula G-1b under reductive amination conditions to provide the hydrazine compound G-2. Contacting G-2 with the bivalent compound, 3-bromopropionyl chloride provides the cyclized and boc-protected compound of formula G-3. Deprotection of G-4 provides a compound of formula G-4.

Scheme 1

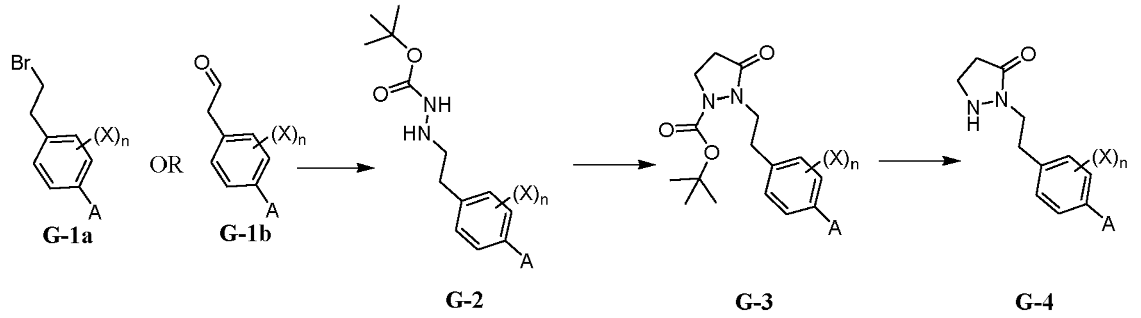

G-1a OR G-1b G-2 G-3 G-4

Compounds of formula G-4 can be alkylated according to Scheme 2, via a conjugate addition reaction with a compound of the formula G-5 to provide a compound of formula G-6. G-6 can then be coupled with an aryl bromide of formula G-7, wherein $R^x$ and $R^{x'}$ are each independently a lower alkyl group, or both $R^x$ and $R^{x'}$ together with the boron atom and oxygen atoms to which they are attached, form a 5 or 6 membered heterocyclic ring. The coupling of G-6 to G-7 typically takes place in the presence of a palladium catalyst, such as $PdCl_2$(dppf).DCM, to provide a biaryl compound of formula G-8. G-8 can be further reduced to provide a compound of G-10. Other functional group interconversions are possible at this point in the synthesis, such as when A is an ester group, the compound of G-8 or G-10 can be further hydrolyzed. Also, reduction (and/or other FGI, e.g. hydrolysis) can be performed prior to Pd catalyzed coupling according to Scheme 2, via a compound of formula G-9.

Scheme 2

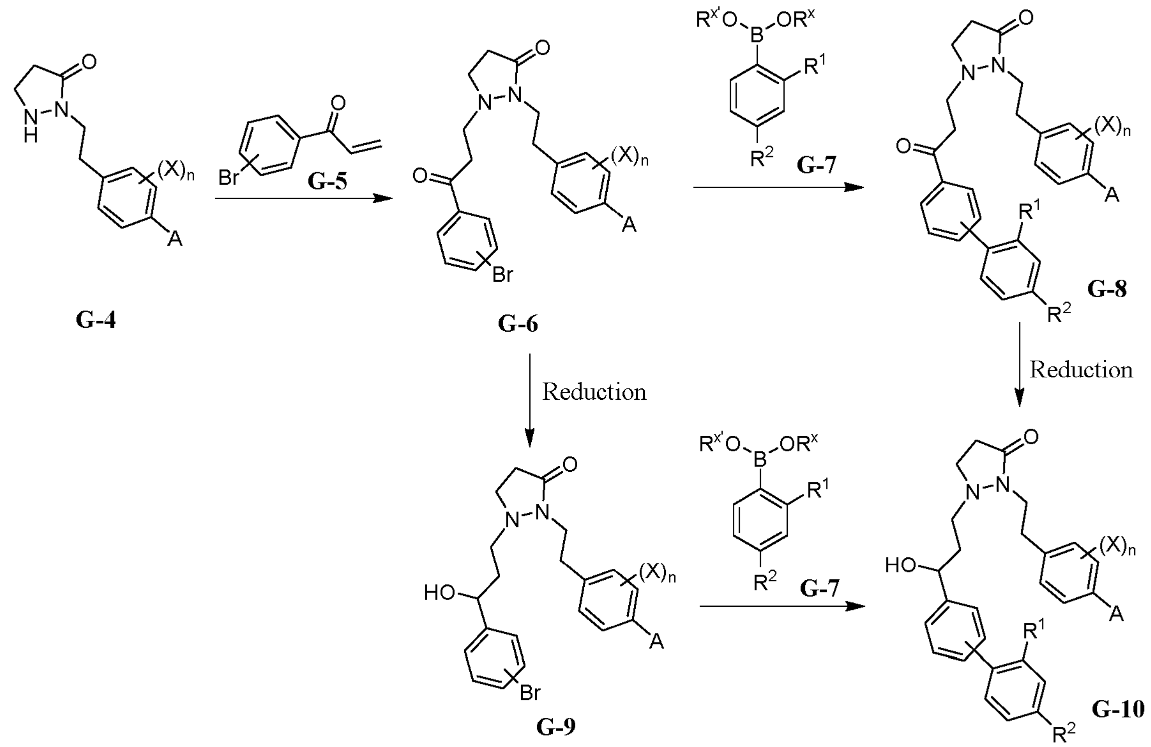


G-4 G-5 G-6 G-7 G-8 G-9 G-7 G-10

Phenol compounds of formula G-11 can be further derivatized to compounds of formula G-12 according to Scheme 3, by reaction with a sulfonamidating reagent such as sulfamoyl chloride.

Scheme 3

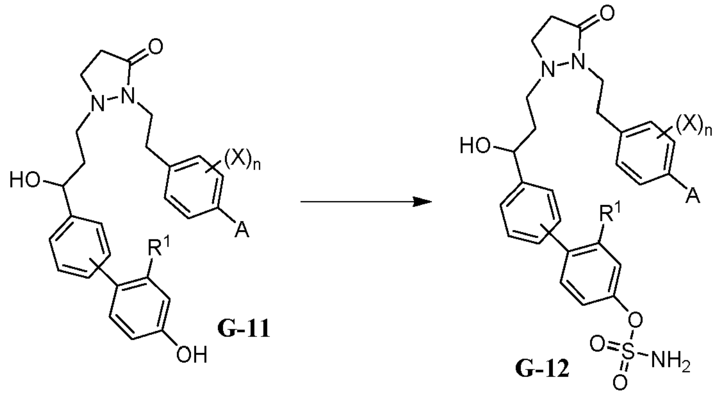

G-11

G-12

EXAMPLES

Additional embodiments are disclosed in further detail in the following examples. In the exemplified chiral separations of racemic compounds in the following examples, "isomer 1" refers to the isomer that eluted first from the chiral column and "isomer 2" refers to the isomer that eluted second from the chiral column.

**Synthetic Preparation of the Intermediates**

**Intermediate 1: Synthesis of methyl 4-(2-(5-oxopyrazolidin-1-yl)ethyl)benzoate (Intermediate 1)**

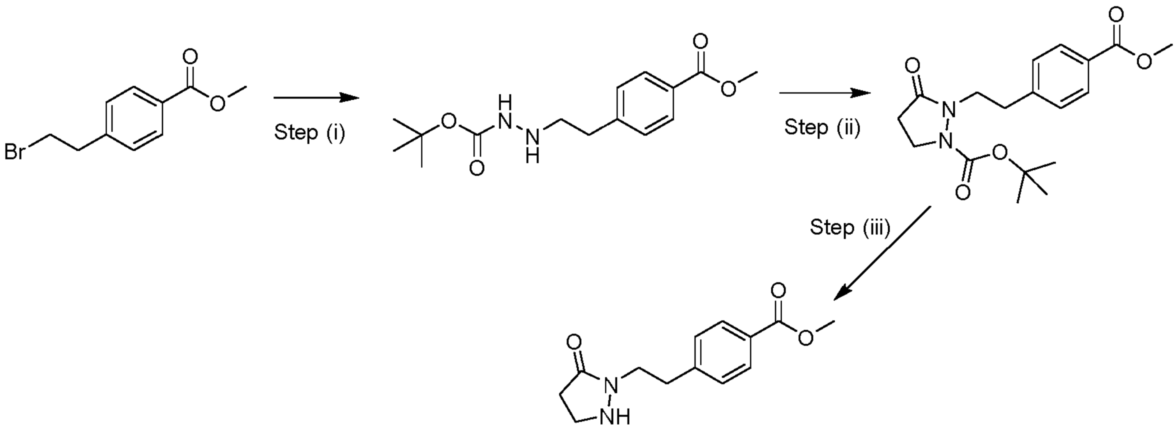


Intermediate1

**Step (i):** Methyl 4-(2-bromoethyl)benzoate (20.00 g, 82.65 mmol), tert-Butyl carbazate (12.01 g, 90.92 mmol), $NaHCO_3$ (27.78 g, 330.60 mmol) and NaI (1.24 g, 8.27 mmol) were suspended in MeCN (200 mL) at room temperature and the reaction mixture was allowed to stir at 80°C for 24 h. The reaction mixture was concentrated under vacuo and the residue was partitioned between water (1000 mL) and EtOAc (800 mL), aqueous layer was further extracted with EtOAc (3 x 300 mL). The organic layers were combined and dried ($Na_2SO_4$). Solvent was removed in vacuo and the crude product was purified by gradient column chromatography (normal phase, silica), product was eluted at 0% to 18% EtOAc in hexane to afford impure product which was further purified by gradient reverse phase flash column chromatography (reverse phase, C18 silica), product eluted at 0% to 55% MeCN in water to afford tert-butyl 2-(4-(methoxycarbonyl)phenethyl) hydrazine-1-carboxylate (5.6g, 23.%) as yellow oil. Product was confirmed by LCMS (Method_3), m/z 239 (ES+, M+H-tBu), at 2.20 min.

**Step (ii):** tert-butyl 2-(4-(methoxycarbonyl)phenethyl)hydrazine-1-carboxylate (5.60 g, 19.04 mmol) was dissolved in MeCN (60 mL) and potassium carbonate (13.16 g, 95.19 mmol) was added to the reaction mixture at room temperature and the reaction mixture was allowed to stir at room temperature for 10 min. After this, 3-bromopropionyl chloride (2.90 mL, 28.56 mmol) was added drop wise at room temperature and reaction mixture was allowed to stir at room temperature for 24 h. The reaction mixture was concentrated under vacuo and the obtained residue was partitioned

between water (800 mL) and EtOAc (500 mL), aqueous layer was further extracted with EtOAc (2 x 200 mL). Organic layers were combined and dried ($Na_2SO_4$). Solvent was removed in vacuo and the product was purified by gradient column chromatography (normal phase, silica), product was eluted at 0% to 22% EtOAc in hexane to afford impure product which was further purified by gradient reverse phase flash column chromatography (reverse phase, C18 silica), product elute at 0% to 45% MeCN in water to afford tert-butyl 2-(4-(methoxycarbonyl)phenethyl)-3-oxopyrazolidine-1-carboxylate (6.5 g, 98%) as yellow oil. Product was confirmed by LCMS (Method_3), m/z 293. (ES+, M+HtBu), at 2.43 min.

**Step (iii):** tert-butyl 2-(4-(methoxycarbonyl)phenethyl)-3-oxopyrazolidine-1-carboxylate (6.5 g, 18.7 mmol) was dissolved in dioxane (70 mL) and 4 N HCl in dioxane (70 mL) was added drop wise to the reaction mixture at 0°C and the reaction mixture was allowed to stir at room temperature for 4 h. Solvent was removed in vacuo to obtain crude product which was purified by trituration with 20% MeOH in diethyl ether to afford methyl 4-(2-(5-oxopyrazolidin-1-yl)ethyl) benzoate (4.8 g, 90.%) as an off-white powder. Product was confirmed by LCMS (Method_3), m/z 249 (ES+, M+H), at 1.57 min. $^1H$ NMR: (400MHz, DMSO) δ: 2.62-2.58 (t, 2H, J=8.2Hz), 3.06-3.02 (t, 2H, J=7.4Hz), 3.58-3.56 (t, 2H, J=4.4Hz), 3.74-3.70 (t, 2H, J=7.2Hz), 7.46-7.44 (d, 2H, J=8.4Hz), 7.90-7.89 (d, 2H, J=8.0Hz).

**Intermediate 2: Synthesis of methyl 4-(2-(2-(3-(3-bromophenyl)-3-hydroxypropyl)-5-oxopyrazolidin-1-yl) ethyl)-2,6-difluorobenzoate (Intermediate 2)**

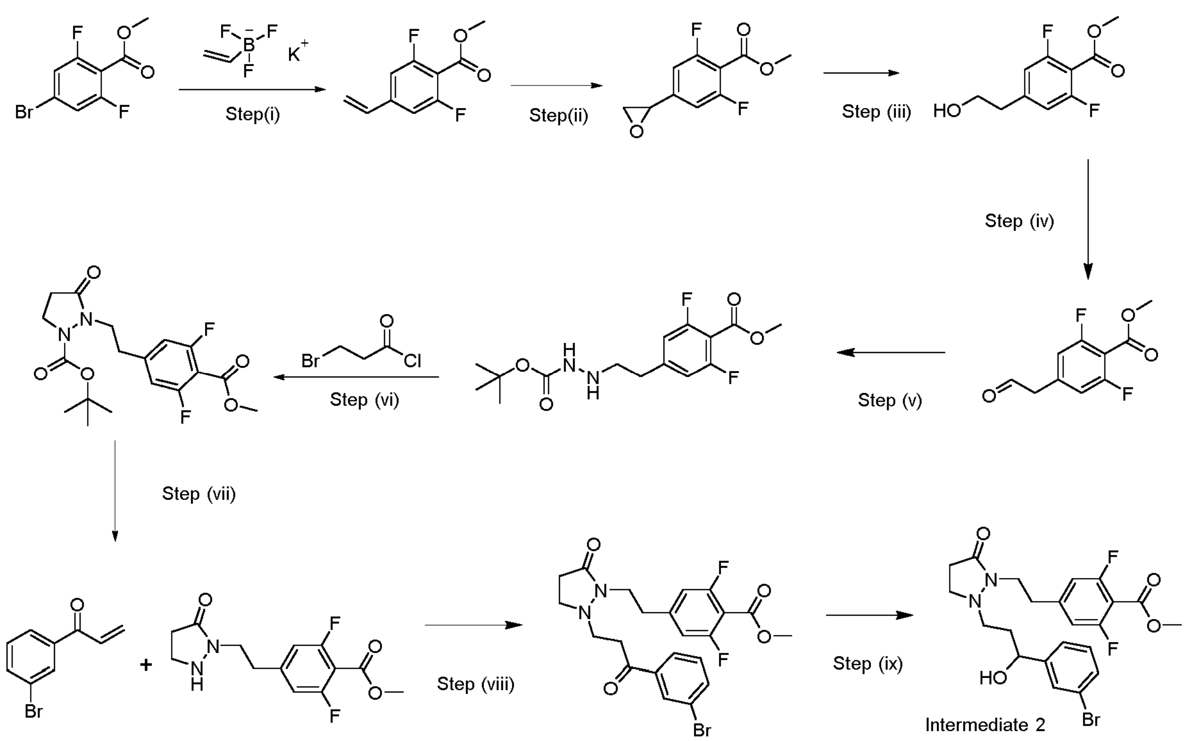


**Step (i):** methyl 4-bromo-2,6-difluorobenzoate (40.00 g, 159.36 mmol), potassium vinyltrifluoroborate (32.00 g, 239.04 mmol) and TEA (42 mL, 318.72 mmol) were dissolved in IPA (400 mL) and nitrogen gas was purged for 30 min at room temperature. After this, $PdCl_2$(dppf).DCM (13.01 g, 15.93 mmol) was added and the reaction mixture was allowed to stir at 80°C for 3 h. The reaction mixture was then partitioned between water (1000 mL) and EtOAc (1000 mL). Aqueous layer was further extracted with EtOAc (2 x 500 mL). Organic layers were combined and dried ($Na_2SO_4$). Solvent was removed in vacuo and the crude product was purified by gradient flash column chromatography (normal phase, Silica) product eluted at 0% to 5% EtOAc in hexane to afford methyl 2,6-difluoro-4-vinylbenzoate (25.0g, 79%) as yellow oil.

**Step (ii)** methyl 2,6-difluoro-4-vinylbenzoate (25.0 g, 126.19 mmol) was dissolved in mixture of *t*-Butanol (130 mL) and water (175 mL) at room temperature. After this, NBS (26.94 g, 151.43 mmol) was added portion wise at room temperature and allowed to stir at 40°C temperature for 16 h. After cooling to 5°C, a NaOH Solution (10.09 g, 252.23 mmol) and allowed to stir at room temperature for 30 min. The reaction mixture was then partitioned between water (800 mL) and EtOAc (500 mL), aqueous layer was further extracted with EtOAc (2 x 300 mL). Organic layers were combined and dried ($Na_2SO_4$). Solvent was removed in vacuo to afford crude methyl 2,6-difluoro-4-(oxiran-2-yl)benzoate (22.8 g, 84%) as yellow oil.

**Step (iii):** methyl 2,6-difluoro-4-(oxiran-2-yl)benzoate (22.8 g, 106.54 mmol) was dissolved in methanol (500 mL)

and 10% palladium on carbon with 50% moisture (6.9 g) was added. After this, ammonium formate (67.22 g, 1065.42 mmol) was added at room temperature and reaction mixture was allowed to stir for 16 h at room temperature. After completion, the reaction mixture was filtered through celite bed, washed with MeOH (3000 mL) and filtrate was concentrated in vacuo. The reaction mixture was then partitioned between water (1000 mL) and EtOAc (500 mL). Aqueous layer was further extracted with EtOAc (2 x 300 mL). Organic layers were combined and dried ($Na_2SO_4$). Solvent was removed in vacuo to afford crude product which was purified by gradient flash column chromatography (normal phase, Silica) product eluted at 0% to 26% EtOAc in hexane to afford methyl 2,6-difluoro-4-(2-hydroxyethyl)benzoate (11 g, 47.80%) as yellow oil.

**Step (iv):** methyl 2,6-difluoro-4-(2-hydroxyethyl)benzoate (11.00 g, 50.87 mmol) was dissolved in DCM (300 mL) at room temperature. After this, Dess-Martin periodinane (32.36 g, 76.31 mmol) was added portion wise at room temperature and reaction mixture was allowed to stir at room temperature for 2 h. The reaction mixture was then partitioned between saturated aqueous $NaHCO_3$ solution (500 mL) and DCM (800 mL). Aqueous layer was further extracted with EtOAc (2 x 500 mL). Organic layers were combined and dried ($Na_2SO_4$). Solvent was removed in vacuo to afford crude methyl 2,6-difluoro-4-(2-oxoethyl)benzoate (7.96 g, 73%) as white solid. Used in the next step without further purification.

**Step (v):** methyl 2,6-difluoro-4-(2-oxoethyl)benzoate (7.92 g, 37.00 mmol) and *tert*-butyl carbazate (4.89 g, 37.00 mmol) were dissolved in methanol (100 mL) under nitrogen atmosphere and 4 Å molecular sieves was also added to maintain moisture free condition. After this, Glacial acetic acid (0.3 mL, 3.70 mmol) was added at room temperature and allowed to stir for 3 h at room temperature. After this, reaction mixture was cooled at 0°C and sodium cyanoborohydride (2.78 g, 44.40 mmol) was added portion wise and allowed to stir at room temperature for 12 h. The reaction mixture was concentrated under vacuo to obtain residue. It was partitioned between saturated aqueous $NaHCO_3$ solution (500 mL) and EtOAc (300 mL), aqueous layer was further extracted with EtOAc (2 x 200 mL). Organic layers were combined and dried ($Na_2SO_4$). Solvent was removed in vacuo to obtain crude product which was purified by gradient column chromatography (normal phase, silica) product eluted at 0% to 3% EtOAc in DCM to afford crude *tert*-butyl 2-(3,5-difluoro-4-(methoxycarbonyl)phenethyl)hydrazine-1-carboxylate (9.10 g, 65%) as white solid. Product was confirmed by LCMS (Method 2), m/z 275 (ES+, M+H-tBu), at 2.32 min.

**Step (vi):** *tert*-butyl 2-(3,5-difluoro-4-(methoxycarbonyl)phenethyl)hydrazine-1-carboxylate (9.00 g, 27.26 mmol) was dissolved in DMF (20 mL) and potassium carbonate (18.83 g, 136.30 mmol) was added to the reaction mixture at room temperature and allowed to stir at room temperature for 10 min. After this, 4-bromobutanoyl chloride (3.4 mL, 40.89 mmol) was added drop wise at room temperature and reaction mixture was allowed to stir at room temperature for 3 h and then at 40°C for 16 h. The reaction mixture was partitioned between water (500 mL) and EtOAc (700 mL), aqueous layer was further extracted with EtOAc (2 x 250 mL). Organic layers were combined and dried ($Na_2SO_4$). Solvent was removed in vacuo and the product was purified by gradient column chromatography (reverse phase, C18 silica), product was eluted at 0% to 50% MeCN In water to afford (3.5 g, 33%) as yellow oil. Product was confirmed by LCMS (Method 2) m/z 329 (ES+, M+H-tBu), at 2.48 min.

**Step (vii):** *tert*-butyl 2-(3,5-difluoro-4-(methoxy carbonyl) phenethyl) -3-oxopyrazolidine-1-carboxylate (3.5 g, 9.11 mmol) was dissolved in dioxane (40 mL) and 4 N HCl in dioxane (20 mL) was added drop wise to the reaction mixture at $0^0$ C. Reaction mixture was allowed to stir at room temperature for 18 h. Solvent was removed in vacuo to obtain crude product which was purified by trituration with 20% MeOH in diethyl ether to afford methyl 2,6-difluoro-4-(2-(5-oxopyrazolidin-1-yl)ethyl)benzoate_HCl_salt) (2.62 g, 79%) as off-white amorphous powder. Product was confirmed by LCMS (Method 2), m/z 285 (ES+, M+H), at 1.67 minmin.

**Step (vii):** *tert*-butyl 2-(3,5-difluoro-4-(methoxy carbonyl) phenethyl) -3-oxopyrazolidine-1-carboxylate (3.5 g, 9.11 mmol) was dissolved in dioxane (40 mL) and 4 N HCl in dioxane (20 mL) was added drop wise to the reaction mixture at 0° C. Reaction mixture was allowed to stir at room temperature for 18 h. Solvent was removed in vacuo to obtain crude product which was purified by trituration with 20% MeOH in diethyl ether to afford methyl 2,6-difluoro-4-(2-(5-oxopyrazolidin-1-yl)ethyl) benzoate_HCl_salt) (2.62 g, 79%) as off-white amorphous powder. Product was confirmed by LCMS (Method 2), m/z 285 (ES+, M+H), at 1.67 min.

**Step (viii):** methyl 2,6-difluoro-4-(2-(5-oxopyrazolidin-1-yl)ethyl)benzoate_HCl_salt) (0.80 g, 2.81 mmol) and 1-(3-bromophenyl)prop-2-en-1-one (1.60 g, 14.08 mmol) were suspended in MeOH (10 mL) at room temperature and reaction mixture was allowed to stir at room temperature for 15 min. After this, TEA (0.70 mL, 5.27 mmol) was added at room temperature and allowed to stir at 60°C for 4 h. The reaction mixture was partitioned between water (200 mL) and EtOAc (200 mL) and aqueous layer was further extracted with EtOAc (2 x 100 mL). Organic layers were combined and dried ($Na_2SO_4$). Solvent was removed in vacuo and the crude product was further purified by gradient reverse phase flash column chromatography (reverse phase, C18 silica), product eluted at 0% to 58% ACN in water to afford methyl 4-(2-(2-(3-(3-bromophenyl)-3-oxopropyl)-5-oxopyrazolidin-1-yl)ethyl)- 2,6-difluorobenzoate (0.65 g, 47%) as colorless sticky material. Product was confirmed by LCMS (Method 2), m/z 495 (ES+, M+H), at 2.58 min.

**Step (ix):** methyl 4-(2-(2-(3-(3-bromophenyl)-3-oxopropyl)-5-oxopyrazolidin-1-yl)ethyl)-2,6-difluorobenzoate (0.650 g, 1.31 mmol) was dissolved in ethanol (4 mL) and water (2 mL), and $CeCl_3$ (0.97 g, 3.94 mmol) was added

and reaction mixture was allowed to stir at 0°C for 5 min. Sodium borohydride (0.20 g, 0.5.26 mmol) was then added at 0°C and reaction mixture was stirred at room temperature for 1 h. The reaction mixture was then partitioned between water (100 mL) and EtOAc (150 mL) and the aqueous layer was further extracted with EtOAc (2 x 50 mL). Organic layers were combined and dried ($Na_2SO_4$). Solvent was removed in vacuo to afford **Intermediate 2:** methyl 4-(2-(2-(3-(3-bromophenyl)-3-hydroxypropyl)-5-oxopyrazolidin-1-yl)ethyl)-2,6-difluorobenzoate (0.620 g, 95.%) as brown solid. Product was confirmed by LCMS (Method 2), m/z 497 (ES+, M+H), at 2.04 min. **$^1$H NMR:** (400Mz, DMSO) $\delta$: 1.74-1.73 (d, 2H, J= 4.8Hz), 2.88-2.76 (m, 4H), 3.12-3.10 (d, 2H, J= 7.2Hz), 3.86 (s, 3H), 4.66 (s, 1H), 5.44-5.43 (d, 1H, J= 4.4Hz), 7.12-7.10 (d, 2H, J= 9.6Hz), 7.36-7.27 (m, 2H), 7.43-7.41 (d, 1H, J= 8.0Hz), 7.55 (s. 1H).

**Intermediate B: Synthesis of 1-(3-bromophenyl)prop-2-en-1-one (Intermediate B)**

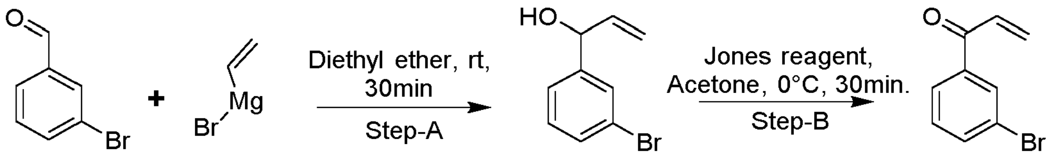


**(A)** **(B)**

**Step (i)**: 3-bromobenzaldehyde (12.0g, 56.6mmole) was dissolved in diethyl ether (30mL) at 0°C, vinylmagnesium bromide (1M in THF) (200mL) was added drop wise at 0°C under nitrogen atmosphere and allowed to stir at room temperature for 3h. The reaction mixture was partitioned between saturated aqueous $NH_4Cl$ (1000mL) and EtOAc (800mL); the aqueous layer was further extracted with EtOAc (2 x 300mL). Organic layers were combined and dried ($Na_2SO_4$). Solvent was removed in vacuo and the crude product was purified by gradient column chromatography (normal phase, Silica) product eluted at 0% to 14% EtOAc in hexane to afford 1-(3-bromophenyl)prop-2-en-1-ol (A) (12.0g, 94%) as a colourless oil..

**Step (ii):** [1-(3-bromophenyl)prop-2-en-1-ol A) (12.0g, 57.1mmole) was dissolved in acetone (30mL) at room temperature. To it, Jones reagent (39mL) was added dropwise at -25°C and allowed to stir at -25°C for 30 min. Then reaction mixture was warmed to 0°C and stirred for 30min. The reaction mixture was partitioned between saturated aqueous $NaHCO_3$ (100mL) and EtOAc (800mL) and aqueous layer was further extracted with EtOAc (2 x 250mL). Organic layers were combined and dried ($Na_2SO_4$). Solvent was removed in vacuo and the crude product was purified by gradient column chromatography (normal phase, Silica) product eluted at 0% to 8% EtOAc in hexane to afford 1-(3-bromophenyl)prop-2-en-1-one (8.0g, 97%) as yellow gum. Product was confirmed by LCMS no mass ion 2.39 min.

**Intermediate 3: Synthesis of methyl 4-(2-(2-(3-(3-bromophenyl)-3-hydroxypropyl)-5-oxopyrazolidin-1-yl) ethyl)-2-hydroxybenzoate (Intermediate 3)**

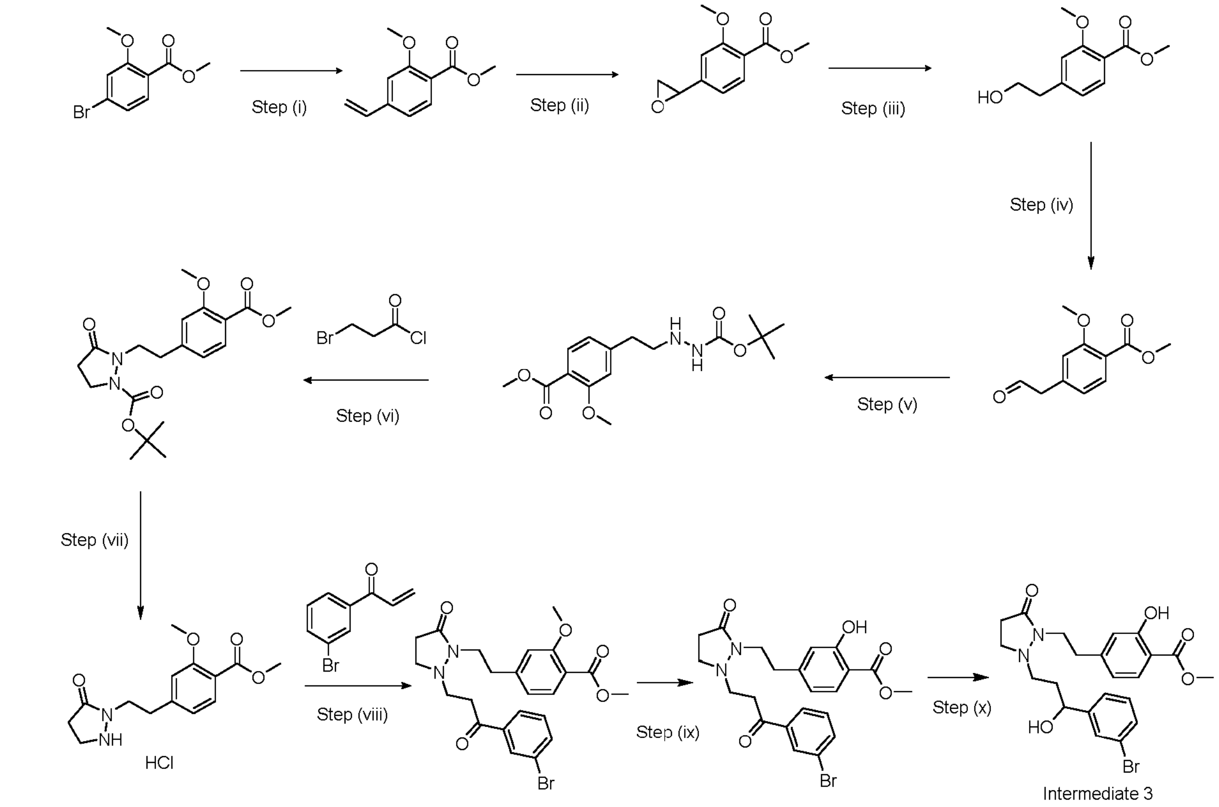


**Step (i):** methyl 4-bromo-2-methoxybenzoate (30.00 g, 156.07 mmol), potassium vinyltrifluoroborate (41.81 g, 312.16 mmol) and TEA (64.93 mL, 468.21 mmol) were dissolved in Isopropyl alcohol (300 mL) and nitrogen gas was purged for 30 min at room temperature. After this, $PdCl_2$(dppf)DCM (20.09 g, 24.60 mmol) was added and the reaction mixture was allowed to stir at 80°C for 3 h. The reaction mixture was then partitioned between water (1200 mL) and EtOAc (700 mL). Aqueous layer was further extracted with EtOAc (2 x 500 mL). Organic layers were combined and dried ($Na_2SO_4$). Solvent was removed in *vacuo* and the crude product was purified by gradient flash column chromatography (normal phase, Silica) product eluted at 0% to 14% EtOAc in hexane to afford methyl 2-methoxy-4-vinylbenzoate (20.00 g, 84%) as yellow oil. Product was confirmed by LCMS (Method 1), m/z 193 (ES+, M+H) at 2.18 min.

**Step (ii):** methyl 2-methoxy-4-vinylbenzoate (20.00 g, 104.05 mmol) was dissolved in mixture of t-Butanol (15 mL) and water (30 mL) at room temperature. After this, NBS (27.78 g, 156.08 mmol) was added portion wise at room temperature and reaction mixture was allowed to stir at 40°C temperature for 2 h. The reaction mixture was then partitioned between water (1000 mL) and EtOAc (800 mL). Aqueous layer was further extracted with EtOAc (2 x 300 mL). Organic layers were combined and dried ($Na_2SO_4$). Solvent was removed in *vacuo* to afford crude methyl 2-methoxy-4-(oxiran-2-yl) benzoate (20.00 g, Quantitative yield) as yellow oil. **Note:** As this product was not stable, carried forward to next step without purification.

**Step (iii):** methyl 2-methoxy-4-(oxiran-2-yl)benzoate (20.00 g, 96.06 mmol) was dissolved in methanol (200 mL) and 10% palladium on carbon with 50% moisture (10.00 g) was added. After this, ammonium formate (60.57 g, 960.60 mmol) was added at room temperature and reaction mixture was allowed to stir at 60°C temperature for 4 h. After completion, the reaction mixture was filtered through celite bed and washed with MeOH (2000 mL). Filtrate was concentrated in *vacuo.* Crude product was purified by gradient flash column chromatography (normal phase, Silica), product eluted at 0% to 45% EtOAc in hexane to afford crude methyl 4-(2-hydroxyethyl)-2-methoxybenzoate (9.80 g, 49%) as colorless oil. Product was confirmed by LCMS (Method 1), m/z 211 (ES+), at 1.60 min.

**Step (iv):** methyl 4-(2-hydroxyethyl)-2-methoxybenzoate (9.80 g, 46.62 mmol) was dissolved in DCM (100 mL) at room temperature. After this, Dess-Martin periodinane (39.54 g, 93.24 mmol) was added portion wise at room temperature and reaction mixture was allowed to stir at room temperature for 4 h. The reaction mixture was then partitioned between saturated aqueous $NaHCO_3$ solution (800 mL) and EtOAc (500 mL). Aqueous layer was further extracted with EtOAc (2 x 200 mL). Organic layers were combined and dried ($Na_2SO_4$). Solvent was removed in *vacuo* to afford methyl 2-methoxy-4-(2-oxoethyl)benzoate (10.0 g, Quantitative yield) as yellow oil, which was used in the next step without further purification.

**Step (v):** methyl 2-methoxy-4-(2-oxoethyl)benzoate (10.0 g, 48.03 mmol) and *tert-butyl* carbazate (7.62 g, 57.63 mmol) were dissolved in methanol (100 mL) under nitrogen atmosphere. 4 Å molecular sieves (1.00 g) was also added to

maintain moisture free condition. After this, glacial acetic acid (0.3 mL, 4.80 mmol) was added at room temperature and the reaction mixture was allowed to stir for 3 h at room temperature. After this, reaction mixture was cooled at 0°C and Sodium cyanoborohydride (4.53 g, 72.05 mmol) was added portion wise and reaction mixture was allowed to stir at room temperature for 16 h. The reaction mixture was filtered under reduced pressure and filtrate was concentrated in *vacuo.* Obtained residue was partitioned between saturated aqueous $NaHCO_3$ solution (700 mL) and EtOAc (500 mL). Aqueous layer was further extracted with EtOAc (3 x 200 mL). Organic layers were combined and dried ($Na_2SO_4$). Solvent was removed in *vacuo* to obtain crude product which was purified by gradient column chromatography (normal phase, silica), product was eluted at 0% to 29% EtOAc in Hexane to afford crude *tert*-butyl 2-(3-methoxy-4-(methoxycarbonyl)phenethyl) hydrazine-1-carboxylate (7.0 g, 45%) as yellow sticky material. Product was confirmed by LCMS (Method 2), m/z 347 (ES+, M+Na), at 2.02.

**Step (vi):** *tert*-butyl 2-(4-(methoxycarbonyl)phenethyl)hydrazine-1-carboxylate (7.00 g, 21.59 mmol) was dissolved in MeCN (70 mL) and potassium carbonate (14.92 g, 107.93 mmol) was added to the reaction mixture at room temperature. Reaction mixture was allowed to stir at room temperature for 10 min. After this, 3-Bromopropionyl chloride (3.26 mL, 32.39 mmol) was added drop wise at room temperature and reaction mixture was allowed to stir at room temperature for 24 h. After this, reaction mixture was stirred at 40°C for 4 h. The reaction mixture was concentrated under *vacuo* and the obtained residue was partitioned between water (700 mL) and EtOAc (500 mL). Aqueous layer was further extracted with EtOAc (2 x 200 mL). Organic layers were combined and dried ($Na_2SO_4$). Solvent was removed in *vacuo* and the product was purified by gradient column chromatography (normal phase, silica), product was eluted at 0% to 32% EtOAc in hexane to afford tert-butyl 2-(3-methoxy-4-(methoxycarbonyl)phenethyl)-3-oxopyrazolidine-1-carboxylate (2.0 g, 24%) as yellow oil. Product was confirmed by LCMS (Method 2), m/z 323 (ES+, M+H-tBu), at 2.27 min.

**Step (vii):** *tert*-butyl 2-(3-methoxy-4-(methoxycarbonyl)phenethyl)-3-oxopyrazolidine-1-carboxylate (2.00 g, 5.29 mmol) was dissolved in 1,4-Dioxane (20 mL) and 4 N HCl in dioxane (20 mL) was added drop wise to the reaction mixture at 0°C and the reaction mixture was allowed to stir at room temperature for 8 h. Solvent was removed in *vacuo* to obtain crude product which was purified by triturating with 20% MeOH in diethyl ether to afford crude methyl 2-methoxy-4-(2-(5-oxopyrazolidin-1-yl)ethyl)benzoate HCl salt (1.50 g, 90.36%) as off white amorphous powder. Product was confirmed by LCMS, m/z 279.10 (ES+), at 1.415 min.

**Step (viii):** methyl 2-methoxy-4-(2-(5-oxopyrazolidin-1-yl)ethyl)benzoate hydrochloride (1.00 g, 3.18 mmol), 1-(3-bromophenyl)prop-2-en-1-one (3.33 g, 15.88 mmol) and TEA (2.2 mL, 15.88 mmol) were suspended in MeOH (10 mL) at room temperature and reaction mixture was allowed to stir at 60°C for 4 h. The reaction mixture was partitioned between saturated aqueous NH4Cl solution (300 mL) and EtOAc (200 mL). Aqueous layer was further extracted with EtOAc (2 x 100 mL). Organic layers were combined and dried ($Na_2SO_4$). Solvent was removed in *vacuo* and the crude product was further purified by reverse phase gradient flash column chromatography (reverse phase, C18 silica), product eluted at 0% to 65% MeCN in water to afford methyl 4-(2-(2-(3-(3-bromophenyl)-3-oxopropyl)-5-oxopyrazolidin-1-yl) ethyl)-2-methoxybenzoate (1.20 g, 69%) as colorless sticky material. Product was confirmed by LCMS (Method 2), m/z 489 (ES+, M+H), at 2.38 min.

**Step (ix):** methyl (R)-4-((1-(3-bromobenzyl)pyrrolidine-2-carboxamido)methyl)-2-methoxybenzoate (0.25 g, 0.51 mmol) was dissolved in DCM (3 mL) and reaction mixture was cooled to -78°C. After this, $BBr_3$ (1 M solution in DCM) (2.5 mL, 2.56 mmol) was added and the reaction mixture was allowed to stir at -78°C for 1 h. Reaction mixture then partitioned between saturated aqueous solution of $NaHCO_3$ (100 mL) and DCM (70 mL). Aqueous layer was again extracted with EtOAc (2x40 mL). Organic layers were combined and dried ($Na_2SO_4$). Solvent was removed in *vacuo* to afford methyl 4-(2-(2-(3-(3-bromophenyl)-3-oxopropyl)-5-oxopyrazolidin-1-yl)ethyl)-2-hydroxybenzoate, (0.18 g, 74%) as yellow sticky material. Productwas confirmed by LCMS (Method 2) m/z 475 (ES+, M+H) at 2.61 min.

**Step (x):** methyl 4-(2-(2-(3-(3-bromophenyl)-3-oxopropyl)-5-oxopyrazolidin-1-yl)ethyl)-2-hydroxybenzoate (0.16 g, 0.34 mmol) was dissolved in ethanol (2 mL) and water (2 mL). To it, $CeCl_3$ (0.25 g, 1.01 mmol) was added and reaction mixture was allowed to stir at $0^0$C for 5 min. After this, $NaBH_4$ (0.085 g, 1.36 mmol) was added at 0°C and reaction mixture was stirred at room temperature for 1 h. The reaction mixture was partitioned between saturated aqueous $NaHCO_3$ solution (30 mL) and EtOAc (30 mL). Aqueous layer was further extracted with EtOAc (2 x 20 mL). Organic layers were combined and dried ($Na_2SO_4$). Solvent was removed in vacuo to afford **Intermediate 3** methyl 4-(2-(2-(3-(3-bromophenyl)-3-hydroxypropyl)-5-oxopyrazolidin-1-yl)ethyl)-2-hydroxybenzoate (0.15 g, 93%) as an off-white solid. Product was confirmed by LCMS (Method 2), m/z 478 (ES+, M+H) at 2.46 min.

**Synthetic Preparation of Compounds of Formula I**

**Example 1: Synthesis of 4-(2-(2-(3-hydroxy-3-(4'-hydroxy-2'-methyl-[1,1'-biphenyl]-3-yl)propyl)-5-oxopyrazolidin-1-yl)ethyl)benzoic acid (Compound 1)**

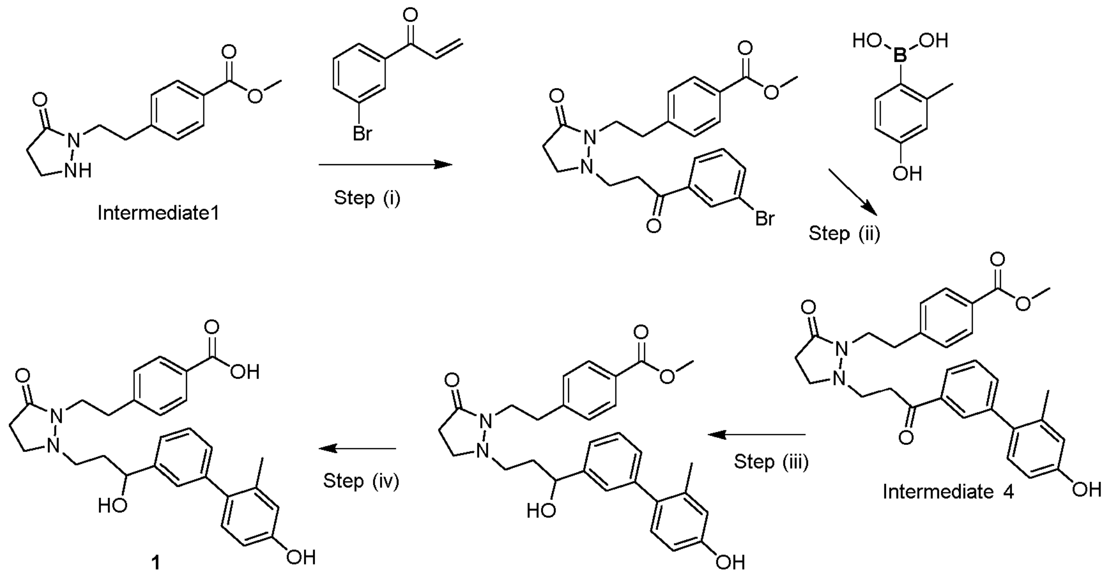

1

**Step (i): Intermediate 1** methyl 4-(2-(5-oxopyrazolidin-1-yl)ethyl)benzoate hydrochloride (2.00 g, 7.26 mmol) and 1-(3-bromophenyl)prop-2-en-1-one (1.50 g, 14.52 mmol) were suspended in IPA (20 mL) at room temperature and reaction mixture was allowed to stir at room temperature for 15 min. After this, TEA (2.5 mL, 36.30 mmol) was added at room temperature and allowed to stir at 80°C for 12 h. The reaction mixture was partitioned between saturated aqueous $NH_4Cl$ solution (500 mL) and EtOAc (300 mL) and aqueous layer was further extracted with EtOAc (2 x 100 mL). Organic layers were combined and dried ($Na_2SO_4$). Solvent was removed in vacuo and the crude product was further purified by gradient reverse phase flash column chromatography (reverse phase, C18 silica), product eluted at 0% to 75% MeCN in water to afford methyl 4-(2-(2-(3-(3-bromophenyl)-3-oxopropyl)-5-oxopyrazolidin-1-yl) ethyl) benzoate (0.80 g, 24%) as colorless sticky material. Product was confirmed by LCMS (Method 3), m/z 459 (ES+, M+H), at 2.48 min.

**Step (ii):** methyl 4-(2-(2-(3-(3-bromophenyl)-3-oxopropyl)-5-oxopyrazolidin-1-yl)ethyl)benzoate (0.25 g, 0.55 mmol), 4-hydroxy-2-methylphenyl)boronic acid (0.99 g, 0.65 mmol) and potassium carbonate (0.23 g, 1.65 mmol) were suspended in dioxane (3 mL) and water (2 mL) at room temperature and reaction mixture was degassed with nitrogen at room temperature for 15 min. After this, $PdCl_2$(dppf).DCM (0.04 g, 0.05 mmol) was added at room temperature and reaction mixture was allowed to stir at 100°C for 4 h. The reaction mixture was partitioned between water (200 mL) and EtOAc (100 mL), aqueous layer was further extracted with EtOAc (2 x 50 mL). The organic layers were combined and dried ($Na_2SO_4$). Solvent was removed in vacuo and the crude product was purified by gradient column chromatography (normal phase, Silica) product eluted at 0% to 80% EtOAc in hexane to afford methyl 4-(2-(2-(3-(4'-hydroxy-2'-methyl-[1,1'-biphenyl]-3-yl)-3-oxopropyl)-5-oxopyrazolidin-1-yl)ethyl) benzoate **Intermediate 4** (0.21 g, 81%) as off-white solid. Product was confirmed by LCMS (Method 3), m/z 509 (ES+, M+Na), at 2.33 min.

**Step (iii):** methyl 4-(2-(2-(3-(3-bromophenyl)-3-oxopropyl)-5-oxopyrazolidin-1-yl)ethyl)benzoate (0.21 g, 0.43 mmol) and cerium(iii) chloride (0.32 g, 1.30 mmol) were dissolved in ethanol (3 mL) and water (3 mL) at room temperature and the reaction mixture was allowed to stir at room temperature for 5 min. After this, sodium borohydride (0.65 g, 1.73 mmol) was added at 0°C and allowed the reaction mixture to stir at room temperature for 2 h. The reaction mixture was partitioned between saturated aqueous $NaHCO_3$ solution (100 mL) and EtOAc (50 mL) and aqueous layer was further extracted with EtOAc (2 x 30 mL). Organic layers were combined and dried ($Na_2SO_4$). Solvent was removed in vacuo to afford methyl 4-(2-(2-(3-hydroxy-3-(4'-hydroxy-2'-methyl-[1,1'-biphenyl]-3-yl)propyl)-5-oxopyrazolidin-1-yl)ethyl)benzoate (0.16 g, 76%) as off white solid. Product was confirmed by LCMS (Method 3), m/z 489 (ES+, M+H), at 2.19 min.

**Step (iv):** methyl 4-(2-(2-(3-hydroxy-3-(4'-hydroxy-2'-methyl-[1,1'-biphenyl]-3-yl)propyl)-5-oxopyrazolidin-1-yl) ethyl)benzoate (0.16 g, 0.33 mmol) was dissolved in Dioxane (2 mL) and water (2 mL). To it, LiOH monohydrate (0.073 g, 1.75 mmol) was added at room temperature and allowed to stir at room temperature for 3 h. Reaction mixture was then partitioned between water (80 mL) and EtOAc (2 x 50 mL). Aqueous layer was further acidified with 1 N aqueous HCl (~20 mL), adjusted pH up to ~3 and extracted with EtOAc (2 x 70 mL). Organic layers were combined and dried ($Na_2SO_4$). Solvent was removed in vacuo to afford 4-(2-(2-(3-hydroxy-3-(4'-hydroxy-2'-methyl-[1,1'-biphenyl]-3-yl)propyl)-5-oxopyrazolidin-1-yl)ethyl)benzoic acid) (0.125 g, 81%) as a brown solid. Product was confirmed by LCMS (Method 3), m/z 475 (ES+, M+H), at 1.97 min. **$^1$H NMR:** (400MHz, DMSO) δ: 1.26-1.23 (m, 3H), 1.82-1.80 (d, 2H, J=6.4Hz), 2.16 (s, 3H), 2.88-2.85 (t, 3H, J=6.0Hz), 3.17 (s, 3H), 4.08 (s, 1H), 4.71-4.69 (d, 1H, J=6.0Hz), 6.87-6.83 (m, 2H), 7.01-6.99 (d, 1H, J=8.4Hz), 7.16-7.14 (d, 1H, J=7.6Hz), 7.37-7.26 (m, 5H), 7.85-7.83 (d, 2H, 8.4Hz), 9.43 (s, 2H).

**Compounds 1R and 1S:** Racemic Compound 1 (0.12 g) was enantiomerically separated with a Shimadzu Prep-HPLC system (PHENOMNENEX AMYLOSE-250*21.2 mm, 5 μm column) using a mobile phase that is 43.0% 10 mM DEA

in heptane and 57.0% IPA, with no gradient and a flow rate of 16.00 mL/min. Isomer 1: (0.004 g, 2.6%; off-white solid) Product was confirmed by LCMS (Method 3), m/z 475 (ES+, M+H), at 2.30 min. **$^1$H NMR:** (400MHz, MeOD) δ: 2.03-1.93 (m, 2H), 2.93-2.90 (t, 4H, J=6.8Hz), 3.07-3.03 (t, 3H, J=7.2Hz), 3.25 (s, 2H), 3.37-3.32 (m, 1H), 3.59-3.56 (m, 1H), 3.71-3.69 (m, 2H), 4.87-4.84 (t, 1H, J=6.0Hz), 6.68-6.66 (m, 1H), 6.73-6.72 (d, 1H, J=2.0z), 7.02-7.00 (d, 1H, J=8.0Hz), 7.25-7.19 (m, 3H), 7.41-7.32 (m, 5H), 7.91-7.89 (d, 2H, J=8.0Hz). Isomer 2: Product was confirmed by LCMS (Method 3), m/z 475 (ES+, M+H), at 2.30 min. **$^1$H NMR:** (400MHz, MeOD) δ: 1.97-1.94 (t, 2H, J=6.4Hz), 2.93-2.90 (t, 4H, J=6.8Hz), 3.07-3.03(m, 3H), 3.26 (s, 2H), 3.59-3.57 (t, 2H J=4.6Hz), 3.71-3.69 (t, 2H J=4.6Hz), 4.87-4.84 (t, 1H, J=6.4Hz), 6.68-6.65 (dd, 1H J=2.4Hz & J=4.0Hz), 6.72-6.72 (d, 1H, J=2.0Hz), 7.02-7.00 (d, 1H, J=8.0Hz), 7.24-7.19 (m, 3H), 7.41-7.32 (m, 5H), 7.91-7.89 (d, 2H, J=8.0Hz).

**Example 2: Synthesis of 4-(2-(2-(3-(4'-carbamoyl-2'-methyl-[1,1'-biphenyl]-3-yl)-3-hydroxypropyl)-5-oxopyrazolidin-1-yl)ethyl)benzoic acid (Compound 2)**

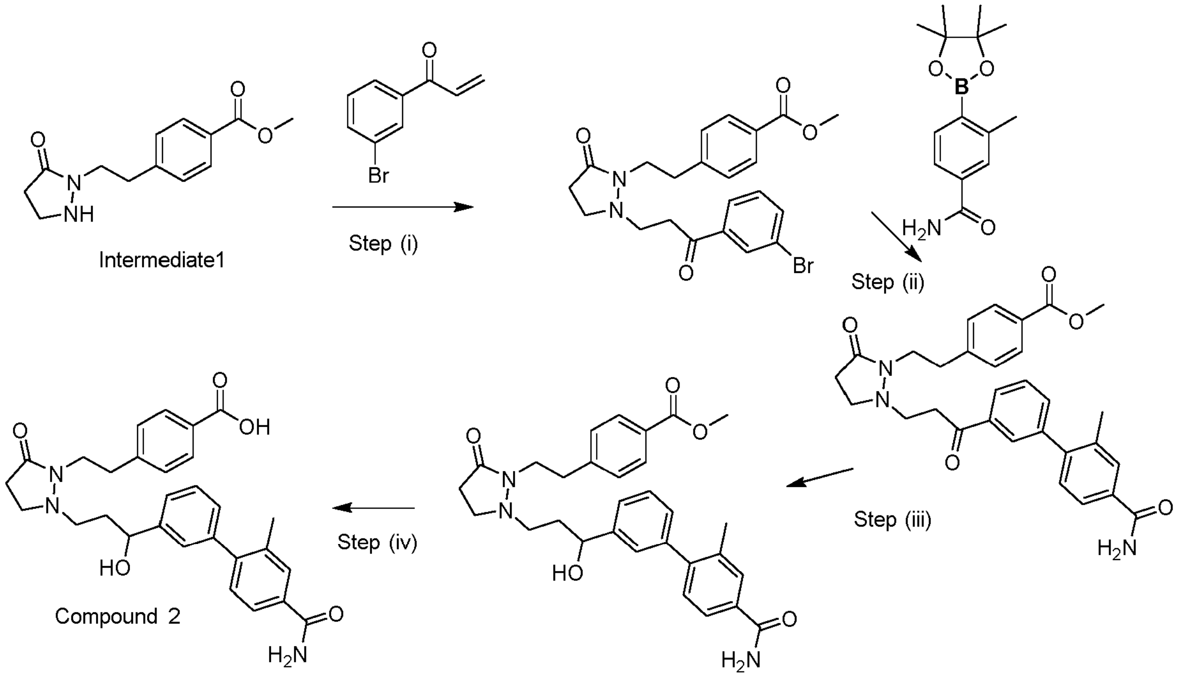


**Step** (i): Intermediate 1, methyl 4-(2-(5-oxopyrazolidin-1-yl)ethyl)benzoate hydrochloride (2.00 g, 7.26 mmol) and 1-(3-bromophenyl)prop-2-en-1-one (1.50 g, 14.52 mmol) were suspended in IPA (20 mL) at room temperature and reaction mixture was allowed to stir at room temperature for 15 min. After this, TEA (2.5 mL, 36.30 mmol) was added at room temperature and allowed to stir at 80°C for 12 h. The reaction mixture was partitioned between saturated aqueous $NH_4Cl$ solution (500 mL) and EtOAc (300 mL) and aqueous layer was further extracted with EtOAc (2 x 100 mL). Organic layers were combined and dried ($Na_2SO_4$). Solvent was removed in vacuo and the crude product was further purified by gradient reverse phase flash column chromatography (reverse phase, C18 silica), product eluted at 0% to 75% MeCN in water to afford methyl 4-(2-(2-(3-(3-bromophenyl)-3-oxopropyl)-5-oxopyrazolidin-1-yl) ethyl) benzoate (0.80 g, 24%) as colorless sticky material. Product was confirmed by LCMS (Method 3), m/z 459 (ES+, M+H), at 2.48 min.

**Step (ii):** Methyl 4-(2-(2-(3-(3-bromophenyl)-3-oxopropyl)-5-oxopyrazolidin-1-yl)ethyl)benzoate (0.60g, 1.30mmole), 3-methyl-4-(4,4,5,5-tetramethyl-1,3,2-dioxaborolan-2-yl) benzamide (1.36g, 5.23mmole) and $K_2CO_3$ (0.54g, 3.92mmole) were dissolved in mixture of dioxane : water (3:2, 5 mL) and nitrogen gas was purged for 20min at room temperature. After this, $PdCl_2$(dppf)DCM (0.21g, 0.26mmole) was added and the reaction mixture was allowed to stir at 80°C for 8h. The reaction mixture was then partitioned between water (100mL) and EtOAc (100mL), aqueous layer was further extracted with EtOAc (2 x 70mL). Organic layers were combined and dried ($Na_2SO_4$). Solvent was removed in vacuo and the crude product was purified by gradient flash column chromatography (reverse phase, C18 silica), product eluted at 0% to 38% MeCN in water to give methyl 4-(2-(2-(3-(4'-carbamoyl-2'-methyl-[1,1'-biphenyl]-3-yl)-3-oxopropyl)-5-oxopyrazolidin-1-yl)ethyl)benzoate (0.60g, 90%) as white solid. LCMS: (Method 3): Product was confirmed m/z 536 (ES+, M+Na), at 2.16min.

**Step(iii):** methyl 4-(2-(2-(3-(4'-carbamoyl-2'-methyl-[1,1'-biphenyl]-3-yl)-3-oxopropyl)-5-oxopyrazolidin-1-yl)ethyl)benzoate (0.60g, 1.16mmole) was dissolved in ethanol (3mL) and water (2mL) and $CeCl_3$(0.86g, 3.50mmole) was added to the reaction mixture. Reaction mixture was allowed to stir at 0°C for 5min. After this, sodium borohydride (0.17g, 4.67mmole) was added at 0°C and reaction mixture was stirred at room temperature for 2h. The reaction mixture was partitioned between saturated aqueous $NaHCO_3$ solution (100mL) and EtOAc (80mL) and aqueous layer was further extracted with EtOAc (2 x 50mL). The organic layers were combined and dried ($Na_2SO_4$). Solvent was removed in vacuo and the crude product was purified by gradient flash column chromatography (reverse phase, C18 silica), product elute at 0% to 35% MeCN in water to afford pure methyl 4-(2-(2-(3-(4'-carbamoyl-2'-methyl-[1,1'-biphenyl]-3-yl)-3-hydroxypropyl)-5-oxopyrazolidin-1-yl)ethyl)benzoate (0.50g, 83%) as off white solid. LCMS: (LCMS Method 3): Product was confirmed m/z 516 (ES+, M+H), at 2.78min.

**Step (iv):** methyl 4-(2-(2-(3-(4'-carbamoyl-2'-methyl-[1,1'-biphenyl]-3-yl)-3-hydroxypropyl)-5-oxopyrazolidin-1-yl)ethyl)benzoate (1.80g, 3.49mmole) was dissolved in dioxane (15mL) and water (8mL). LiOH monohydrate (0.73g, 17.46mmole) was added at room temperature and reaction mixture was allowed to stir at room temperature for 3h. Reaction mixture was then partitioned between water (250mL) and EtOAc (2 x 350mL). Aqueous layer further acidified with 4N aqueous HCl (60mL) to adjust the pH up to ~1 and extracted with EtOAc (2 x 300mL). Organic layers were combined and dried ($Na_2SO_4$). Solvent was removed in vacuo to afford pure 4-(2-(2-(3-(4'-carbamoyl-2'-methyl-[1,1'-biphenyl]-3-yl)-3-hydroxypropyl)-5-oxopyrazolidin-1-yl)ethyl)benzoic acid (1.47g, 83%) as an off-white solid.

LCMS (Method 2): Product was confirmed m/z 502 (ES+, M+H), at 1.53 min. **$^1$H NMR:** (400MHz, DMSO) δ: 1.76-1.84 (m, 2H,), 2.26 (s, 3H), 2.70-2.90 (m, 4H), 3.18 (s, 3H), 4.72 (brs, 1H), 5.37 (d, 1H, J=4.0Hz), 7.21-7.45 (m, 8H), 7.74 (dd, 1H, J= 7.9 and 1.9Hz), 7.80-7.86 (m, 3H), 7.98 (s, 1H), 12.86 (brs, 1H).**Compounds 2R and 2S:** Racemic compound 2 was enatiomericaly separated on a Waters Prep-HPLC system using following method: Column ADH-9.5*250mm 5micron, Solvents A: 0.1% TFA in N-heptane, B: IPA: methanol (70:30) isocratic 75%A/25%B with variable flow rate (time/flow, 0.01/4.0, 5.00/4.0, 10.00/8.0, 100.00/8.0, min/ml per min)

**Isomer 1** (0.489g, 28%) as off-white solid. LCMS: (Method 2): Product was confirmed m/z 502 (ES+, M+H), at 1.56 min. 1H NMR: (400MHz, DMSO) : δ 1.77-1.86 (m, 2H), 2.27 (s, 3H), 2.74-2.90 (m, 4H), 3.14 (brs, 2H), 4.73 (t, 1H, J=6.4Hz), 7.21-7.47 (m, 8H), 7.75 (dd, 1H, J= 7.9Hz, 1.9Hz), 7.80-7.86 (m, 3H), 7.98 (s, 1H), 12.86 (brs, 1H). (Note: Some of the aliphatic protons obscured by DMSO-and/or water)

**Isomer 2 (0.477g, 27%)** as off-white solid. LCMS: (Method 2): Product was confirmed m/z 502 (ES+, M+H), at 1.56 min. 1H NMR: (400MHz, DMSO) δ 1.77-1.86 (m, 2H), 2.27 (s, 3H), 2.76-2.90 (m, 4H), 3.14 (brs, 2H), 4.69-4.76 (m, 1H), 5.37 (brs, 1H), 7.22-7.45 (m, 8H), 7.75 (dd, 1H, J= 7.9Hz, 1.9Hz), 7.81-7.86 (m, 3H), 7.97 (s, 1H), 12.84 (brs, 1H). (Note: Some of the aliphatic protons obscured by DMSO-and/or water)

**Example 3: Synthesis of 4-(2-(2-(3-hydroxy-3-(2'-methyl-4'-sulfamoyl-[1,1'-biphenyl]-3-yl)propyl)-5-oxopyrazolidin-1-yl)ethyl)benzoic acid (Compound 3)**

was synthesized by the method described in Example 1 using 3-Methyl-4-(4,4,5,5-tetramethyl-1,3,2-dioxaborolan-2-yl)benzenesulfonamide, which was synthesized from 3-methyl-4-bromobenzsulfonamide, $PdCl_2$(dppf).DCM, and KOAc, in dioxane (80°C; 2 h). and reverse phase flash chromatography at the final step to give 4-(2-(2-(3-hydroxy-3-(2'-methyl-4'-sulfamoyl-[1,1'-biphenyl]-3-yl)propyl)-5-oxopyrazolidin-1-yl)ethyl)benzoic acid; (0.027 g, 28%) as an off white solid. Product was confirmed by LCMS (Method 3), m/z 538 (ES+, M+H), at 1.90 min. **$^1$H NMR:** (400MHz, DMSO) δ: 1.10-1.07 (t, 1H, J=7Hz), 1.82-1.80 (d, 2H, 5.6Hz), 2.08 (s, 1H), 2.29 (s, 4H), 2.87-2.85 (d, 4H, 6.4Hz), 3.13 (s, 3H), 4.73 (s, 1H) 5.39-5.38 (d, 1H, 4.4Hz), 7.35-7.24 (m, 3H), 7.46-7.37 (m, 6H), 7.71-7.68 (m, 1H), 7.75 (s, 1H), 7.84-7.82 (d, 2H, J=8.4Hz), 12.85 (s, 1H).

**Compounds 3R and 3S:** Racemic Compound 3 was enantiomerically separated with a Waters Prep-HPLC system (CROMEGACHIRAL CCO 250*20 mm, 5 μm column) using a mobile phase that is 75% 0.1% FA in heptane and 25% a mixture of IPA and methanol (50:50) as the mobile phase, with a flow rate of 18.00 mL/min and no gradient. Isomer 1: (0.019 g, 6.5%; off-white solid) Product was confirmed by LCMS (Method 3), m/z 538 (ES+, M+H), at 1.90 min. **$^1$H NMR:** (400MHz, DMSO) δ: 1.23 (s, 2H), 1.82-1.81 (d, 2H, J=6.4Hz), 2.33-2.29 (m, 4H), 2.83 (s, 4H), 3.16 (s, 5H), 4.74 (s, 1H), 5.40 (s, 1H), 7.25-7.23 (m, 3H), 7.44-7.34 (m, 6H),7.71-7.68 (m, 1H), 7.75-7.75 (d, 1H, J=1.6Hz), 7.82-7.8- (d, 2H, J=8Hz). Isomer 2: (0.017 g, 5.8%; off-white solid). Product was confirmed by LCMS (Method 3), m/z 538 (ES+,M+H), at 1.93 min. **$^1$H NMR:** (400MHz, DMSO) δ: 1.23-1.16 (d, 3H, J= 28.0Hz), 1.82 (s, 2H), 2.29 (s, 4H), 2.82 (s, 4H), 3.14 (s, 2H), 4.74 (s, 1H), 5.39 (s, 1H) 7.24-7.21 (d, 3H, J= 12Hz), 7.42-7.35 (q, 5H), 7.80-7.69 (m, 4H).

**Example 4: Synthesis of 4-(2-(2-(3-(4'-hydroxy-2'-methyl-[1,1'-biphenyl]-3-yl)-3-oxopropyl)-5-oxopyrazolidin-1-yl)ethyl)benzoate (Compound 4)**

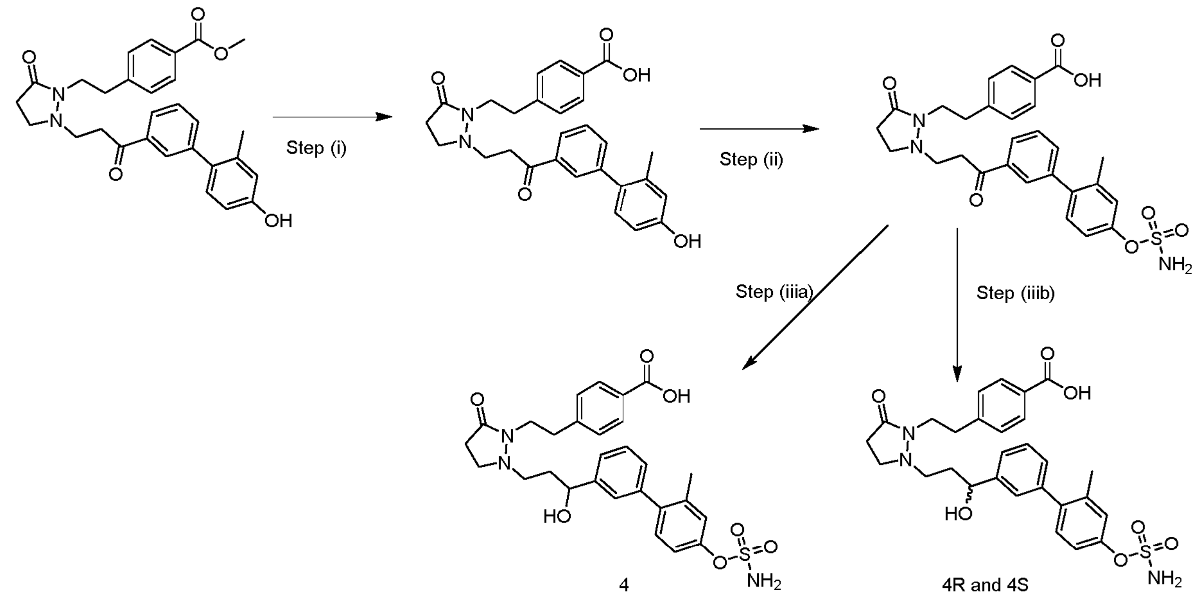


**Step (i): Intermediate 4** from Example 1 (methyl 4-(2-(2-(3-(4'-hydroxy-2'-methyl-[1,1'-biphenyl]-3-yl)-3-oxopropyl)-5-oxopyrazolidin-1-yl)ethyl)benzoate, 0.17 g, 0.34 mmol) was dissolved in Dioxane (3 mL) and water (2 mL). LiOH monohydrate (0.073 g, 1.74 mmol) was added at room temperature and allowed to stir at room temperature for 3 h. The reaction mixture was then partitioned between water (80 mL) and EtOAc (2 x 50 mL). Aqueous layer was further acidified with 4 N aqueous HCl (3 mL) to adjust pH up to ~1 and extracted with EtOAc (2 x 50 mL). Organic layers were combined and dried ($Na_2SO_4$). Solvent was removed in vacuo to afford pure 4-(2-(2-(3-(4'-hydroxy-2'-methyl-[1,1'-biphenyl]-3-yl)-3-oxopropyl)-5-oxopyrazolidin-1-yl)ethyl)benzoic acid (0.13 g, 79%) as white solid. Product was confirmed by LCMS (Method 1), m/z 473 (ES+, M+H), at 2.13 min.

**Step (ii):** 4-(2-(2-(3-(4'-hydroxy-2'-methyl-[1,1'-biphenyl]-3-yl)-3-oxopropyl)-5-oxopyrazolidin-1-yl)ethyl)benzoic acid (0.130 g, 0.27 mmol) was dissolved in DMA (3 mL). Sulfamoyl chloride (0.095 g, 0.82 mmol) was added at room temperature and allowed to stir at room temperature for 3 h. The reaction mixture was then partitioned between water (70 mL) and EtOAc (30 mL). Aqueous layer was further extracted with EtOAc (2 x 20 mL). Organic layers were combined and dried ($Na_2SO_4$). Solvent was removed in vacuo and the crude product was purified by gradient flash column chromatography product eluted at 0% to 5% methanol in DCM to afford pure 4-(2-(2-(3-(2'-methyl-4'-(sulfamoyloxy)-[1,1'-biphenyl]-3-yl)-3-oxopropyl)-5-oxopyrazolidin-1-yl)ethyl) benzoic acid (0.15 g, 99%) as white solid. Product was confirmed by LCMS (Method 1),, m/z 552 (ES+), at 2.14 min.

**Step (iiia) (racemic method):** 4-(2-(2-(3-(2'-methyl-4'-(sulfamoyloxy)-[1,1'-biphenyl]-3-yl)-3-oxopropyl) -5-oxopyrazolidin-1-yl)ethyl)benzoic acid (0.03 g, 0.054 mmol) was dissolved in methanol (3 mL) and 10% palladium on carbon with 50% moisture (0.02 g) was added. $H_2$ gas was purged through reaction mixture at room temperature for 2 h. After completion, the reaction mixture was filtered through celite bed, washed with MeOH (30 mL) and filtrate was concentrated in vacuo. Crude product was purified by gradient flash column chromatography (reverse phase, C18 silica), product eluted at 0% to 35% MeCN in water to afford 4-(2-(2-(3-hydroxy-3-(2'-methyl-4'-(sulfamoyloxy)-[1,1'-biphenyl]-3-yl)propyl)-5-oxopyrazolidin-1-yl)ethyl)benzoic acid (0.010 g, 33%) as brown solid. Product was confirmed by LCMS (Method 1), m/z 554 (ES+, M+H), at 2.01 min. **$^1$H NMR:** (400Mz, MeOD): 1.98-1.95 (t, 3H, J= 6.4Hz), 2.26 (s, 3H), 2.95-2.92 (m, 3H, J= 6.8Hz), 3.26 (s, 2H), 3.36 (s, 1H), 3.58-3.56 (m, 1H), 3.70-3.68 (m, 1H), 4.87-4.85 (d, 2H, J= 6.4Hz), 7.25-7.12 (m, 5H), 7.25 (s, 1H), 7.46-7.39 (m, 2H), 7.92 (s, 2H), 12.72 (s, 1H).

**Step (iiib) (chiral separation method):** 4-(2-(2-(3-(2'-methyl-4'-(sulfamoyloxy)-[1,1'-biphenyl]-3-yl)-3-oxopropyl)-5-oxopyrazolidin-1-yl)ethyl)benzoic acid (0.10 g, 0.18 mmol) was dissolved in ethanol (3 mL) and water (2 mL) and $CeCl_3$ (0.13 g, 0.54 mmol) was added to the reaction mixture. Reaction mixture was allowed to stir at 0°C for 5min. After this, sodium borohydride (0.027 g, 0.72 mmol) was added at 0°C and reaction mixture was stirred at room temperature for 1 h. The reaction mixture was then partitioned between water (30 mL) and EtOAc (2 x 20 mL). Aqueous layer was further acidified with 4 N aqueous HCl (3 mL), adjusted pH up to ~1 and extracted with EtOAc (2 x 50 mL). Organic layers were combined and dried ($Na_2SO_4$). Solvent was removed in vacuo to provide the crude product.

**Compounds 4R and 4S:** Racemic Compound 4 was enantiomerically separated with a Schimadzu Prep-HPLC system (CROMEGACHIRAL CCJ 25CM*20 mm, 5 $\mu$m column) using a mobile phase that is 60% 0.1% FA in heptane and 40% a mixture of IPA and methanol (60:40) at a flow rate of 19.00 mL/min, and with no gradient. Isomer 1: (0.0057 g, 24%; white solid) Product was confirmed by LCMS (Method 1), m/z 554 (ES+, M+H), at 2.02 min. Chiral HPLC: product purity was confirmed at 16.23 min. **$^1$H NMR:** (400MHz, MeOD) $\delta$: 1.98-1.93 (m, 2H), 2.27 (s, 3H), 2.97-2.91 (m, 4H), 3.27-3.27

(m, 2H), 4.88-4.85 (t, 2H, J= 6.4Hz), 7.23-7.18 (m, 4H), 7.34-7.31 (m, 3H), 7.46-7.41 (m, 2H), 7.93-7.92 (d, 2H, J= 6.8Hz) 7.63-7.61 (d, 2H, J= 8.8Hz), 7.73-7.71 (d, 1H, J= 7.6Hz), 7.83-7.78 (m, 3H), 8.58-8.56 (t, 1H, J= 5.2Hz). Isomer 2: (0.0047 g, 26%; white solid) Product was confirmed by LCMS(Method 1), m/z 554 (ES+, M+H), at 2.02 min. Chiral HPLC: product purity was confirmed at 10.62 min. **$^1$H NMR:** (400MHz, MeOD) δ: 1.98-193 (m, 3H), 2.27 (s, 3H), 2.97-2.93 (m, 4H), 3.27 (s, 2H), 4.88-4.85 (t, 2H, J= 6.4Hz), 7.24-7.25 (m, 4H), 7.31-7.29 (m, 2H), 7.34 (s, 1H), 7.46-7.39 (m, 2H) 7.93-7.91 (d, 2H, J= 7. 6Hz), 8.51 (s, 1H).

**Example 5: Synthesis of 4-(2-(2-(3-(4'-carbamoyl-2'-methyl-[1,1'-biphenyl]-3-yl)-3-hydroxypropyl)-5-oxopyrazolidin-1-yl)ethyl)-2,6-difluorobenzoic acid (Compound 5)**

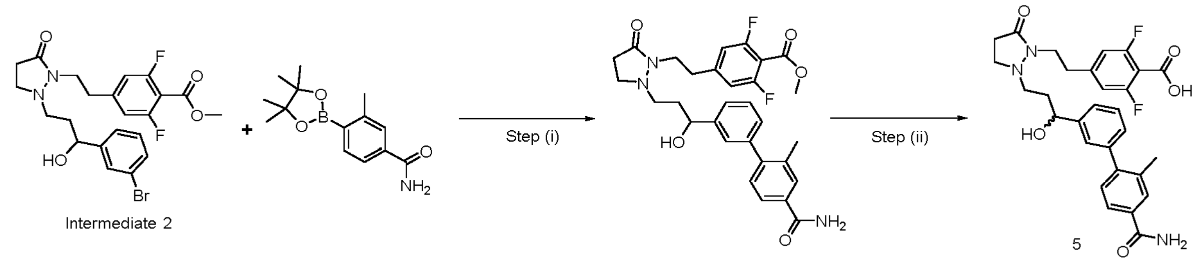


**Step (i): Intermediate 2,** methyl 4-(2-(2-(3-(3-bromophenyl)-3-hydroxypropyl)-5-oxopyrazolidin-1-yl)ethyl)-2,6-difluorobenzoateate (0.20 g, 0.60 mmol), 3-methyl-4-(4,4,5,5-tetramethyl-1,3,2-dioxaborolan-2-yl)benzamide (0.21 g, 0.79 mmol) and $K_2CO_3$ (0.17 g, 0.1.30 mmol) were dissolved in mixture of Dioxane: Water (1:1, 5 mL) and nitrogen gas was purged for 30 min at room temperature. After this, $PdCl_2$(dppf).DCM (0.099 g, 0.12 mmol) was added and the reaction mixture was allowed to stir at 80°C for 1 h. The reaction mixture was then partitioned between water (70 mL) and EtOAc (70 mL), aqueous layer was further extracted with EtOAc (2 x 30 mL). Organic layers were combined and dried ($Na_2SO_4$). Solvent was removed in vacuo and the crude product was purified by reverse phase gradient flash column chromatography (reverse phase, C18 silica), product eluted at 0% to 28% MeCN in water to afford methyl 4-(2-(2-(3-(4'-carbamoyl-2'-methyl-[1,1'-biphenyl]-3-yl)-3-hydroxypropyl)-5-oxopyrazolidin-1-yl)ethyl)-2,6-difluorobenzoate (0.29 g, 87%) as light brown solid. Product was confirmed by LCMS (Method 2), m/z 552 (ES+, M+H) at 1.92 min.

**Step (ii):** methyl 4-(2-(2-(3-(4'-carbamoyl-2'-methyl-[1,1'-biphenyl]-3-yl)-3-hydroxypropyl)-5-oxopyrazolidin-1-yl)ethyl)-2,6-difluorobenzoate (0.29 g, 0.29 mmol) was dissolved in Dioxane (4 mL) and water (2 mL). LiOH (0.11 g, 0.88 mmol) was added at room temperature and allowed to stir at room temperature for 16 h. Reaction mixture was acidified with 4 N aqueous HCl (4 mL) to adjust the pH ~1 and extracted with EtOAc (40 mL). The aqueous layer was further extracted with EtOAc (3 x 30 mL). Organic layers were combined and dried ($Na_2SO_4$). Solvent was removed in vacuo and the crude product was purified by reverse phase gradient flash column chromatography (reverse phase, C18 silica), product eluted at 0% to 23% ACN in water to afford 4-(2-(2-(3-(4'-carbamoyl-2'-methyl-[1,1'-biphenyl]-3-yl)-3-hydroxypropyl)-5-oxopyrazolidin-1-yl) ethyl)-2,6-difluorobenzoic acid (0.15 g, 53%) as an off-white solid. Product was confirmed by LCMS (Method 2), m/z 538 (ES+, M+H) at 1.49 min. **$^1$H NMR:** (400MHz, DMSO) δ: 1.23 (s, 2H), 1.81-1.80 (d, 2H, J= 4.8Hz), 2.26 (s, 3H), 2.75 (s, 3H), 3.17-3.16 (d, 4H, 3.6Hz), 4.12-4.11 (d, 1H, J= 4.8Hz), 4.71 (s, 1H), 5.47-5.46 (d, 1H, J= 3.6Hz), 6.75-6.73 (d, 2H, J= 7.2Hz), 7.25-7.22 (m, 2H), 7.37-7.33 (m, 2H), 7.43-7.39 (m, 2H), 7.76-7.74 (m, 1H), 7.82 (s, 1H), 8.02 (s, 1H).

**Compounds 5R and 5S:** The racemic mixture (70 mg) was enantiomerically separated with a Shimadzu Prep-HPLC system (CHROMEGACHIRAL CCO 250*25 mm, 5 μm column) using a mobile phase that is 80% 0.1% FA in heptane and 20% a mixture of IPA and methanol (70:30) with no gradient, at a flow rate of 20.00 mL/min. Isomer 1: (0.038 g, 13%; white solid) Product was confirmed by LCMS (Method 2), m/z 538 (ES+, M+H) at 1.59 min. Chiral HPLC: Product purity was confirmed at 14.48 min. **$^1$H NMR:** (400MHz, DMSO) δ:1.81-1.80 (d, 2H, J= 6.4Hz), 2.27 (s, 4H), 2.85 (s, 4H), 3.13-3.12 (d, 2H, J= 5.6Hz), 4.73 (s, 1H), 5.36 (s, 1H), 7.10-7.04 (m, 2H), 7.27-7.22 (m, 2H), 7.43-7.34 (m, 4H), 7.76-7.74 (m, 1H), 7.82 (s, 1H), 7.97 (s, 1H), 13.78 (s, 1H). Isomer 2: (0.052 g, 18%; off-white solid) Product was confirmed by LCMS (Method 2),, m/z 538 (ES+,M+H), at 1.60 min. Chiral HPLC: Product purity was confirmed at 10.50 min. **$^1$H NMR:** (400MHz, DMSO) δ: 1.81-1.80 (d, 2H, J= 5.6Hz), 2.27 (s, 3H), 2.85 (s, 3H), 3.14-3.12 (d, 2H, 7.6Hz), 4.72 (s, 1H), 5.37 (s, 1H), 7.06-7.04 (d, 2H, J= 8.8Hz), 7.27-7.22 (m, 2H), 7.43-7.34 (m, 4H), 7.76-7.74 (m, 1H), 7.82 (s, 1H), 7.97 (s, 1H), 13.79 (s, 1H).

**Example 6: Synthesis of 2-hydroxy-4-(2-(2-(3-hydroxy-3-(2'-methyl-4'-sulfamoyl-[1,1'-biphenyl]-3-yl)propyl)-5-oxopyrazolidin-1-yl)ethyl)benzoic acid (Compound 6)**

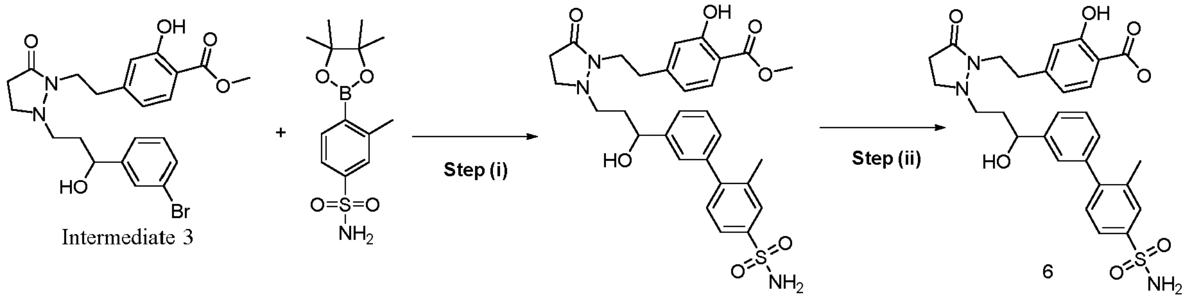


Intermediate 3

6

**Step (i): Intermediate 3,** methyl 4-(2-(2-(3-(3-bromophenyl)-3-hydroxypropyl)-5-oxopyrazolidin-1-yl)ethyl)-2-hydroxybenzoate (0.15 g, 0.32 mmol), 3-methyl-4-(4,4,5,5-tetramethyl-1,3,2-dioxaborolan-2-yl) benzenesulfonamide (0.14 g, 0.47 mmol) and $K_2CO_3$ (0.89 g, 0.64 mmol) were dissolved in 4 mL of a 1:1 dioxane: water mixture. Nitrogen gas was purged for 30 min at room temperature. After this, $PdCl_2$(dppf).DCM (0.026 g, 0.032 mmol) was added and the reaction mixture was allowed to stir at 80°C for 1 h. The reaction mixture was then partitioned between water (50 mL) and EtOAc (50 mL). Aqueous layer was further extracted with EtOAc (2 x 20 mL). Organic layers were combined and dried ($Na_2SO_4$). Solvent was removed in vacuo and the crude product was purified by gradient flash column chromatography (reverse phase, C18 silica), product eluted at 0% to 55% MeCN in water to afford methyl 2-hydroxy-4-(2-(2-(3-hydroxy-3-(2'-methyl-4'-sulfamoyl-[1,1'-biphenyl]-3-yl)propyl)-5-oxopyrazolidin-1-yl)ethyl)benzoate (0.13 g, 73%) as white solid. Product was confirmed by LCMS (Method 2),, m/z 568 (ES+), at 1.86 min.

**Step (ii):** methyl 2-hydroxy-4-(2-(2-(3-hydroxy-3-(2'-methyl-4'-sulfamoyl-[1,1'-biphenyl]-3-yl)propyl)-5-oxopyrazolidin-1-yl)ethyl)benzoate (0.13 g, 0.23 mmol) was dissolved in 4 mL of a 1:1 dioxane: water mixture at room temperature, and LiOH monohydrate (0.048 g, 1.15 mmol) was added, and the reaction mixture was allowed to stir at room temperature for 3 h. The Reaction mixture was partitioned between water (30 mL) and EtOAc (2 x 50 mL). The aqueous layer was further acidified with 4 N aqueous HCl (2 mL) to adjust pH to ~1, and then extracted with EtOAc (2 x 50 mL). Organic layers were combined and dried ($Na_2SO_4$). Solvent was removed in vacuo to afford pure 2-hydroxy-4-(2-(2-(3-hydroxy-3-(2'-methyl-4'-sulfamoyl-[1,1'-biphenyl]-3-yl)propyl)-5-oxopyrazolidin-1-yl)ethyl)benzoic acid (0.050 g, 39%) as off white solid. Product was confirmed by LCMS (Method 2), m/z 554 (ES+, M+H), at 1.90 min.

**Compounds 6R and 6S:** Racemic Compound 6 (50 mg) was enantiomerically separated with a Waters Prep-HPLC system (CHIRALPAK AD-H 250X10 mm 5 $\mu$m column) using a mobile phase that was 80% 0.1% FA in heptane and 20% IPA:acetonitrile (70:30) with no gradient, at a flow rate of 7.00 mL/min. Isomer 1: (0.006 g, 4.7%; sticky solid) Product was confirmed by LCMS (Method 2),, m/z 554 (ES+, M+H), at 1.86 min. Chiral HPLC: Product purity was confirmed at 18.45 min. **$^1$H NMR:** (400MHz, MeOD) $\delta$: 1.971-1.955 (d, 3H, J=6.4Hz), 2.331 (s, 3H), 2.908-2.853 (m, 4H), 3.59-3.56 (t, 3H, J= 4.8Hz), 3.70-3.68 (t, 3H, J= 4.6Hz), 6.81 (s, 2H), 7.27-7.25 (d, 1H, J= 7.2Hz), 7.38-7.36 (d, 2H, J= 8.0Hz), 7.49-7.43 (m, 2H), 7.78-7.76 (d, 2H, J= 7.6Hz), 7.84 (s, 1H). Isomer 2: (0.007 g, 4.0%; sticky solid) Product was confirmed by LCMS (Method 3), m/z 554 (ES+, M+H), at 1.87 min. Chiral HPLC: Product purity was confirmed at 29.47 min. **$^1$H NMR:** (400MHz, MeOD) $\delta$: 1.99-1.96 (m, 3H), 2.33-2.30 (d, 3H, J= 10.4Hz), 2.91-2.86 (m, 4H), 3.58-3.56 (t, 2H, J= 4.6Hz), 3.70-3.68 (t, 2H, J= 4.6Hz), 6.82 (s, 2H), 7.27-7.25 (d, 1H, J= 7.2Hz), 7.38-7.36 (d, 2H, J= 8.0Hz), 7.49-7.43 (m, 2H), 7.78-7.76 (d, 2H, J= 8.0Hz), 7.83 (s, 1H).

**Example 7: Synthesis of 4-(2-(2-(3-(4'-carbamoyl-2'-methyl-[1,1'-biphenyl]-3-yl)-3-hydroxypropyl)-5-oxopyrazolidin-1-yl)ethyl)-2-hydroxybenzoic acid (Compound 7)**

Compound 7 was synthesized by method described in Example 6 using 3-methyl-4-(4,4,5,5-tetramethyl-1,3,2-dioxaborolan-2-yl)benzamide to give 4-(2-(2-(3-(4'-carbamoyl-2'-methyl-[1,1'-biphenyl]-3-yl)-3-hydroxypropyl)-5-oxopyrazolidin-1-yl)ethyl)-2-hydroxybenzoic acid (0.075 g, 86 %) as an off-white solid. Product was confirmed by LCMS (Method 2),, m/z 518 (ES+, M+H), at 1.62 min.

**Compounds 7R and 7S:** Racemic Compound 7 (75 mg) was separated with a Waters Prep-HPLC system (CHIRALPAK AD-H 250X10 mm 5 $\mu$m column) using a mobile phase that was 75% 0.1% FA in heptane and 25% a mixture of IPA and acetonitrile (70:30) at a flow rate of 8.00 mL/min, with no gradient. Isomer 1: (0.016 g, 19%; off-white solid) Product was confirmed by LCMS (Method 2), m/z 518 (ES+, M+H), at 1.74 min. Chiral HPLC: product purity was confirmed at 11.21 min. **$^1$H NMR:** (400MHz, DMSO) $\delta$: 1.81-1.80 (d, 2H, J= 6.0Hz), 2.27 (s, 3H), 2.54 (s, 1H), 2.80-2.7 (d, 4H, J=

6.4Hz), 3.153 (s, 3H), 4.74-4.71 (t, 1H, J= 6.0Hz), 5.32 (s, 1H), 6.78-6.74 (m, 2H) 7.27-7.22 (m, 2H), 7.36-7.33 (m, 2H), 7.43-7.38 (m, 2H), 7.68-7.66(d, 1H, J= 8.0Hz), 7.76-7.73 (dd, 1H, J= 1.6Hz), 7.81 (s, 1H), 7.97 (s, 1H), 11.20 (s,1H), 13.84 (s, 1H). Isomer 2: (0.025 g, 29%; off-white solid) Product was confirmed by LCMS (Method 2), m/z 518 (ES+, M+H), at 1.73 min. Chiral HPLC: product purity was confirmed at 9.72 min. **$^1$H NMR:** (400MHz, DMSO) δ: 1.81-1.80 (d, 2H, J= 6.4Hz), 2.27 (s, 4H), 2.80 (s, 4H), 3.15-3.14 (d, 3H, J= 6.4Hz), 4.74-4.72 (d, 1H, J= 6.0Hz), 5.31 (s, 1H),6.76-6.74 (d, 1H, J= 8.4Hz), 6.78 (s, 1H), 7.27-7.22 (m, 2H), 7.36-7.33 (m, 2H), 7.43-7.38 (m, 2H), 7.68-7.66 (d, 1H, J= 8.0Hz), 7.76-7.73 (dd, 1H, J= 1.6Hz), 7.81 (s, 1H), 7.97 (s, 1H), 11.20 (s,1H), 13.84 (s, 1H).

**Example 8: Synthesis of 2,6-difluoro-4-(2-(2-(3-hydroxy-3-(2'-methyl-4'-sulfamoyl-[1,1'-biphenyl]-3-yl)propyl)-5-oxopyrazolidin-1-yl)ethyl)benzoic acid (Compound 8)**

Compound (8) was synthesized by the method described in Example 5, using 3-methyl-4-(4,4,5,5-tetramethyl-1,3,2-dioxaborolan-2-yl) benzenesulfonamide to give 2,6-difluoro-4-(2-(2-(3-hydroxy-3-(2'-methyl-4'-sulfamoyl-[1,1'-biphenyl]-3-yl)propyl)-5-oxopyrazolidin-1-yl)ethyl)benzoic acid (0.040 g, 23%) as white solid. Product was confirmed by LCMS (Method 2), m/z 574 (ES+, M+H) at 1.58 min.

**Compounds 8R and 8S:** Racemic Compound (8) (40 mg) was enantiomerically separated with a Shimadzu Prep-HPLC system (CHROMEGACHIRAL CCO 250*25 mm, 5 μm column) using a mobile phase that is 80% 0.1% FA in heptane and 20% a mixture of IPA and acetonitrile (70:30) with no gradient, and a flow rate of 22.00 mL/min. Isomer 1: (0.010 g, 5.7%; sticky gum) Product was confirmed by LCMS (Method 2), m/z 574 (ES+, M+H) at 1.58 min. Chiral HPLC: Product purity was confirmed at 28.03 min. **$^1$H NMR:** (400MHz, MeOD) δ: 1.30 (s, 3H), 1.98-1.96 (d, 3H, J= 6.0Hz), 2.34 (s, 4H), 2.96-2.93 (t, 6H, J= 6.6Hz), 6.98-6.95 (d, 2H, J= 9.2Hz), 7.28-7.25 (m, 1H), 7.38-7.36 (m, 2H), 7.50-7.43 (m, 2H), 7.78-7.76 (dd, 1H, J= 1.5Hz), 7.84-7.84 (m, 1H). Isomer 2: (0.012 g, 6.8%; sticky gum) Product was confirmed by LCMS (Method 2),, m/z 574 (ES+, M+H), at 1.58 min. Chiral HPLC: Product purity was confirmed at 20.05 min. **$^1$H NMR:** (400MHz, MeOD) δ: 1.30 (s, 3H), 1.97-1.96 (d, 2H, J=5.6Hz), 2.34-2.32 (m, 3H), 2.96-2.93 (t, 4H, J= 6.4Hz), 6.98-6.95 (d, 2H, J= 9.2Hz), 7.28-7.26 (m, 1H), 7.38-7.36 (m, 2H), 7.48-7.45 (m, 2H), 7.78-7.76 (dd, 1H, J= 1.5Hz), 7.84 (s, 1H).

**Example 9: Synthesis of (3'-(3-(2-(4-(1H-tetrazol-5-yl)phenethyl)-3-oxopyrazolidin-1-yl)-1-hydroxypropyl)-2-methyl-[1,1'-biphenyl]-4-carboxamide (Compound 9)**

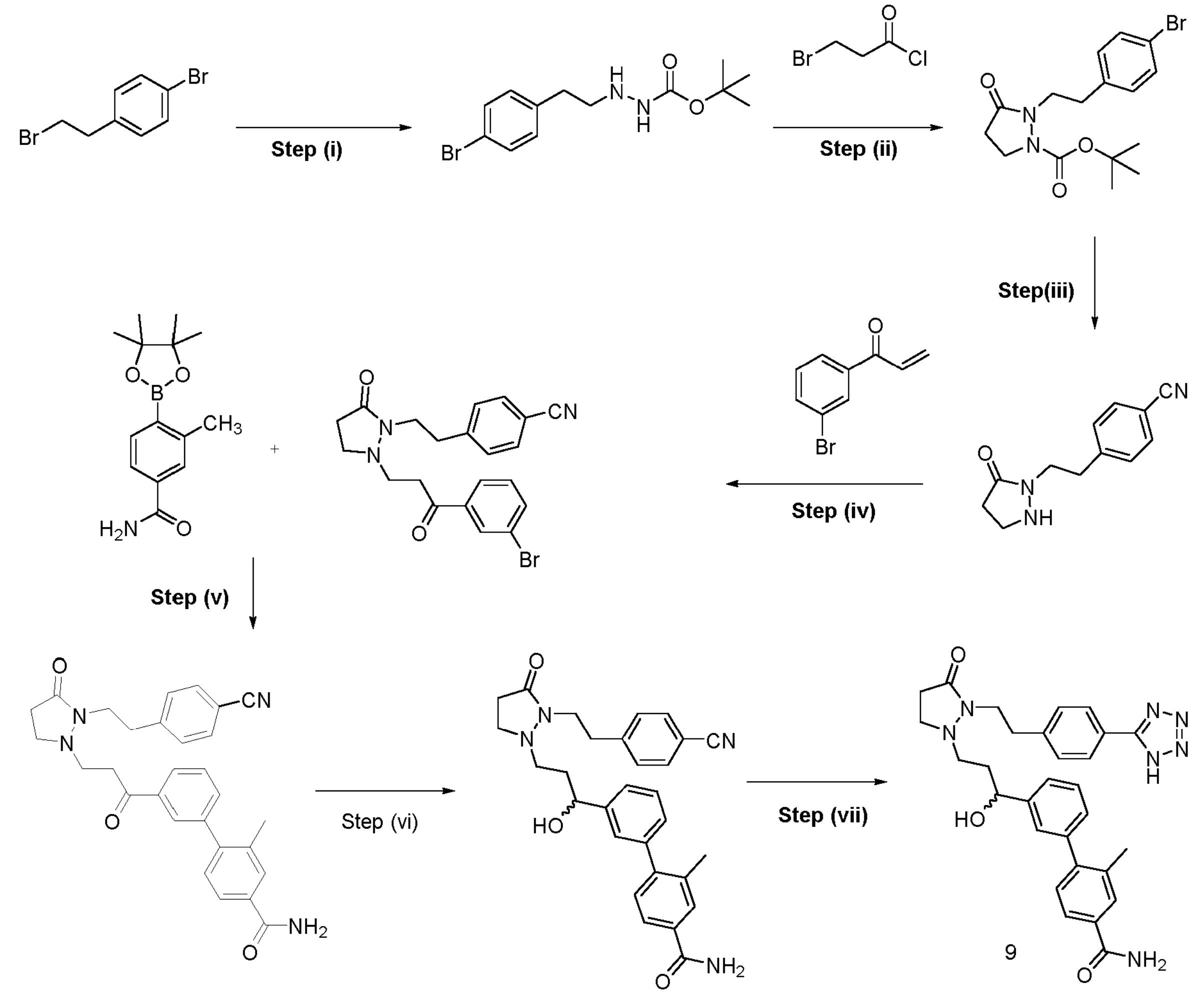


**Step (i):** 1-bromo-4-(2-bromoethyl) benzene (10.00 g, 38.18 mmol), *tert*-Butyl hydrazine carboxylate (7.57 g, 57.27 mmol), $NaHCO_3$ (12.84 g, 15.27 mmol) and NaI (0.57 g, 3.82 mmol) were suspended in MeCN (100 mL) at room temperature and the reaction mixture was allowed to stir at 70°C for 72 h. The reaction mixture was concentrated under *vacuo* and the residue was partitioned between water (1000 mL) and EtOAc (800 mL), aqueous layer was further extracted with EtOAc (3 x 500 mL). The organic layers were combined and dried ($Na_2SO_4$). Solvent was removed in *vacuo* and the crude product was purified by gradient column chromatography (normal phase, silica), product was eluted at 0% to 15% EtOAc in hexane to afford *tert*-butyl 2-(4-bromophenethyl)hydrazine-1-carboxylate (5.0 g, 42%) as an off-white solid. Product was confirmed by LCMS (Method 2), m/z 260 (ES+, M+H-tBu) at 2.34 min.

**Step (ii):** *tert*-butyl 2-(4-bromophenethyl)hydrazine-1-carboxylate (5.00 g, 15.89 mmol) was dissolved in DMF (50 mL) at room temperature, and potassium carbonate (10.98 g, 79.47 mmol) was added, and allowed to stir at room temperature for 15 min. 3-Bromopropionyl chloride (2.40 mL, 23.56 mmol) was then added drop wise at room temperature and the reaction mixture was allowed to stir at room temperature for 24 h. The reaction mixture was concentrated under *vacuo.* Obtained residue was partitioned between water (500 mL) and EtOAc (400 mL), the aqueous layer was further extracted with EtOAc (2 x 300 mL). Organic layers were combined and dried over $Na_2SO_4$), and the solvent was removed in *vacuo.* The crude product was purified by gradient column chromatography (normal phase, silica), with 0% to 30% EtOAc in hexane to afford tert-butyl 2-(4-bromophenethyl)-3-oxopyrazolidine-1-carboxylate (4.0 g, 68%) as yellow sticky solid. Product was confirmed by LCMS (Method 2), m/z 313 (ES+, M+H-tBu), at 2.65 min.

**Step (iii):** *tert*-butyl 2-(4-bromophenethyl)-3-oxopyrazolidine-1-carboxylate (2.00 g, 5.43 mmol) was dissolved in DMF (20 mL). Nitrogen gas was purged at room temperature for 15 min. After this, zinc cyanide (1.28 g, 10.87 mmol) and tetrakistriphenylphosphine-palladium 0 (0.63 g. 0.54 mmol) were added and reaction mixture was allowed to stir at 150°C for 1 h in microwave (reaction split over 4 reaction vials). The reaction mixture was partitioned between water (400 mL) and EtOAc (300 mL). Aqueous layer was further extracted with EtOAc (2 x 200 mL). Organic layers were combined and dried ($Na_2SO_4$). Solvent was removed in *vacuo* and the crude product was purified by reverse phase gradient flash column chromatography (reverse phase, C18 Silica), product eluted at 0% to 68% MeCN in water to afford 4-(2-(5-oxopyrazolidin-1-yl)ethyl)benzonitrile (0.65 g, 41%) as yellow solid. Product was confirmed LCMS (Method 2), m/z 216 (ES+, M+H),

at 1.63 min.

**Step (iv):** 4-(2-(5-oxopyrazolidin-1-yl)ethyl) (0.60 g, 2.79 mmol) and 1-(3-bromophenyl) prop-2-en-1-one (2.90 g, 13.95 mmol) were suspended in MeOH (6 mL) at room temperature and reaction mixture was allowed to stir at room temperature for 15 min. After this, TEA (2.00 mL, 13.95 mmol) was added at room temperature and allowed to stir at 60°C for 4 h. The reaction mixture was partitioned between water (200 mL) and EtOAc (150 mL) and aqueous layer was further extracted with EtOAc (2 x 120 mL). Organic layers were combined and dried ($Na_2SO_4$). Solvent was removed in *vacuo* and crude product was purified by reverse phase gradient flash column chromatography (reverse phase, C18 silica), product eluted at 0% to 72% ACN in water to afford 4-(2-(2-(3-(3-bromophenyl)-3-oxopropyl)-5-oxopyrazolidin-1-yl) ethyl) benzoate (0.50 g, 49%) as yellow oil. Product was confirmed by LCMS (Method 2), m/z 426 (ES+, M+H), at 2.58 min.

**Step (v):** 4-(2-(2-(3-(3-bromophenyl)-3-oxopropyl)-5-oxopyrazolidin-1-yl)ethyl) benzonitrile (0.50 g, 1.18 mmol), 3-methyl-4-(4,4,5,5-tetramethyl-1,3,2-dioxaborolan-2-yl) benzamide (0.46 g, 1.76 mmol) and $K_2CO_3$ (0.33 g, 2.35 mmol) were dissolved in 1,4-dioxane (5 mL) and water (5 mL). Nitrogen gas was purged at room temperature for 20 min. After this, $PdCl_2$(dppf).DCM (0.096 g, 0.12 mmol) was added and the reaction mixture was allowed to stir at 80°C for 2 h. The reaction mixture was partitioned between water (200 mL) and EtOAc (150 mL). Aqueous layer was further extracted with EtOAc (2 x 100 mL). Organic layers were combined and dried ($Na_2SO_4$). Solvent was removed in *vacuo* and crude product was purified by reverse phase gradient flash column chromatography (reverse phase, C18 Silica), product eluted at 0% to 55% MeCN in water to afford **3'-(3-**(2-(4-cyanophenethyl)-3-oxopyrazolidin-1-yl) propanoyl)-2-methyl-[1,1'-biphenyl]-4-carboxamide (0.25 g, 44%) as off-white solid. Product was confirmed by LCMS (Method 2), m/z 481 (ES+, M+H), at 2.18 min.

**Step (vi)** 3'-(3-(2-(4-cyanophenethyl)-3-oxopyrazolidin-1-yl) propanoyl)-2-methyl-[1,1'-biphenyl]-4-carboxamide (0.20 g, 0.42 mmol) was dissolved in ethanol (2 mL) and water (2 mL). After this, $NaBH_4$ (0.03 g, 0.83 mmol) was added at 0°C and allowed to stir at room temperature for 1 h. The reaction mixture was partitioned between water (50 mL) and EtOAc (50 mL) and aqueous layer was further extracted with EtOAc (2 x 30 mL). Organic layers were combined and dried ($Na_2SO_4$). Solvent was removed in *vacuo* to afford pure 3'-(3-(2-(4-cyanophenethyl)-3-oxopyrazolidin-1-yl)-1-hydroxypropyl)-2-methyl-[1,1'-biphenyl]-4-carboxamide (0.17 g, 85%) as off white solid. Product was confirmed by LCMS (Method 2), m/z 483 (ES+, M+H), at 2.14 min.

**Step (vii):** 3'-(3-(2-(4-cyanophenethyl)-3-oxopyrazolidin-1-yl)-1-hydroxypropyl)-2-methyl-[1,1'-biphenyl]-4-carboxamide (0.17 g, 0.35 mmol) was suspended in DMF (2 mL) at room temperature, and sodium azide (0.24 g, 3.50 mmol) and ammonium chloride (0.20 g, 3.50 mmol) were added and allowed it to stir at 100°C for 48 h. The reaction mixture was partitioned between water (70 mL) and EtOAc (50 mL) and aqueous layer was further extracted with EtOAc (3 x 50 mL). Organic layers were combined and dried ($Na_2SO_4$). Solvent was removed in *vacuo* and the crude product was purified by reverse phase gradient flash column chromatography (reverse phase, C18 Silica), product eluted at 0% to 45% MeCN in water to afford (3'-(3-(2-(4-(1H-tetrazol-5-yl)phenethyl)-3-oxopyrazolidin-1-yl)-1-hydroxypropyl)-2-methyl-[1,1'-biphenyl]-4-carboxamide (0.10 g, 54%) as an off-white solid. Product was confirmed by LCMS (Method 2),, m/z 526 (ES+), at 1.91 min. Chiral HPLC: Product purity was confirmed at 13.67 min. and 15.68 min. **$^1$H NMR** (400MHz, DMSO): 1.85-1.83 (d, 2H, J=6.4Hz), 2.29 (s, 3H), 2.89-2.86 (m, 4H), 3.18 (S, 4H), 4.76 (S, 1H), 5.40 (m, 1H), 7.29-7.24 (m, 2H), 7.45-7.36 (m, 6H), 7.76-7.76 (d, 1H, J=1.2Hz), 7.83 (s, 1H), 7.95-7.93 (d, 2H, J=8.0Hz), 8.00 (s, 1H).

**Compounds 9R and 9S:** Racemic Compound 9 (90 mg) was enantiomerically separated with a Waters 600 Controller -HPLC system (CHIRALPAK_IG_SFC_21mm*250 mm, 5 $\mu$m column) using a mobile phase that is 90% heptane and 10% IPA and acetonitrile (70:30), with no gradient and a flow rate of 23.00 mL/min. Isomer 1: (0.028 g, 31%; white solid) Product was confirmed by LCMS (Method 2), m/z 526 (ES+, M+H), at 1.75 min. Chiral HPLC: product purity was confirmed at 13.19 min. **$^1$H NMR:** (400MHz, DMSO) 1.82-1.81 (d, 2H, J=5.6Hz), 2.10 (s, 3H), 2.87-2.83 (t, 4H, J=6.6Hz), 3.15 (s,4H), 4.73 (s, 1H), 5.36 (s, 1H), 7.26-7.22 (m, 2H), 7.42-7.33 (m, 6H), 7.75-7.73 (m, 1H), 7.81 (s, 1H), 7.92-7.90 (d, 2H, J=8.0Hz), 7.97 (s, 1H). Isomer 2: (0.025 g, 28%; white solid) Product was confirmed by LCMS (Method 2),, m/z 526 (ES+, M+H), at 1.76min. Chiral HPLC: product purity was confirmed at 14.89 min. (98%). **$^1$H NMR:** (400MHz, DMSO): 1.82-1.81 (d, 2H, J=6.0Hz), 2.26 (s, 3H), 2.83-2.66 (m, 4H), 3.07 (s, 4H), 4.73 (s, 1H), 5.37 (s, 1H), 7.43-7.22 (m, 8H), 7.75-7.73 (m, 1H), 7.80 (s, 1H), 7.90-7.89 (d, 2H, J=8.0Hz), 7.98 (s, 1H).

**Example 10: Biological Assays**

***Cloning, Baculovirus generation, large scale infection of HEK293 cells and membrane preparation:*** Human prostaglandin E2 receptor 4 ($EP_4$) was cloned into pBacMam expression vector (GeneScript, UK). Transposition of $EP_4$ DNA was performed using Invitrogen's Bac-to-Bac Baculovirus Expression Systems. P0 baculovirus was generated by transfecting SF9 Cells with bacmid DNA using Cellfectin II transfection reagent (ThermoFisher Scientific, UK). Following P0 generation P1 virus was then generated ready for large scale infection and membrane preparation. HEK293 cells were grown in DMEM (ThermoFisher Scientific, UK), supplemented with 10% heat inactivated fetal bovine serum (FBS). Cells were infected at a seeding density of 3.5 million cells/mL in 500 $cm^3$ flasks at 5% v/v $EP_4$ Bacman. Expression was carried out over a 36 hr period at 37°C with 5% $CO_2$. The cells were removed using PBS and a cell

scrapper. The cell culture was centrifuged at 2500 RPM for 10 mins at 4°C. The supernatant was then poured off and the pellet stored at -80°C. The pellet was defrosted and re-suspended in 15 mL of homogenising buffer (20 mM HEPES, 10 mM EDTA, pH 7.4). Then homogenised in mechanical homogeniser (VMR) for 10 seconds. The membrane was centrifuged in centrifuge tubes at 40,000 g for 15 mins at 4°C. The supernatant was poured away and re-suspended in 15 mL of homogenising buffer. Homogenised for 20 seconds. The membrane was centrifuged at 40,000 g for 45 mins at 4°C. The membrane was re-suspended in 3 mL of storage buffer (20 mM HEPES, 0.1 mM EDTA, pH 7.4) mixing well. The resulting membranes were then stored at - 80°C.

***cAMP Gs Functional Assay:*** cAMP production following $EP_4$ receptor activation was determined using the Homogeneous Time-Resolved Fluorescence (HTRF) cAMP dynamic-2 assay (Cisbio, France). HEK293 cells were transfected using a 0.5 % EP4 Bacmam virus for 36 hours, before dissociating the cells, and freezing at -150°C.

On the day of testing, increasing concentration of test compounds, alongside positive controls (10 uM $PGE_2$ (Tocris, Abingdon, UK)) and negative control (DMSO (Sigma-Aldrich, UK) were added to a ProxiPlate-384 Plus, White 384-shallow well Mircoplate, (PerkinElmer, USA) using the ECHO dispense.

Cells were defrosted in a water bath and resuspended in DMEM supplemented with 10% FBS before centrifuging at 1200 RPM for 5 mins to form a pellet. The pellet was resuspended in assay buffer (DMEM + 0.5 mM IBMX (Tocris, Abingdon, UK)) to 0.5 x $10^6$ cells/mL. Cell suspension, for a final assay concentration of 5000 cell/well was added using the multidrop to the pre-dispensed assay plate. The plate was then incubated at 37°C for 30 mins, with 5% $CO_2$. The cAMP production was determined as manufacturer's instructions, before plates were read on a PheraStar fluorescence plate reader (BMG LabTech, Germany).

The $pEC_{50}$ values (-Log M) were calculated from the mid-point of the curve using Dotmatics as shown in Table 2.

**Table 2: Activity ($pEC_{50}$ and Emax values) of selected compounds of Formula (1) for the prostaglandin $E_2$ receptor 4 (cAMP human $EP_4$)**

| Example | cAMP human $EP_4$ $pEC_{50}$ | cAMP human $EP_4$ Emax |
|---|---|---|
| 1; racemate | 8.4 | 97 |
| 1; isomer 1 | 7.3 | 97 |
| 1; isomer 2 | 8.7 | 99 |
| 2; racemate | 8.5 | 99 |
| 2; isomer 1 | 6.8 | 95 |
| 2; isomer 2 | 9.3 | 98 |
| 3; racemate | 8.6 | 97 |
| 3; isomer 1 | 7.4 | 93 |
| 3; isomer 2 | 8.9 | 98 |
| 4; racemate | 8.2 | 97 |
| 4; isomer 1 | 6.6 | 93 |
| 4; isomer 2 | 8.6 | 97 |
| 5; racemate | 7.3 | 97 |
| 5; isomer 1 | 6.1 | 88 |
| 5; isomer 2 | 7.8 | 96 |
| 6; isomer 1 | 8.4 | 97 |
| 6; isomer 2 | 7.3 | 96 |
| 7; isomer 1 | 8.4 | 99 |
| 7; isomer 2 | 7 | 97 |
| 8; isomer 1 | 5.6 | 96 |
| 8; isomer 2 | 7.5 | 99 |
| 9; racemate | 9.8 | 97 |
| 9; isomer 1 | 7.4 | 93 |

(continued)

| Example | cAMP human $EP_4$ $pEC_{50}$ | cAMP human $EP_4$ Emax |
|---|---|---|
| 9; isomer 2 | 9.6 | 96 |

## UNIDIRECTIONAL CACO-2 CELL PERMEABILITY ASSAY

Caco-2 cells (ECACC) were seeded onto 24-well Transwell plates at 2 x $10^5$ cells per well and used in confluent monolayers after a 21 day culture at 37 °C under 5% $CO_2$. Test compounds were incubated at 10 μM, 0.2% DMSO final, n=2 in assay buffer (Hanks balanced salt solution supplemented with 25 mM HEPES, adjusted to pH 6.5). Hanks balanced salt solution supplemented with 25 mM HEPES, adjusted to pH 7.4 (0.2% DMSO final) was used for the basolateral chamber (as receiver).

Incubations were performed at 37 °C, with samples removed from both donor and acceptor chambers at T=0 and 1 hour and compound analysed by mass spectrometry (LC-MS/MS) including an analytical internal standard (0.5 μM carbamazepine).

Apparent permeability ($P_{app}$) values are shown in Table 3 and were determined from the relationship:

$$P_{app} = \frac{Compound_{Acceptor\,T=end} / (Compound_{Donor} \times V_{Donor})}{Incubation\ Time} \times \frac{V_{Donor}}{Area \times 60 \times 10^{-6}\ cm/s}$$

Where V is the volume of each Transwell compartment (apical 125 μL, basolateral 600 μL), and concentrations are the relative MS responses for compound (normalized to internal standard) in the donor chamber before incubation and acceptor chamber at the end of the incubation Area = area of cells exposed for drug transfer (0.33 $cm^2$).

Lucifer Yellow (LY) was added to the apical buffer in all wells to assess viability of the cell layer. As LY cannot freely permeate lipophilic barriers, a high degree of LY transport indicates poor integrity of the cell layer and wells with a LY $P_{app}$ > 10 x $10^{-6}$ cm/s were rejected.

Compound recovery from the wells was determined from MS responses (normalized to internal standard) in donor and acceptor chambers at the end of incubation compared to response in the donor chamber pre-incubation.

**Table 3 - Mean apparent permeability ($P_{app}$) values**

| Example | Caco-2 Mean $P_{app}$ A-B ($10^{-6}$ cm/s) |
|---|---|
| 1; racemate | 0.68 |
| 1; isomer 1 | 0.86 |
| 2; isomer 1 | <0.29 |
| 2; isomer 2 | <0.47 |
| 3; racemate | 0.3 |
| 3; isomer 1 | 0.23 |
| 3; isomer 2 | 0.72 |
| 4; isomer 1 | 0.3 |
| 4; isomer 2 | 0.67 |
| 5; isomer 2 | <0.1 |
| 6; isomer 1 | <0.1 |
| 7; isomer 1 | <0.1 |
| 7; isomer 2 | <0.1 |
| 8; isomer 2 | <0.3 |
| 9; racemate | <0.1 |
| 9; isomer 2 | <0.1 |

## Claims

**1.** A compound of the Formula I:

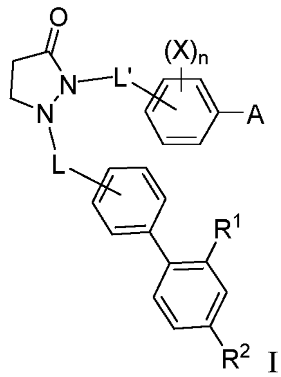

I

or a pharmaceutically acceptable salt solvate, hydrate, tautomer or optical isomer thereof, wherein;

A is OR', C(O)R', $CO_2R'$, C(O)N(R')$_2$, C(O)N(R')S(O)$_2R^3$, S(O)$_2$R', S(O)$_2$OR', $SO_2N(R')_2$, $C_{1-8}$ alkyl, cycloalkyl, heterocycloalkyl, aryl, or heteroaryl;
X is halo, OR', COOR', or $C_{1-6}$ alkyl;
L and L' are each independently a $C_{2-4}$ alkylene;
$R^1$ is H, halo, CN, $NO_2$, OR', SR', COOR', $C_{1-6}$ alkoxy, or $C_{1-6}$ alkyl;
$R^2$ is OR', OC(O)$R^3$, OC(O)O$R^3$, $CO_2R'$, CON(R')$_2$, $SO_2N(R')_2$, $SO_2R^3$, $OSO_2R^3$, or $OSO_2N(R')_2$;
$R^3$ is $C_{1-6}$ alkyl, $C_{3-6}$ cycloalkyl, or a phenyl;
R' is H, $C_{1-6}$ alkyl, or $C_{3-6}$ cycloalkyl; and
n is 0, 1, 2, or 3;
wherein at each occurrence, alkyl, alkylene, alkoxy, and cycloalkyl are each optionally and independently substituted with up to 3 instances of OH, SH, CN, $NO_2$, COOH, halo, or $COOC_{1-4}$ alkyl;
wherein at each occurrence, heterocycloalkyl, aryl, and heteroaryl are each optionally and independently substituted with up to 3 instances of OR', SR', CN, $NO_2$, $CO_2R'$, halo, $C_{1-4}$ alkyl, or oxo.

**2.** The compound according to claim 1, wherein

(i) L' is the group

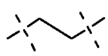

;

and/or
(ii) L is the group

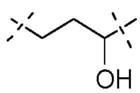

.

**3.** The compound according to claim 1 or 2, wherein the compound is a compound of Formula (1):

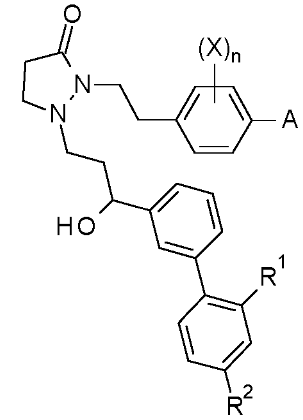

(1),

Or a pharmaceutically acceptable salt, solvate, hydrate, tautomer, or optical isomer thereof, wherein A, X, $R^1$, $R^2$ and n are the same as defined in claim 1.

**4.** The compound according to any one of claims 1-3, wherein A is selected from C(O)OR', $C(O)N(R')S(O)_2R^3$, $S(O)_2OR'$, $C_{1-8}$ alkyl, heterocycloalkyl, or heteroaryl, wherein the heterocycloalkyl, and heteroaryl are each optionally and independently substituted with up to 3 instances of OR', SR', halo, $C_{1-4}$ alkyl, or oxo; preferably wherein A is selected from the group consisting of:

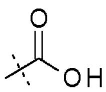 , 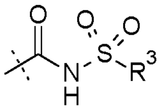 , 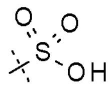 , 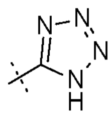 , 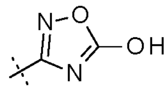 , 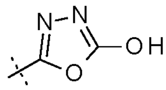 ,

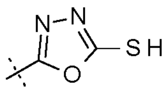 , 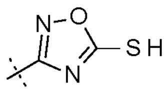 , 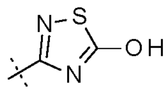 , 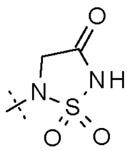 , 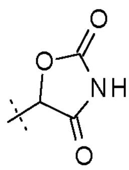 ;

more preferably wherein A is:

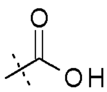 or 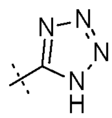 ;

most preferably wherein A is

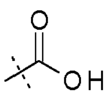 .

**5.** The compound according to any one of claims 1-4, wherein:

(i) X is halo or OR', preferably wherein X is F or OH; more preferably wherein X is F or OH, and n is 1, or 2; or
(ii) n is 0.

**6.** The compound according to any one of claims 1-5, wherein $R^1$ is H, OH, halo, CN, $C_{1-6}$ alkoxy optionally substituted with 1-3 fluorine atoms or $C_{1-6}$ alkyl optionally substituted with 1-3 fluorine atoms; preferably wherein $R^1$ is $C_{1-6}$ alkyl

optionally substituted with 1-3 fluorine atoms; more preferably wherein $R^1$ is methyl.

**7.** The compound according to any one of claims 1-6, wherein:

(i) $R^2$ is OR', $CON(R')_2$, $SO_2N(R')_2$, or $OSO_2N(R')_2$, preferably wherein $R^2$ is OH, $CONH_2$, $SO_2NH_2$, or $OSO_2NH_2$; or

(ii) $R^2$ is $CON(R')_2$, $SO_2N(R')_2$, $OSO_2R^3$, or $OSO_2N(R')_2$; preferably wherein $R^2$ is $CONH_2$, $SO_2NH_2$, or $OSO_2NH_2$.

**8.** The compound according to any one of claims 1-7 which is a compound of Formula (2a), (2b), (2c), or (2d):

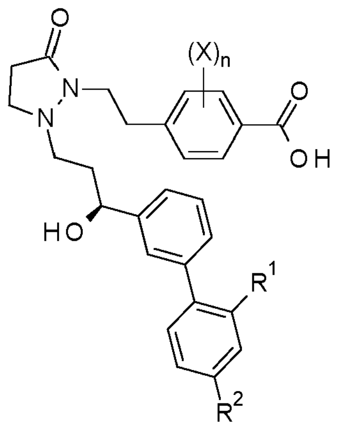
(2a)

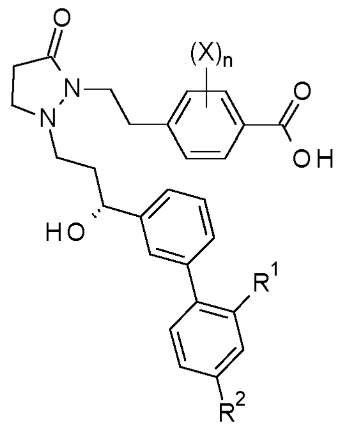
(2b)

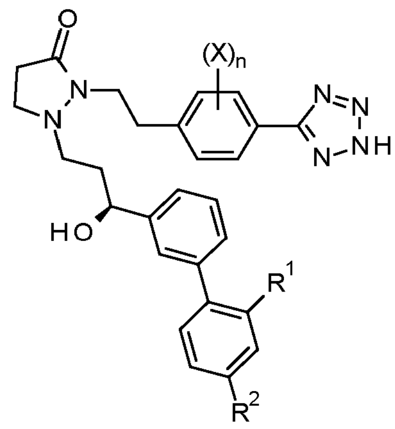
(2c)

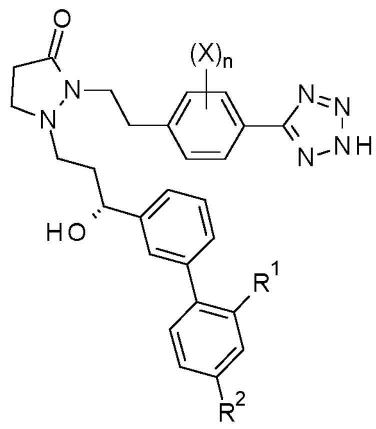
(2d),

or a pharmaceutically acceptable salt, solvate, hydrate or tautomer thereof, wherein X, $R^1$, $R^2$ and n are the same as defined in any preceding claim.

**9.** The compound according to claim 1, which is a compound of Formula (5), (5a), (5b), (6), (6a), (6b):

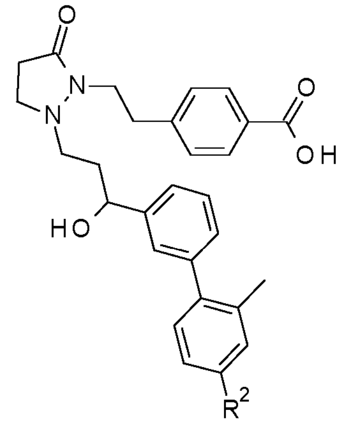
(5)

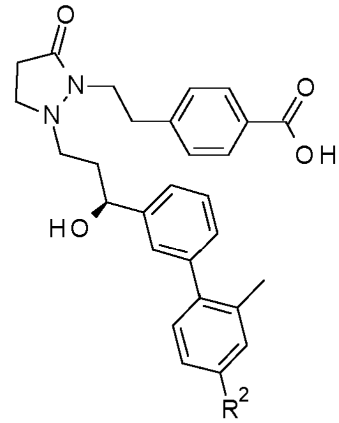
(5a)

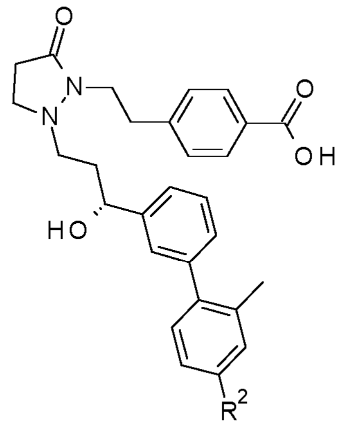
(5b)

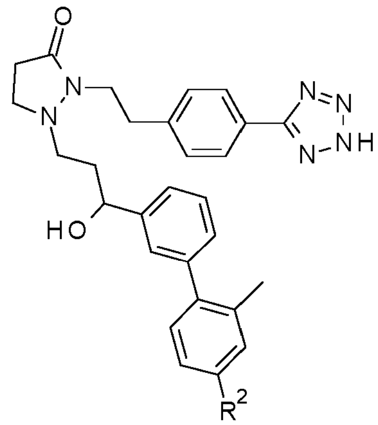
(6)

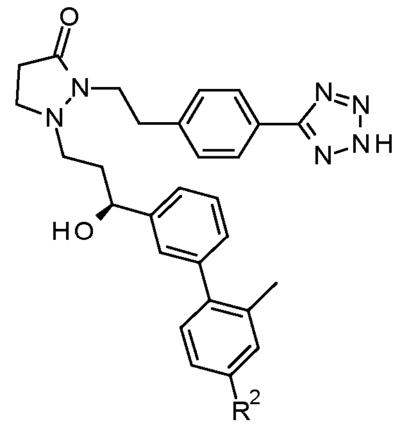
(6a)

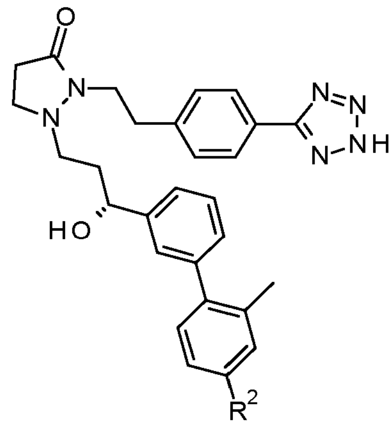
(6b)

or a pharmaceutically acceptable salt, solvate, hydrate or tautomer thereof.

**10.** The compound according to claim 1, wherein the compound is selected from the group consisting of:

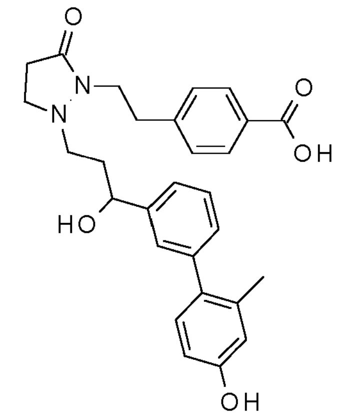
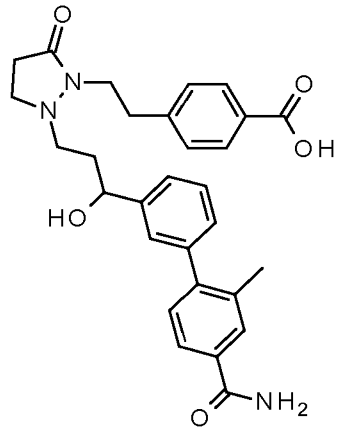
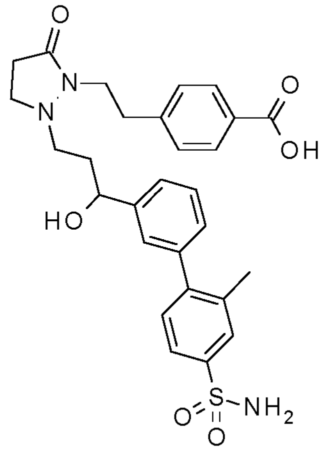
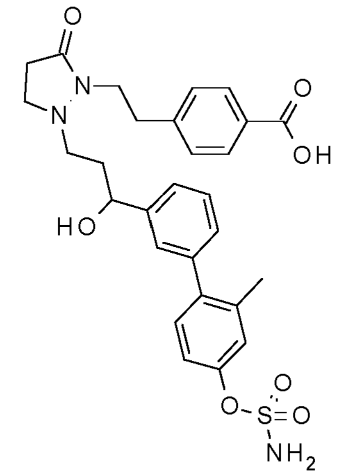
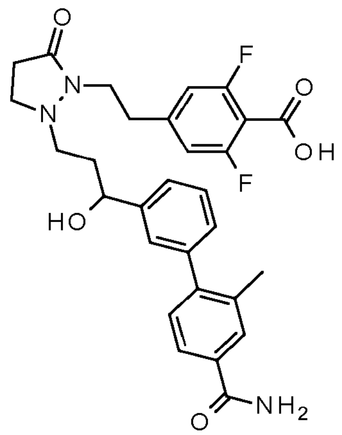
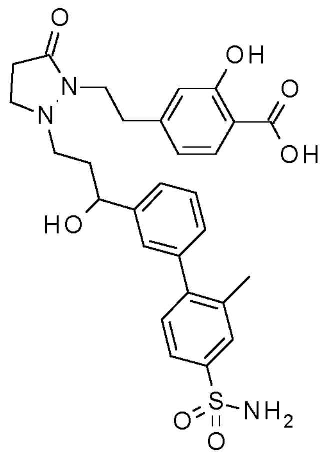
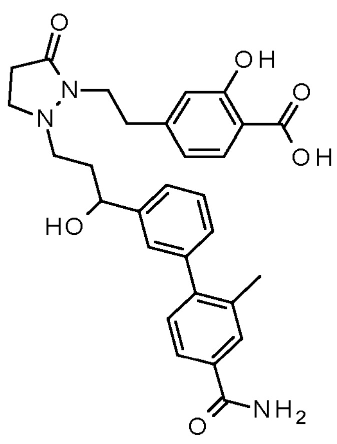
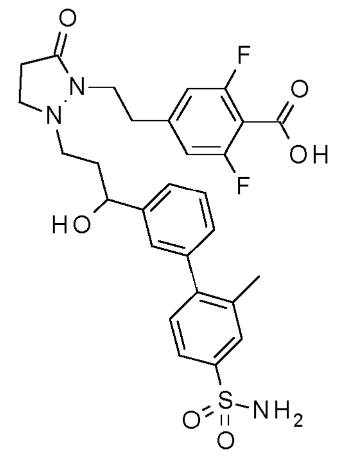
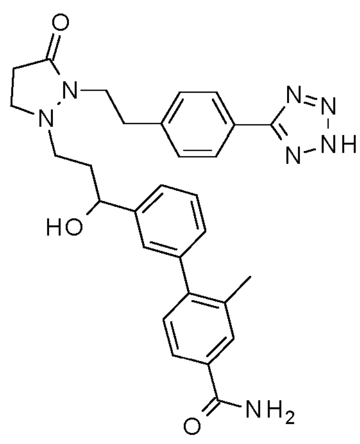

or a pharmaceutically acceptable salt, solvate, hydrate, tautomer, or optical isomer thereof.

**11.** A pharmaceutical composition comprising a compound, pharmaceutically acceptable salt, solvate, hydrate, or tautomer, according to any one of claims 1-10 and a pharmaceutically acceptable excipient; and optionally wherein the composition further comprises at least one additional therapeutic agent selected from the group consisting of aminosalicylates, corticosteroids, immunomodulators and combinations thereof.

**12.** A kit comprising a compound, pharmaceutically acceptable salt, solvate, hydrate, tautomer or optical isomer according to any one of claims 1-10 and at least one additional therapeutic agent selected from the group consisting of aminosalicylates, corticosteroids, immunomodulators and combinations thereof.

**13.** A compound according to any one of claims 1 to 10, a composition according to claim 11, or a kit according to claim 12, for use as a medicament.

**14.** The compound according to any one of claims 1 to 10, composition according to claim 11, or kit according to claim 12, for use in the treatment of an EP4 receptor mediated disease, wherein the EP4 receptor mediated disease is a gastrointestinal disorder or a pulmonary disease or condition.

**15.** The compound, composition or kit for use according to claim 14, wherein:

(i) the gastrointestinal disorder is selected from the group consisting of constipation disorders, constipation-predominant irritable bowel syndrome, mixed type irritable bowel syndrome, chronic idiopathic constipation, gastrointestinal symptoms associated with Parkinson's disease, gastrointestinal symptoms associated with cystic fibrosis, intestinal dysmotility, postoperative ileus, food allergy or food intolerance, celiac disease, gastrointestinal motility disorders, functional gastrointestinal disorders, drug induced enteropathy, NSAID induced gastric and intestinal injury, chemotherapy induced mucositis, gastroesophageal reflux disease (GERD), duodenogastric reflux, diarrhoeal diseases, immune mediated gastrointestinal diseases, Crohn's disease, ulcerative colitis, inflammatory bowel disease, and ischemic colitis; and/or
(ii) the pulmonary disease or condition is selected from chronic obstructive pulmonary diseases, asthma, chronic bronchitis, cystic fibrosis, emphysema, chronic idiopathic cough, hyperactive airway disorder, and idiopathic pulmonary fibrosis.

**Patentansprüche**

**1.** Verbindung der Formel I:

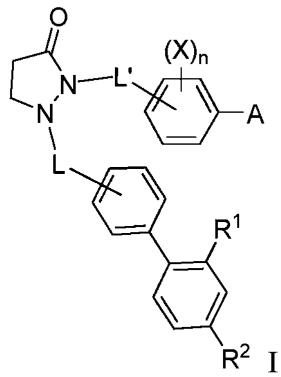

I

oder ein pharmazeutisch akzeptables Salz, Solvat, Hydrat, Tautomer oder optisches Isomer davon, wobei;
A OR', C(O)R', $CO_2R'$, $C(O)N(R')_2$, $C(O)N(R')S(O)_2R^3$, $S(O)_2R'$, $S(O)_2OR'$, $SO_2N(R')_2$, $C_{1-8}$-Alkyl, Cycloalkyl, Heterocycloalkyl, Aryl oder Heteroaryl ist;
X Halogen, OR', COOR' oder $C_{1-6}$-Alkyl ist;
L und L' jeweils unabhängig voneinander ein $C_{2-4}$-Alkylen sind;
$R^1$ H, Halogen, CN, $NO_2$, OR', SR', COOR', $C_{1-6}$-Alkoxy oder $C_{1-6}$-Alkyl ist;
$R^2$ OR', $OC(O)R^3$, $OC(O)OR^3$, $CO_2R'$, $CON(R')_2$, $SO_2N(R')_2$, $SO_2R_3$, $OSO_2R^3$ oder $OSO_2N(R')_2$ ist;
$R^3$ $C_{1-6}$-Alkyl, $C_{3-6}$-Cycloalkyl oder ein Phenyl ist;
R' H, $C_{1-6}$-Alkyl oder $C_{3-6}$-Cycloalkyl ist; und
n 0, 1, 2, oder 3 ist;
wobei bei jedem Auftreten Alkyl, Alkylen, Alkoxy und Cycloalkyl jeweils wahlweise und unabhängig voneinander substituiert mit bis zu 3 Vorkommen von OH, SH, CN, $NO_2$, COOH, Halogen oder $COOC_{1-4}$-Alkyl sind;
wobei bei jedem Auftreten Heterocycloalkyl, Aryl und Heteroaryl jeweils wahlweise und unabhängig voneinander substituiert mit bis zu 3 Vorkommen von OR', SR', CN, $NO_2$, $CO_2R'$, Halogen, $C_{1-4}$-Alkyl oder Oxo sind.

**2.** Verbindung nach Anspruch 1, wobei

(i) L' die Gruppe

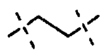

ist; und/oder
(ii) L die Gruppe

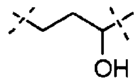

ist.

3. Verbindung nach Anspruch 1 oder 2, wobei die Verbindung eine Verbindung der Formel (1) ist:

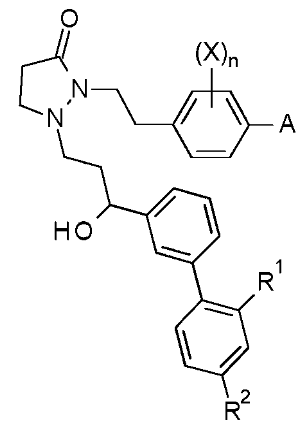

(1),

oder ein pharmazeutisch akzeptables Salz, Solvat, Hydrat, Tautomer oder optisches Isomer davon, wobei A, X, $R^1$, $R^2$ und n dieselben sind wie in Anspruch 1 definiert.

4. Verbindung nach einem der Ansprüche 1-3, wobei A aus C(O)OR', C(O)N(R')$S(O)_2R^3$, S(O)2OR', $C_{1-8}$-Alkyl, Heterocycloalkyl, oder Heteroaryl ausgewählt ist, wobei das Heterocycloalkyl, und das Heteroaryl jeweils wahlweise und unabhängig voneinander substituiert mit bis zu 3 Vorkommen von OR', SR', Halogen, $C_{1-4}$-Alkyl, oder Oxo sind; vorzugsweise wobei A aus der Gruppe ausgewählt ist, die aus Folgendem besteht:

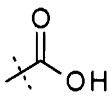 , 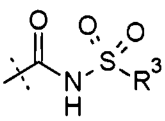 , 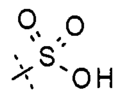 , 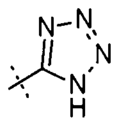 , 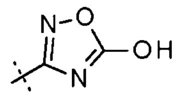 , 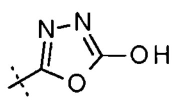 ,

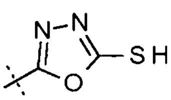 , 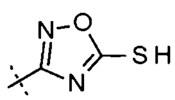 , 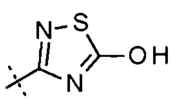 , 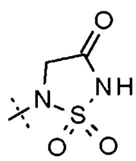 , 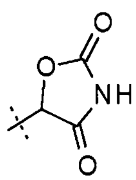 ;

bevorzugter wobei A ist:

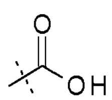 oder 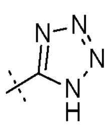 ;

besonders bevorzugt, wobei A

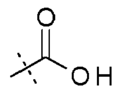

ist.

**5.** Verbindung nach einem der Ansprüche 1-4, wobei:

(i) X Halogen oder OR' ist, vorzugsweise wobei X F oder OH ist; bevorzugter wobei X F oder OH ist, und n 1, oder 2 ist; oder
(ii) n 0 ist.

**6.** Verbindung nach einem der Ansprüche 1-5, wobei $R^1$ H, OH, Halogen, CN, $C_{1-6}$-Alkoxy wahlweise substituiert mit 1-3 Fluoratomen oder $C_{1-6}$-Alkyl wahlweise substituiert mit 1-3 Fluoratomen ist; vorzugsweise wobei $R^1$ $C_{1-6}$-Alkyl wahlweise substituiert mit 1-3 Fluoratomen ist; bevorzugter wobei $R^1$ Methyl ist.

**7.** Verbindung nach einem der Ansprüche 1-6, wobei:

(i) $R^2$ OR', CON(R')2, $SO_2N(R')_2$ oder $OSO_2N(R')_2$ ist, vorzugsweise wobei $R^2$ OH, $CONH_2$, $SO_2NH_2$, oder $OSO_2NH_2$ ist; oder
(ii) $R^2$ $CON(R')_2$, $SO_2N(R')_2$, $OSO_2R'$, oder $OSO_2N(R')_2$ ist; vorzugsweise wobei $R^2$ $CONH_2$, $SO_2NH_2$, oder $OSO_2NH_2$ ist.

**8.** Verbindung nach einem der Ansprüche 1-7, welche eine Verbindung der Formel (2a), (2b), (2c) oder (2d) ist:

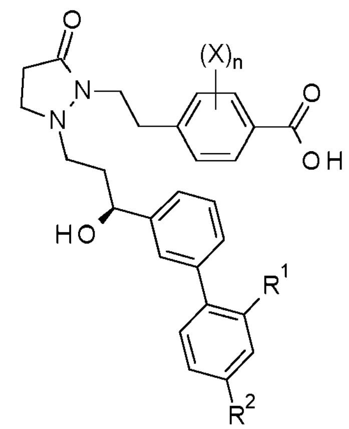
(2a)

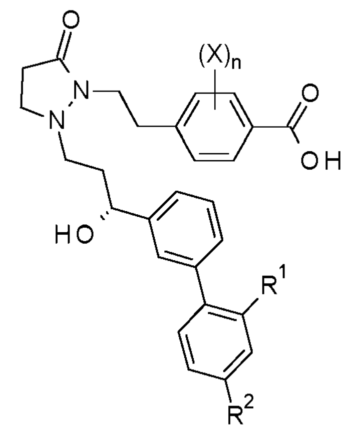
(2b)

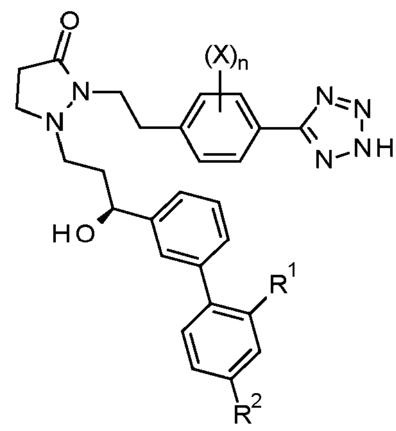
(2c)

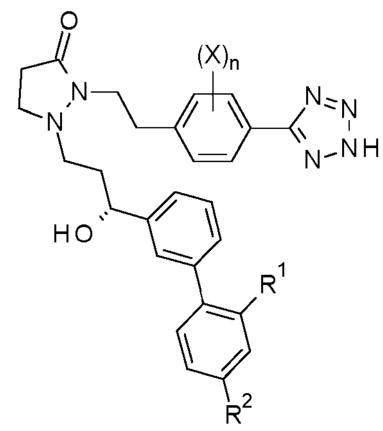
(2d),

oder ein pharmazeutisch akzeptables Salz, Solvat, Hydrat oder Tautomer davon, wobei X, $R^1$, $R^2$ und n dieselben sind wie in einem beliebigen vorangehenden Anspruch definiert.

**9.** Verbindung nach Anspruch 1, welche eine Verbindung der Formel (5), (5a), (5b), (6), (6a), (6b) ist:

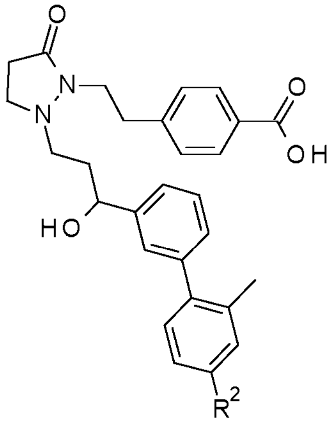
(5)

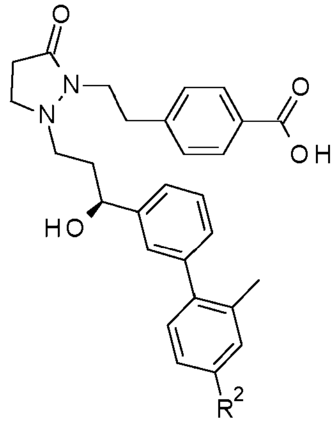
(5a)

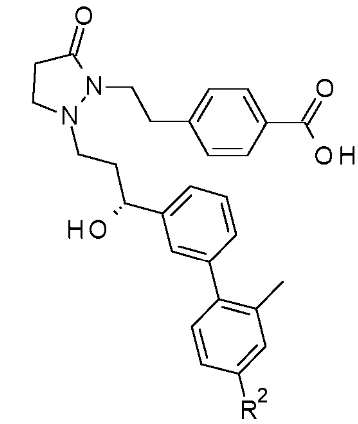
(5b)

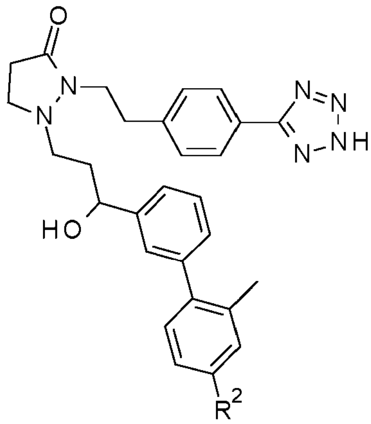
(6)

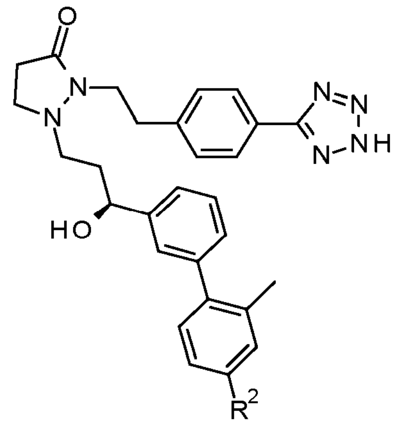
(6a)

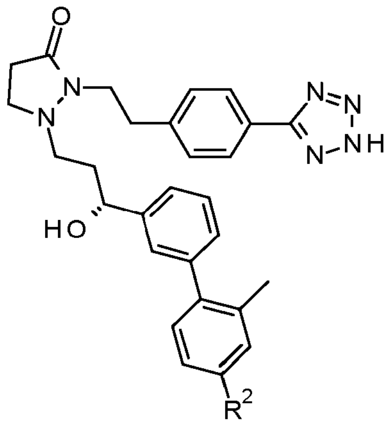
(6b)

oder ein pharmazeutisch akzeptables Salz, Solvat, Hydrat oder Tautomer davon.

**10.** Verbindung nach Anspruch 1, wobei die Verbindung aus der Gruppe ausgewählt ist, die aus Folgendem besteht:

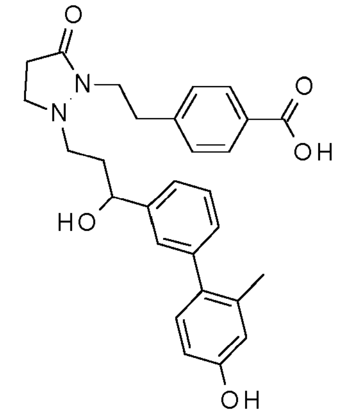
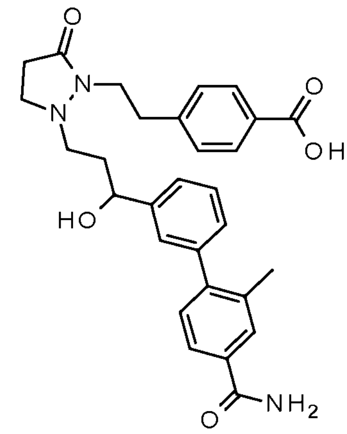
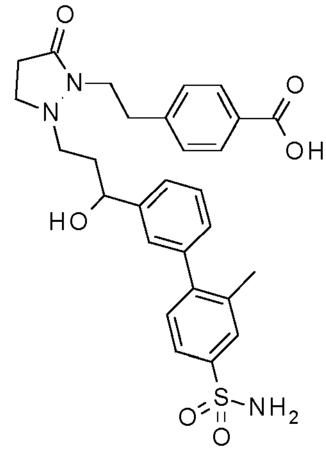

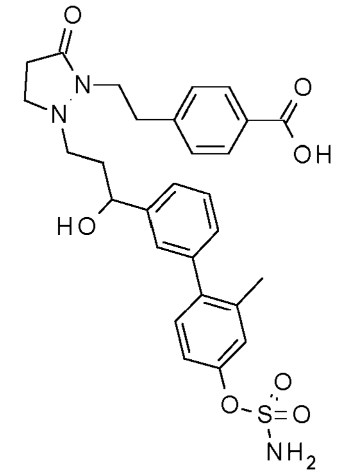
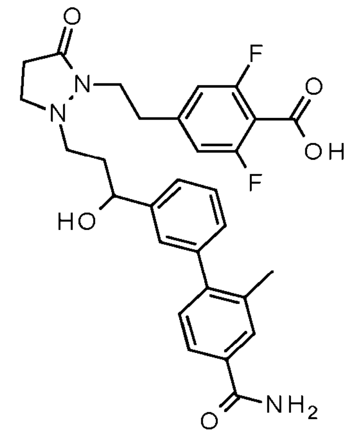
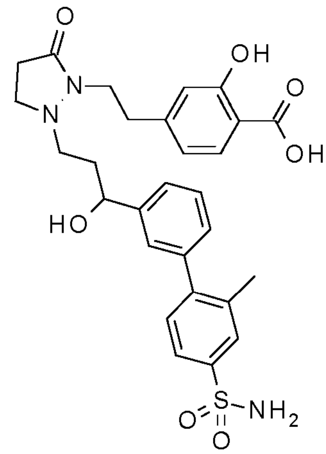

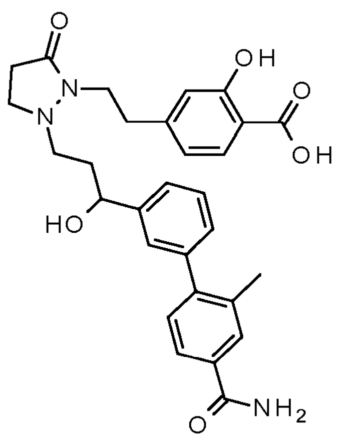
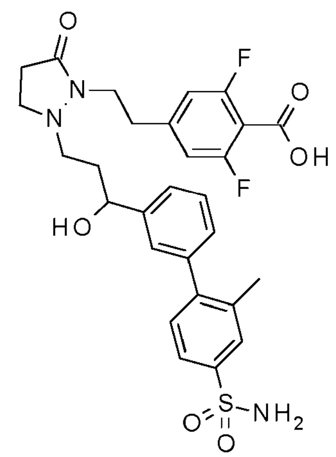
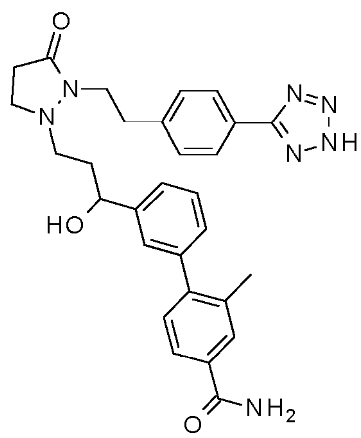

oder einem pharmazeutisch akzeptablem Salz, Solvat, Hydrat, Tautomer oder optischem Isomer davon.

**11.** Pharmazeutische Zusammensetzung, umfassend eine Verbindung, ein pharmazeutisch akzeptables Salz, Solvat, Hydrat oder Tautomer, nach einem der Ansprüche 1-10 und einen pharmazeutisch akzeptablen Hilfsstoff; und wahlweise wobei die Zusammensetzung weiter mindestens einen zusätzlichen therapeutischen Wirkstoff umfasst, der aus der Gruppe ausgewählt ist, die aus Aminosalicylaten, Corticosteroiden, Immunmodulatoren und Kombinationen davon besteht.

**12.** Kit, umfassend eine Verbindung, ein pharmazeutisch akzeptables Salz, Solvat, Hydrat, Tautomer oder optisches Isomer nach einem der Ansprüche 1-10 und mindestens einen zusätzlichen therapeutischen Wirkstoff, der aus der

Gruppe ausgewählt ist, die aus Aminosalicylaten, Corticosteroiden, Immunmodulatoren und Kombinationen davon besteht.

**13.** Verbindung nach einem der Ansprüche 1 bis 10, eine Zusammensetzung nach Anspruch 11, oder ein Kit nach Anspruch 12, zur Verwendung als Arzneimittel.

**14.** Verbindung nach einem der Ansprüche 1 bis 10, Zusammensetzung nach Anspruch 11, oder Kit nach Anspruch 12, zur Verwendung bei der Behandlung einer EP4-Receptor-vermittelten Erkrankung, wobei die EP4-Receptor-vermittelte Erkrankung eine Magen-Darm-Störung oder eine Lungenerkrankung oder -zustand ist.

**15.** Verbindung, Zusammensetzung oder Kit zur Verwendung nach Anspruch 14, wobei:

(i) die Magen-Darm-Störung aus der Gruppe ausgewählt ist, die aus Obstipationsstörungen, obstipationsprädominantem Reizdarmsyndrom, Reizdarmsyndrom vom Mischtyp, chronischer idiopathischer Obstipation, Magen-Darm-Symptomen in Zusammenhang mit Parkinson-Krankheit, Magen-Darm-Symptomen in Zusammenhang mit zystischer Fibrose, intestinale Dysmotilität, postoperativer Ileus, Nahrungsmittelallergie oder Nahrungsmittelintoleranz, Zöliakie, Magen-Darm-Motilitätsstörungen, funktionelle Magen-Darm-Störungen, arzneimittelinduzierte Enteropathie, durch NSAID induzierte Magen- und Darmschädigung, durch Chemotherapie induzierte Mukositis, gastroösophageale Refluxkrankheit (GERD), duodenogastrischer Reflux, Diarrhö-Erkrankungen, immunvermittelte Magen-Darm-Erkrankungen, Morbus Crohn, Colitis ulcerosa, entzündliche Darmerkrankung,und ischämische Kolitis besteht; und/oder
(ii) die Lungenerkrankung oder -zustand aus chronisch obstruktiven Lungenerkrankungen, Asthma, chronischer Bronchitis, zystischer Fibrose, Emphysem, chronischem idiopathischem Husten, hyperaktiver Atemwegsstörung und idiopathischer Lungenfibrose ausgewählt ist.

## Revendications

**1.** Composé de la Formule I :

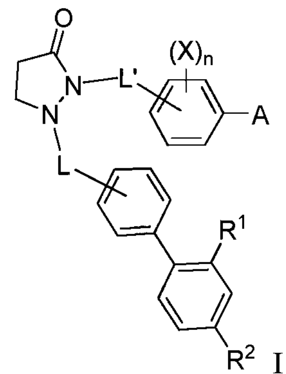
I

ou sel pharmaceutiquement acceptable, solvate, hydrate, tautomère ou isomère optique de celui-ci, dans lequel ;
A est OR', C(O)R', $CO_2R'$, $C(O)N(R')_2$, $C(O)N(R')S(O)_2R^3$, $S(O)_2R'$, $S(O)_2OR'$, $SO_2N(R')_2$, $C_{1-8}$ alkyle, cycloalkyle, hétérocycloalkyle, aryle ou hétéroaryle ;
X est halo, OR', COOR' ou $C_{1-6}$ alkyle ;
L et L' sont chacun indépendamment un $C_{2-4}$ alkylène ;
$R^1$ est H, halo, CN, $NO_2$, OR', SR', COOR', $C_{1-6}$ alcoxy ou $C_{1-6}$ alkyle ;
$R^2$ est OR', $OC(O)R^3$, $OC(O)OR^3$, $CO_2R'$, $CON(R')_2$, $SO_2N(R')_2$, $SO_2R^3$, $OSO_2R^3$ ou $OSO_2N(R')_2$ ;
$R^3$ est $C_{1-6}$ alkyle, $C_{3-6}$ cycloalkyle ou un phényle ;
R' est H, $C_{1-6}$ alkyle ou $C_{3-6}$ cycloalkyle ; et
n vaut 0, 1, 2 ou 3 ;
dans lequel, à chaque occurrence, alkyle, alkylène, alcoxy et cycloalkyle sont chacun facultativement et indépendamment substitués par jusqu'à 3 occurrences de OH, SH, CN, $NO_2$, COOH, halo ou $COOC_{1-4}$ alkyle ;
dans lequel, à chaque occurrence, hétérocycloalkyle, aryle et hétéroaryle sont chacun facultativement et indépendamment substitués par jusqu'à 3 occurrences de OR', SR', CN, $NO_2$, $CO_2R'$, halo, $C_{1-4}$ alkyle ou oxo.

**2.** Composé selon la revendication 1, dans lequel

(i) L' est le groupe

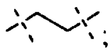;

et/ou
(ii) L est le groupe

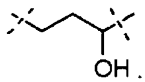.

**3.** Composé selon la revendication 1 ou la revendication 2, dans lequel le composé est un composé de la Formule (1) :

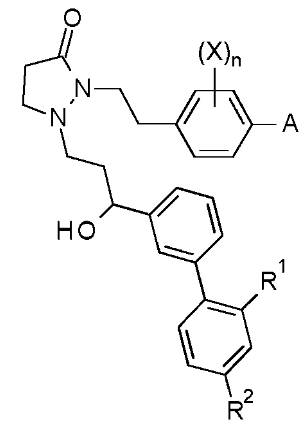

(1),

ou un sel pharmaceutiquement acceptable, un solvate, un hydrate, un tautomère ou un isomère optique de celui-ci, dans lequel A, X, $R^1$, $R^2$ et n sont les mêmes que ceux définis dans la revendication 1.

**4.** Composé selon l'une quelconque des revendications 1 à 3, dans lequel A est sélectionné parmi C(O)OR', C(O)N(R') $S(O)_2R^3$, $S(O)_2OR'$, $C_{1-8}$ alkyle, hétérocycloalkyle ou hétéroaryle, dans lequel l'hétérocycloalkyle et l'hétéroaryle sont chacun facultativement et indépendamment substitués par jusqu'à 3 occurrences de OR', SR', halo, $C_{1-4}$ alkyle ou oxo ; de préférence dans lequel A est sélectionné dans le groupe consistant en :

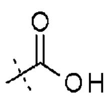, 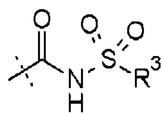, 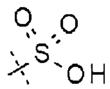, 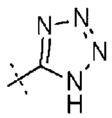, 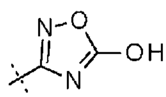, 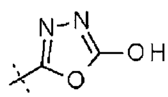,

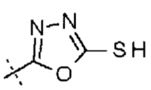, 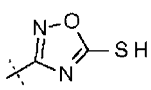, 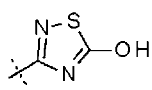, 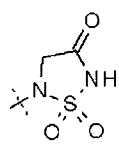, 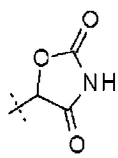;

plus préférentiellement dans lequel A est :

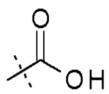

ou

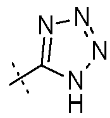

;

le plus préférentiellement dans lequel A est

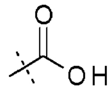

.

**5.** Composé selon l'une quelconque des revendications 1 à 4, dans lequel :

(i) X est halo ou OR', de préférence dans lequel X est F ou OH ; plus préférentiellement dans lequel X est F ou OH, et n vaut 1 ou 2 ; ou
(ii) n vaut 0.

**6.** Composé selon l'une quelconque des revendications 1 à 5, dans lequel $R^1$ est H, OH, halo, CN, $C_{1-6}$ alcoxy facultativement substitué par 1 à 3 atomes de fluor ou $C_{1-6}$ alkyle facultativement substitué par 1 à 3 atomes de fluor ; de préférence dans lequel $R^1$ est $C_{1-6}$ alkyle facultativement substitué par 1 à 3 atomes de fluor ; plus préférentiellement dans lequel $R^1$ est du méthyle.

**7.** Composé selon l'une quelconque des revendications 1 à 6, dans lequel :

(i) $R^2$ est OR', $CON(R')_2$, $SO_2N(R')_2$ ou $OSO_2N(R')_2$, de préférence dans lequel $R^2$ est OH, $CONH_2$, $SO_2NH_2$ ou $OSO_2NH_2$ ; ou
(ii) $R^2$ est $CON(R')_2$, $SO_2N(R')_2$, $OSO_2R^3$ ou $OSO_2N(R')_2$ ; de préférence dans lequel $R^2$ est $CONH_2$, $SO_2NH_2$ ou $OSO_2NH_2$.

**8.** Composé selon l'une quelconque des revendications 1 à 7 qui est un composé de Formule (2a), (2b), (2c) ou (2d) :

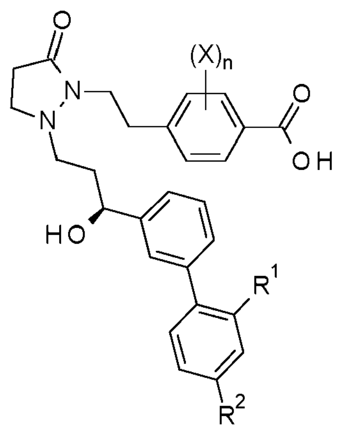

(2a)

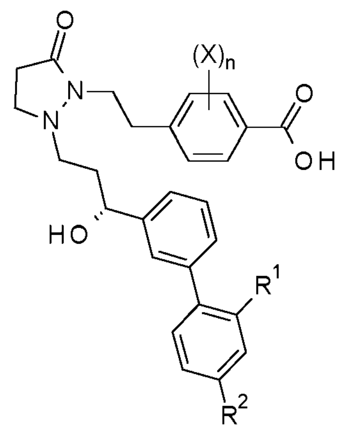

(2b)

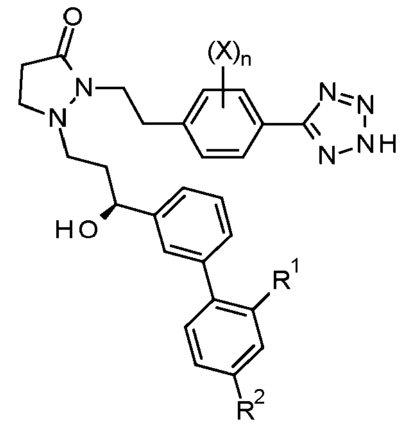
(2c)

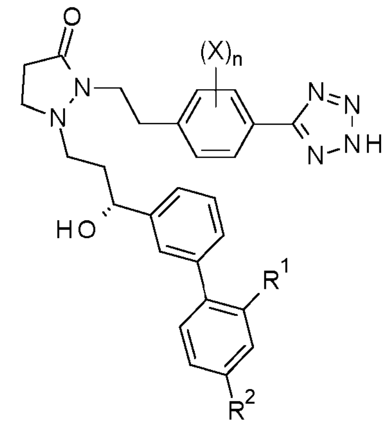
(2d),

ou un sel pharmaceutiquement acceptable, un solvate, un hydrate ou un tautomère de celui-ci, dans lequel X, $R^1$, $R^2$ et n sont les mêmes que ceux définis dans une quelconque revendication précédente.

**9.** Composé selon la revendication 1, qui est un composé de Formule (5), (5a), (5b), (6), (6a), (6b) :

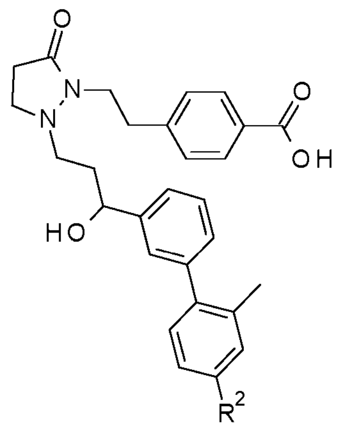
(5)

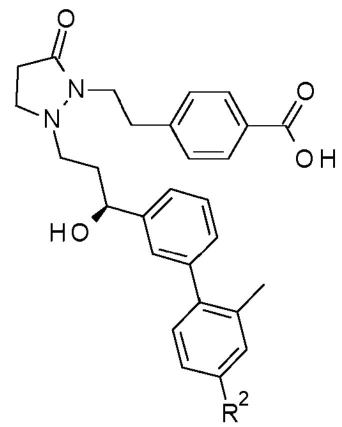
(5a)

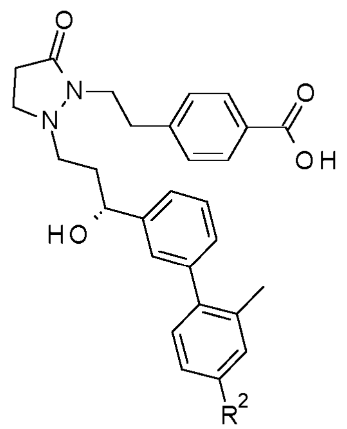
(5b)

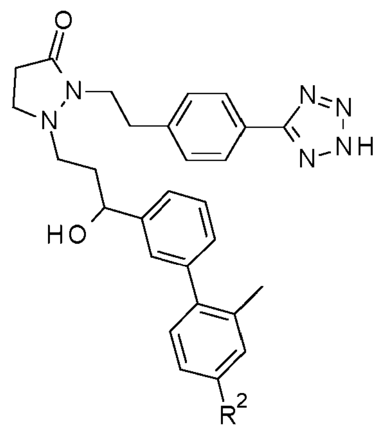
(6)

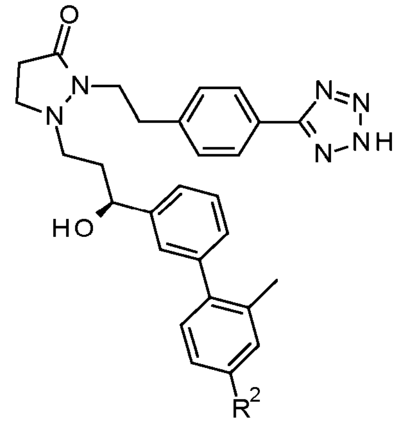

(6a)

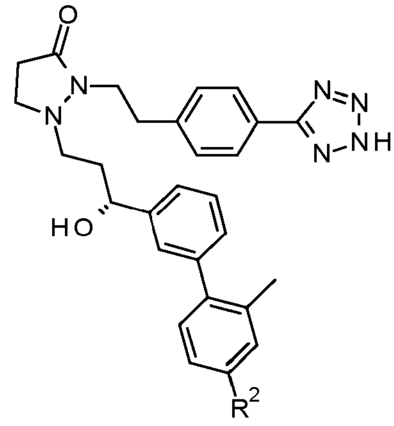

(6b)

ou un sel pharmaceutiquement acceptable, solvate, hydrate ou tautomère de celui-ci.

**10.** Composé selon la revendication 1, dans lequel le composé est sélectionné dans le groupe consistant en :

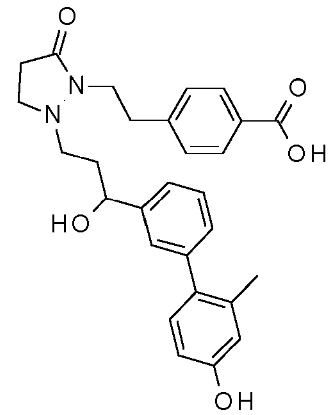

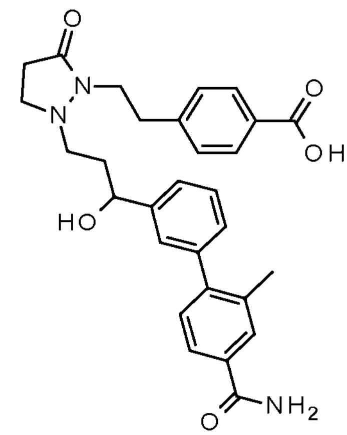

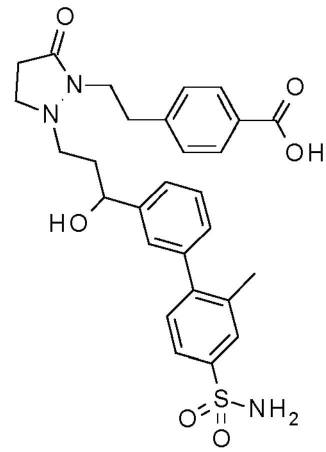

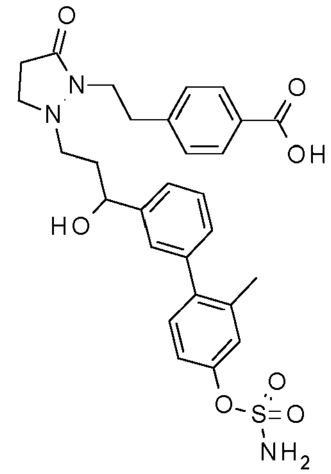

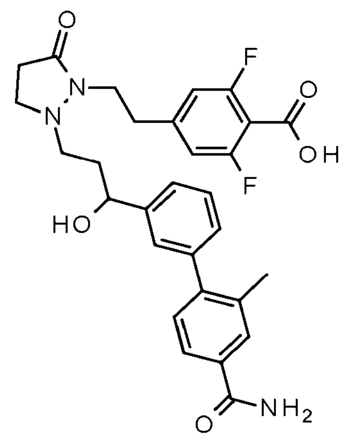

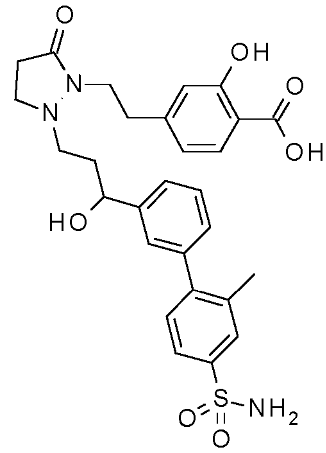

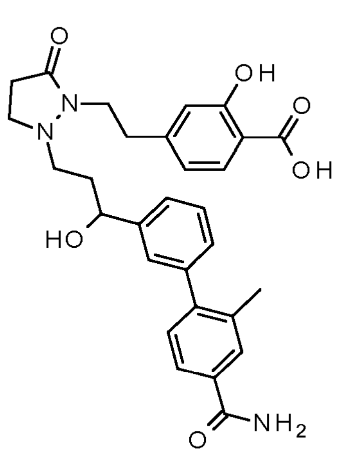 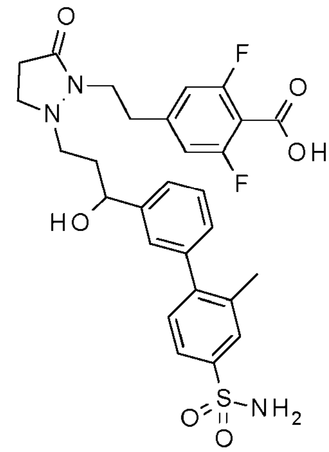 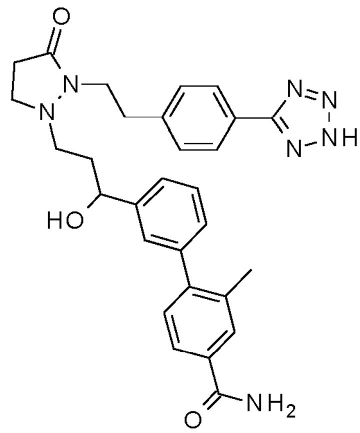

ou un sel pharmaceutiquement acceptable, un solvate, un hydrate, un tautomère ou un isomère optique de celui-ci.

**11.** Composition pharmaceutique comprenant un composé, un sel pharmaceutiquement acceptable, un solvate, un hydrate ou un tautomère, selon l'une quelconque des revendications 1 à 10 et un excipient pharmaceutiquement acceptable ; et facultativement dans laquelle la composition comprend en outre au moins un agent thérapeutique supplémentaire sélectionné dans le groupe consistant en aminosalicylates, corticostéroïdes, immunomodulateurs et des combinaisons de ceux-ci.

**12.** Kit comprenant un composé, un sel pharmaceutiquement acceptable, un solvate, un hydrate, un tautomère ou un isomère optique selon l'une quelconque des revendications 1 à 10 et au moins un agent thérapeutique supplémentaire sélectionné dans le groupe consistant en aminosalicylates, corticostéroïdes, immunomodulateurs et des combinaisons de ceux-ci.

**13.** Composé selon l'une quelconque des revendications 1 à 10, une composition selon la revendication 11, ou un kit selon la revendication 12, pour une utilisation en tant que médicament.

**14.** Composé selon l'une quelconque des revendications 1 à 10, composition selon la revendication 11, ou kit selon la revendication 12, pour une utilisation dans le traitement d'une maladie médiée par le récepteur EP4, dans lequel ou dans laquelle la maladie médiée par le récepteur EP4 est un trouble gastro-intestinal ou une maladie ou affection pulmonaire.

**15.** Composé, composition ou kit pour utilisation selon la revendication 14, dans lequel ou dans laquelle :

(i) le trouble gastro-intestinal est sélectionné dans le groupe consistant en troubles de la constipation, syndrome du côlon irritable à prédominance de constipation, syndrome du côlon irritable de type mixte, constipation idiopathique chronique, symptômes gastro-intestinaux associés à la maladie de Parkinson, symptômes gastro-intestinaux associés à la fibrose kystique, dysmotilité intestinale, iléus postopératoire, allergie alimentaire ou intolérance alimentaire, maladie cœliaque, troubles de la motilité gastro-intestinale, troubles gastro-intestinaux fonctionnels, entéropathie induite par un médicament, lésion gastrique et intestinale induite par NSAID, mucosite induite par une chimiothérapie, maladie de reflux gastro-œsophagien (GERD), reflux duodénogastrique, maladies diarrhéiques, maladies gastro-intestinales médiées par le système immunitaire, maladie de Crohn, colite ulcéreuse, maladie inflammatoire de l'intestin et colite ischémique ; et/ou
(ii) la maladie ou affection pulmonaire est sélectionnée parmi des maladies pulmonaires obstructives chroniques, asthme, bronchite chronique, fibrose kystique, emphysème, toux idiopathique chronique, trouble des voies aériennes hyperactives et fibrose pulmonaire idiopathique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03035064 A


### Non-patent literature cited in the description


- **MIYOSHI, H et al.** *EMBO J.*, 2017, vol. 36, 5-24
- **KABASHIMA, K. et al.** *J. Clin. Invest.*, 2002, vol. 109, 883-893
- **WATANABE, Y. et al.** *Eur. J. Pharmacol.*, 2015, vol. 754, 179-189
- **NITTA, M. et al.** *Scand. J. Immunol.*, 2002, vol. 56, 66-75
- **NAKASE, H. et al.** *Inflamm. Bowel Dis.*, 2010, vol. 16, 731-733
- **BUCKLEY, J et al.** *Thorax*, 2011, vol. 66, 1029-1035
- **HONDA, A. et al.** *Eur. J. Pharmacol.*, 2016, vol. 775, 130-137
- **BRYN et al.** Solid-State Chemistry of Drugs, Second Edition. SSCI, Inc of West Lafayette, 1999
- **HOLFORD, N.H.G** ; **SCHEINER, L.B.** *Clin. Pharmacokinet.*, 1981, vol. 6, 429-453
- **LOEWE, S.** ; **MUISCHNEK, H.** *Arc h. Exp. Pathol Pharmacol.*, 1926, vol. 114, 313-326
- **CHOU, T.C.** ; **TALALAY, P.** *Adv. Enzyme Regul.*, 1984, vol. 22, 27-55